(19) Europäisches Patentamt
European Patent Office
Office européen des brevets

(11) EP 4 143 578 B1

(12) EUROPEAN PATENT SPECIFICATION

(45) Date of publication and mention of the grant of the patent:
**05.06.2024 Bulletin 2024/23**

(21) Application number: **21719839.9**

(22) Date of filing: **28.04.2021**

(51) International Patent Classification (IPC):
***G01N 33/574*** *(2006.01)*

(52) Cooperative Patent Classification (CPC):
**G01N 33/57423**

(86) International application number:
**PCT/EP2021/061085**

(87) International publication number:
**WO 2021/219696 (04.11.2021 Gazette 2021/44)**

(54) **BIOMARKERS FOR DETECTION OF LUNG CANCER**
BIOMARKER ZUR DETEKTION VON LUNGENKREBS
BIOMARQUEURS POUR LA DÉTECTION DU CANCER DU POUMON

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **28.04.2020 EP 20171726**

(43) Date of publication of application:
**08.03.2023 Bulletin 2023/10**

(73) Proprietors:
- **Luxembourg Institute of Health (LIH)**
  **1445 Strassen (LU)**
- **Fred Hutchinson Cancer Center**
  **Seattle, WA 98109 (US)**
- **The Translational Genomics Research Institute (TGEN)**
  **Phoenix, AZ 85004 (US)**

(72) Inventors:
- **EL KHOURY, Victoria**
  **2410 Strassen (LU)**
- **SCHRITZ, Anna, Elisabeth**
  **54453 Nittel (DE)**
- **KIM, Yeoun, Jin**
  **Gaithersburg, Maryland 20878 (US)**
- **BERCHEM, Guy**
  **3348 Leudelange (LU)**
- **PAULOVICH, Amanda**
  **Seattle, WA 98115 (US)**
- **WHITEAKER, Jeffrey**
  **Bellevue, WA 98006 (US)**
- **PETRITIS, Konstantinos**
  **Peachtree Corners, GA 30092 (US)**
- **PIRROTTE, Patrick**
  **Scottsdale, AZ 85260 (US)**
- **TEGELER, Tony**
  **Sacramento, California 95691 (US)**

(74) Representative: **De Clercq & Partners**
**Edgard Gevaertdreef 10a**
**9830 Sint-Martens-Latem (BE)**

(56) References cited:
**WO-A1-2015/115922** **WO-A1-2018/148600**
**US-A1- 2013 230 877** **US-A1- 2014 274 772**
**US-A1- 2016 153 053**

- **EL-KHOURY VICTORIA ET AL: "Identification of beta-arrestin-1 as a diagnostic biomarker in lung cancer", BRITISH JOURNAL OF CANCER, NATURE PUBLISHING GROUP, GB, vol. 119, no. 5, 6 August 2018 (2018-08-06), pages 580-590, XP036871305, ISSN: 0007-0920, DOI: 10.1038/S41416-018-0200-0 [retrieved on 2018-08-06]**
- **HE: "Expression profile of RhoGDI2 in lung cancers and role of RhoGDI2 in lung cancer metastasis", ONCOLOGY REPORTS, vol. 24, no. 2, 25 June 2010 (2010-06-25), XP055722484, ISSN: 1021-335X, DOI: 10.3892/or_00000880**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

Remarks:

The complete document including Reference Table(s) and the Sequence Listing(s) can be downloaded from the EPO website

## Description

### FIELD

The present invention relates to methods and kits for diagnosing lung cancer in a biological sample of a subject.

### BACKGROUND

Lung cancer is the most common malignancy in terms of incidence and the deadliest cancer worldwide. Smoking, particularly of cigarettes, is by far the main contributor to lung cancer. The high lung cancer mortality is mainly based on an advanced level of progression at time of diagnosis. The 5-year survival rate drops significantly from 83% for stage IA to 6% for stage IV tumors. At present, more than half of lung cancer patients are diagnosed at a metastatic stage.

Due to the severity of lung cancer and the incurability of lung cancer in an advanced state, there is a high need for methods that allow for early diagnosis of lung cancer. Early diagnosis is a prerequisite for improved patient survival and treatment outcome.

Presently only 15% of newly diagnosed lung tumors are diagnosed at an early stage. In this context, lung cancer screening using low-dose computerized tomography (LDCT) can reduce lung cancer-specific mortality by 20% compared to chest radiography. However, the high percentage of false positive results (96.4% and 94.5% in the LDCT and the radiography groups, respectively), and the malignancy risk associated with cumulative radiation exposure are serious limitations of LDCT.

US 20140274772 A1 describes a method for assessing risk of lung cancer versus benign disease in a subject comprising determining a panel of biomarkers in a biological sample, the panel comprising at least two biomarkers selected from the group consisting of IL-6, IL-1ra, IL-10, SDF-1$\alpha$+$\beta$, TNF-$\alpha$, MIP-1$\alpha$, sIL-2R$\alpha$, CA-125, sE-Selectin, Eotaxin, sEGFR, MMP-2, OPN, MCP-1, CRP, sICAM-1 and CYFRA 21.1.

WO 2018148600 A1 describes a method of determining the risk of a subject for harboring lung cancer, comprising measuring the level of CEA, the level of CA125, the level of CYFRA21-1 and the level of Pro-SFTPB in a biological sample from said subject.

WO 2015115922 A1 describes a method for the detection and/or exclusion of lung cancer comprising the measurement of serum concentrations of the following protein markers: DRG-cyfra21-l, IBL- hCRP, DRG-NSE, NT-CEA, NT-CA125, USCN-SCCAl, h_tPA, S-100 and NT-CA19-9.

Victoria El-Khoury et al., Identification of beta-arrestin-1 as a diagnostic biomarker in lung cancer, British Journal of Cancer (2018) 119:580-590 describes beta-arrestin-1 as a diagnostic marker to differentiate lung adenocarcinoma from squamous cell carcinoma and indicates its potential as a plasma biomarker for non-invasive diagnosis of lung cancer.

US 20130230877 A1 describes a method of determining the likelihood that a lung condition in a subject is cancer, comprising measuring at least 3 proteins selected from the group consisting of ALDOA, FRIL, LG3BP, IBP3, LRP1, ISLR, TSP1, COIA1, GRP78, TETN, PRDX1 and CD14.

Huiyan Niu et al., Expression profile of RhoGD 12 in lung cancers and role of RhoGD 12 in lung cancer metastasis (2010): 24: 465-471 describes that RhoGD12 is likely involved in lung tumor malignancy and metastasis.

US 20160153053 A1 describes a method for assaying a biological sample from a subject in aid of diagnosis, prognosis or monitoring of a disease or other medical condition in the subject, comprising the steps of: a. obtaining or using a microvesicle fraction from a biological sample from a subject; b. extracting nucleic acid from the fraction; and c. detecting the presence or absence of a biomarker in the extracted nucleic acid; wherein the biomarker is a genetic aberration associated with diagnosis, prognosis, status or stage of a disease or other medical condition, and wherein the genetic aberration is in or corresponds to: i. a c-myc gene; ii. a transposable element; iii. a retrotransposon element; iv. a satellite correlated gene; v. a repeated DNA element; vi. non-coding RNA other than miRNA; or vii. a fragment of any of the foregoing.

Therefore, there remains a pressing need for highly sensitive, preferably minimally invasive, methods for detecting lung cancer, in particular during the early stages of the disease, to improve prognosis and reduce overdiagnosis.

### SUMMARY

The present inventors have addressed the challenges for the detection and diagnosis of lung cancer by developing biomarkers and biomarker panels, particularly biomarker panels. Indeed they have established that certain biomarkers are able to detect lung cancer independently of the stage and have a particular strong diagnostic performance in early-stage lung cancer and can thus be useful for diagnosis. Furthermore, present inventors have established that specific methods can successfully be used to identify and validate suitable biomarker panels.

Accordingly, particular aspect of the invention relates to biomarkers and biomarker panels suitable for use in the diagnosis of lung cancer. In particular embodiments, the biomarkers are Rho GDP dissociation inhibitor beta (ARHGDIB), and at least one biomarker selected from the group consisting of alpha-tubulin 4A (TUBA4A), glutathione S-transferase omega 1 (GSTO1), filamin A (FLNA), peroxiredoxin 6 (PRDX6) and cadherin 13 (CDH13). Each of these markers has a predictive value on their own. Generally however, panels of at least two and preferably at least three biomarkers are used. Accordingly particularly preferred combinations are envisaged. In particular embodiments, the method comprises detecting ARHGDIB and at least two other markers selected from the group consisting of alpha-tubulin 4A (TUBA4A), glutathione S-transferase omega 1 (GSTO1), filamin A (FLNA), peroxiredoxin 6 (PRDX6) and cadherin 13 (CDH13). In particular embodiments the biomarker panel comprises at least ARHGDIB and one or two other markers selected from set group. The lung cancer biomarkers of present invention have an excellent performance in that they combine high area under the receiver operating characteristic curve (AUC), high positive predictive value (PPV), high negative predictive value (NPV), high sensitivity and/or high specificity, preferably a high sensitivity and a high NPV or a high specificity, more preferably a high sensitivity, a high NPV and a high specificity. This is supported by an AUC equal to or more than 0.90, a PPV equal to or more than 0.90, a NPV equal to or more than 0.90, a specificity equal to or more than 0.90 and/or a sensitivity equal to or more than 0.90, preferably by a sensitivity equal to or more than 0.90 and a NPV or a specificity equal to or more than 0.90, more preferably by a sensitivity equal to or more than 0.90, a NPV equal to or more than 0.90 and a specificity equal to or more than 0.90. Furthermore, the lung cancer biomarkers of present invention are able to detect lung cancer, in a non-invasive manner, independently of the disease stage. As a result, the biomarker panels as taught herein can be used as a routine test for high- and average- risk individuals (e.g. smokers or former smokers). The biomarkers and biomarker panels taught herein may also efficiently complement currently used techniques in lung cancer screening, such as LDCT, which would reduce the number of false-positive cases that often lead to additional invasive tests and unnecessary costs and expose the patients to physical and mental hardships. In order to make the lung cancer biomarkers of present invention easy to use by medical practitioners, present inventors have further also adopted a threshold-based approach, attributing a threshold value per biomarker, then a score per sample to classify the subject as having lung cancer or not.

A first aspect provides an *in vitro* method for diagnosing lung cancer in a subject, wherein the method comprises detecting Rho GDP dissociation inhibitor beta (ARHGDIB) and at least one biomarker selected from the group consisting of alpha-tubulin 4A (TUBA4A), glutathione S-transferase omega 1 (GSTO1), filamin A (FLNA), peroxiredoxin 6 (PRDX6) and cadherin 13 (CDH13) in a biological sample from the subject.

In particular embodiments, the method comprises detecting ARHGDIB and at least two other biomarkers selected from the group consisting of TUBA4A, GSTO1, FLNA and PRDX6 and CDH13.In particular embodiments, the method comprises detecting at least three biomarkers selected from the group consisting of ARHGDIB, TUBA4A, GSTO1, FLNA, PRDX6 and CDH13.

In particular embodiments, the method comprises detecting ARHGDIB, TUBA4A, GSTO1, FLNA, PRDX6 and CDH13.

In particular embodiments, the biological sample is a body fluid sample; preferably a body fluid sample selected from the group consisting of plasma, serum, whole blood, urine, tissue lysate, cerebrospinal fluid (CSF), saliva and sweat; more preferably a plasma sample.

In particular embodiments, the at least two biomarkers are detected using mass spectrometry analysis methods, biochemical or molecular biological assay methods, immunoassay methods, chromatography methods, or combinations thereof.

In particular embodiments, the method comprises the steps of

(a) measuring the quantity or expression levels of at least ARHGDIB and at least one biomarker selected from the group consisting of TUBA4A, GSTO1, FLNA, PRDX6 and CDH13 in the biological sample from the subject;
(b) calculating a score based on the quantity or expression levels of said biomarkers measured in (a);
(c) comparing the score calculated in (b) with a threshold score; and
(d) diagnosing the subject with lung cancer if the score calculated in (b) is equal to or higher than the threshold score.

A further aspect provides the use a kit, wherein the kit consists of:

(a) means specifically adapted for measuring the quantity or expression level of ARHGDIB and at least one biomarker selected from the group consisting of TUBA4A, GSTO1, FLNA, PRDX6 and CDH13 in a biological sample from a subject; and
(b) a threshold value for said biomarkers or means for establishing said threshold value, wherein said threshold value represents a known diagnosis of lung cancer,

in the diagnosis of lung cancer based on the detection of said biomarkers in a biological sample of a subject.

## BRIEF DESCRIPTION OF DRAWINGS

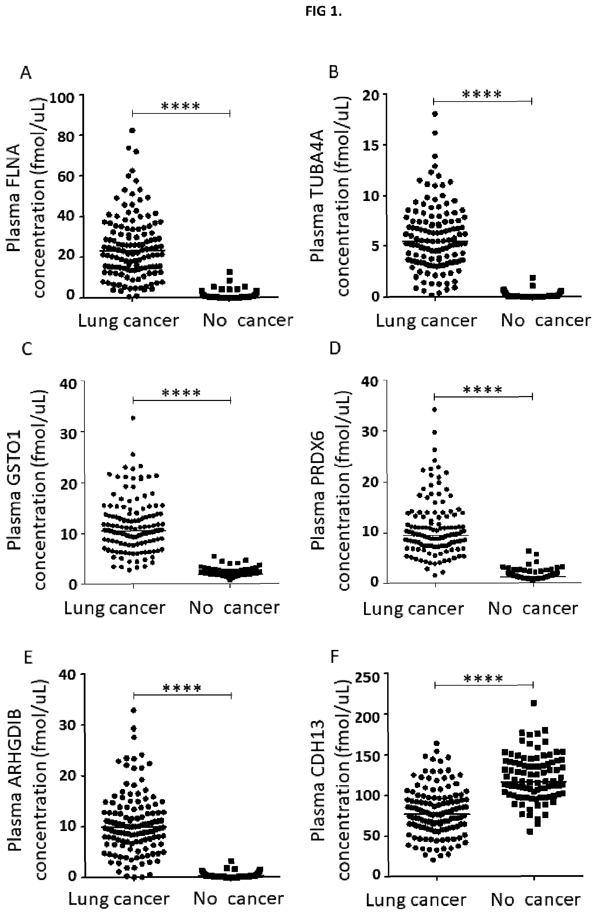

**Figure 1.** Plasma levels of the 6 protein biomarkers identified as a lung cancer diagnostic panel. Scatter plots of (A) FLNA, (B) TUBA4A, (C) GSTO1, (D) PRDX6, (E) ARHGDIB and (F) CDH13 concentrations obtained from lung cancer patients (n = 128) and healthy volunteers (n = 93) using the LC-PRM assay targeting proteotypic peptides. Data points and their median are shown. **** Adjusted P < 0.0001 using non-parametric Kruskal-Wallis test.

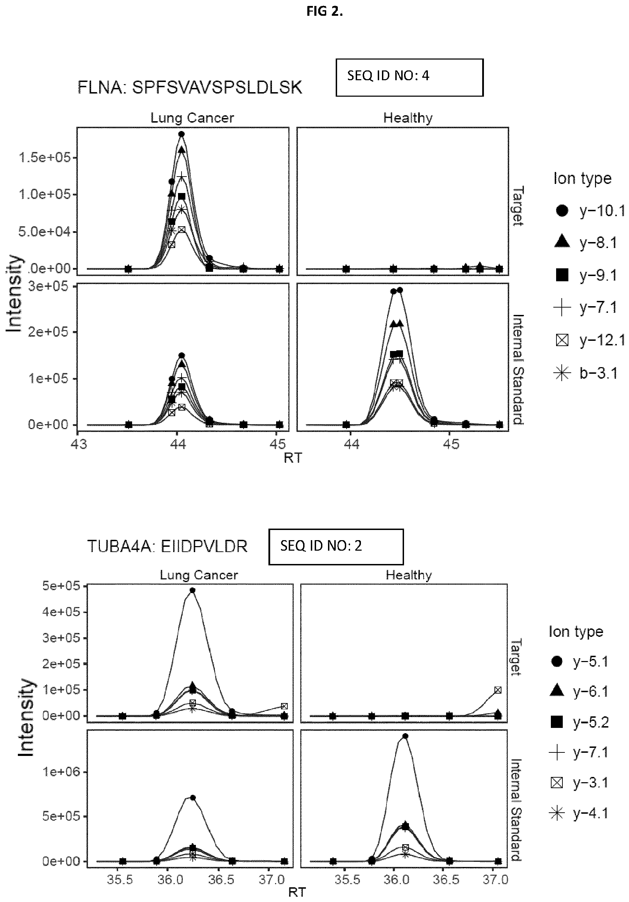

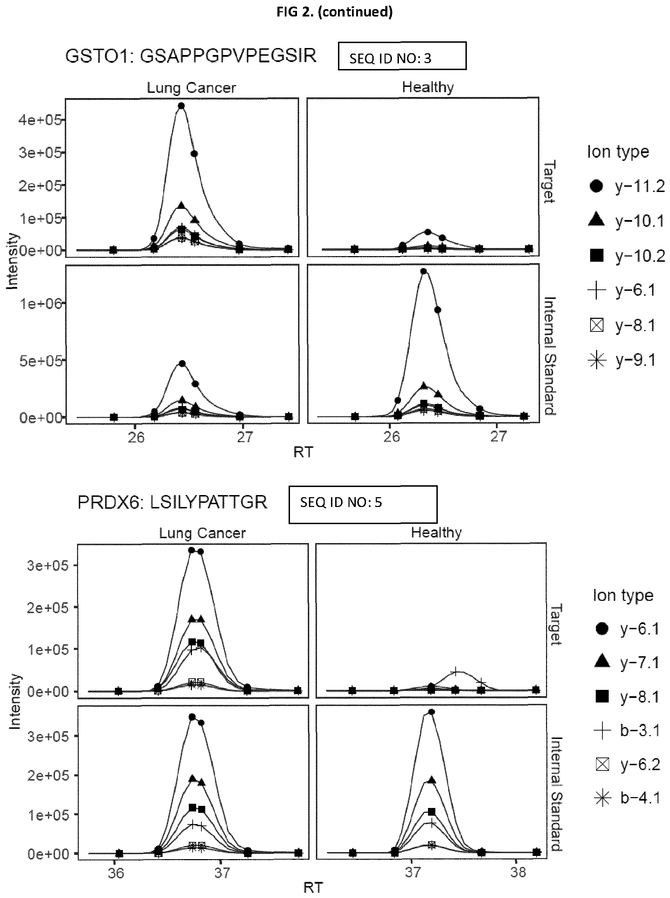

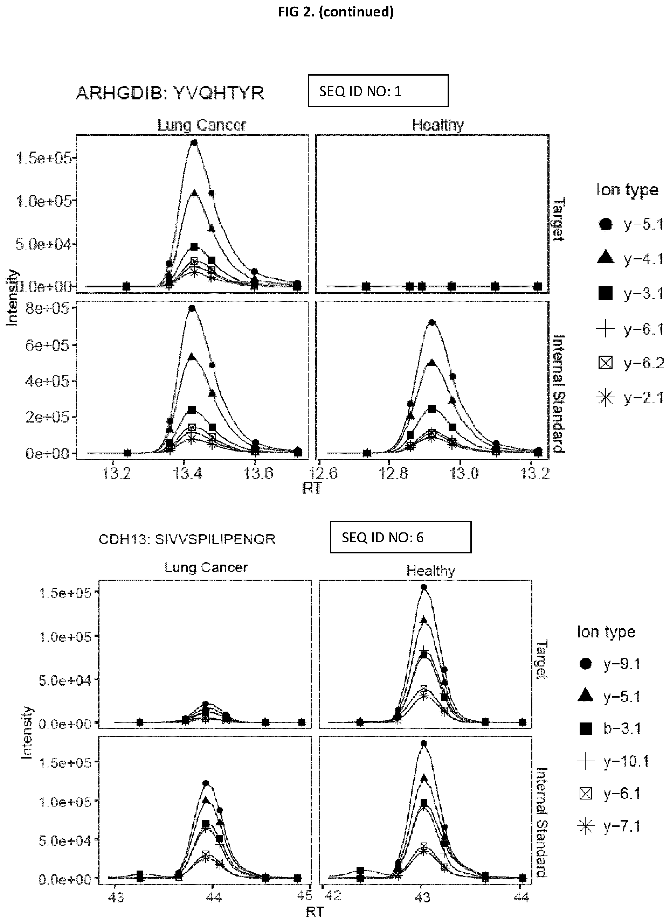

**Figure 2.** Parallel reaction monitoring (PRM) readouts of the 6 proteins included in the diagnostic panel. Representative PRM traces recorded in samples from a lung cancer patient and a healthy donor are shown for each protein. The detected product ions of the endogenous target peptide (upper parts) and the internal standard peptide (lower parts) are displayed.

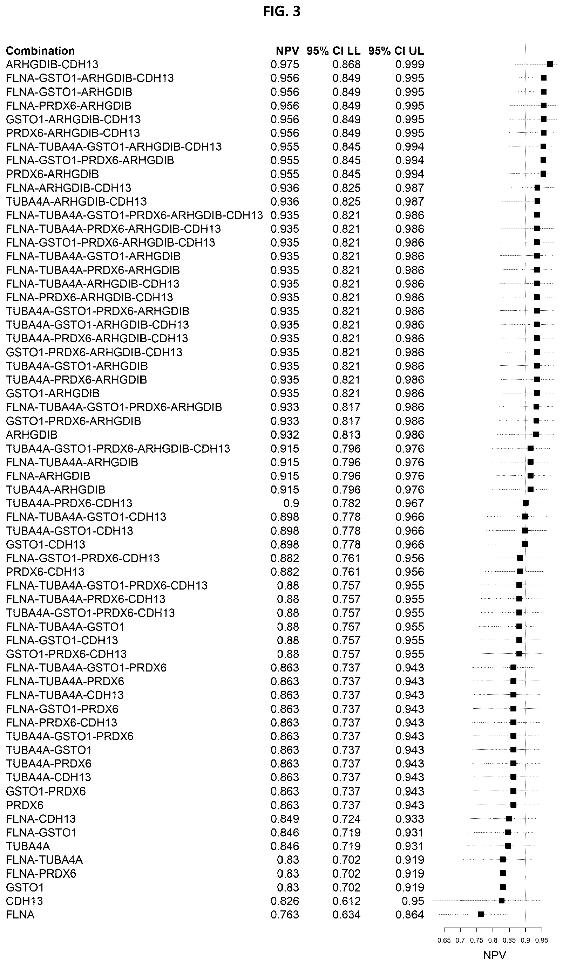

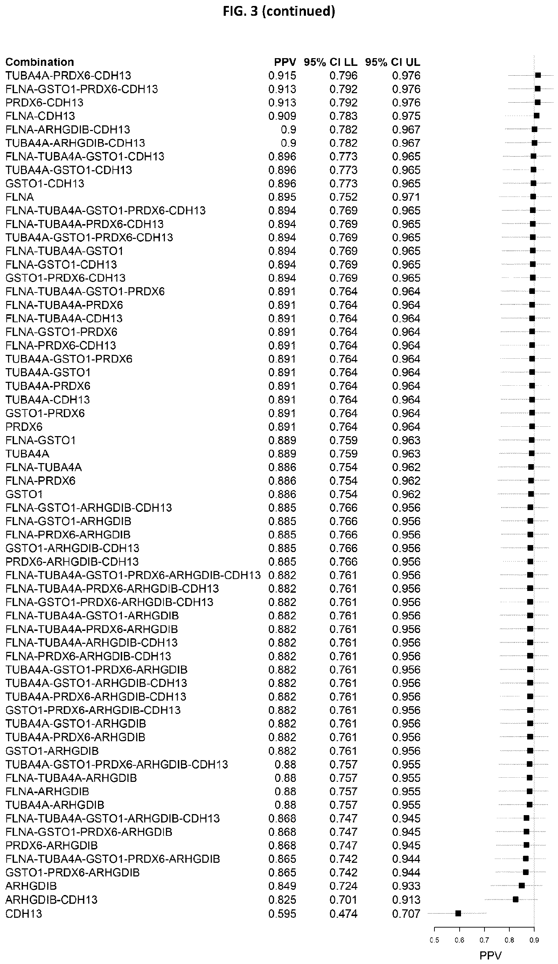

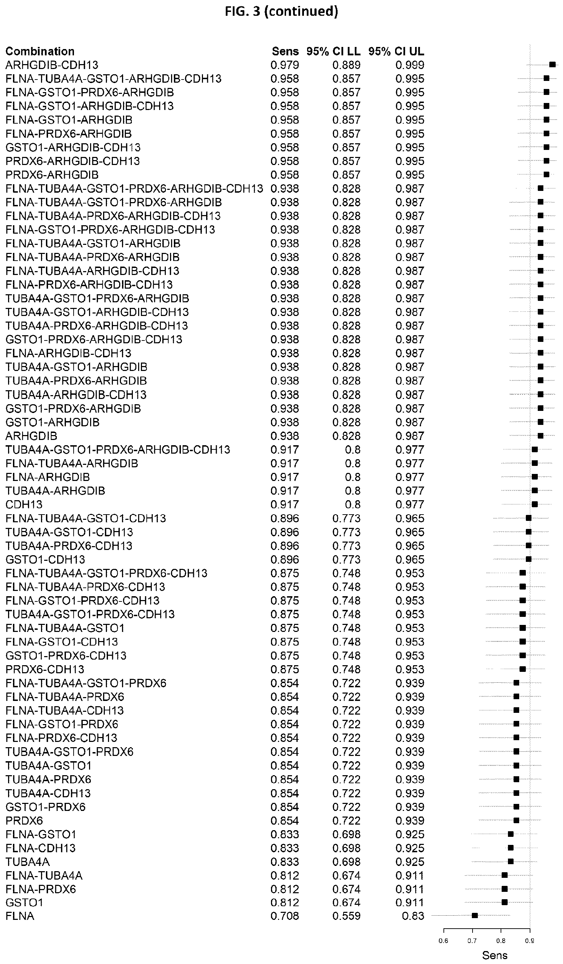

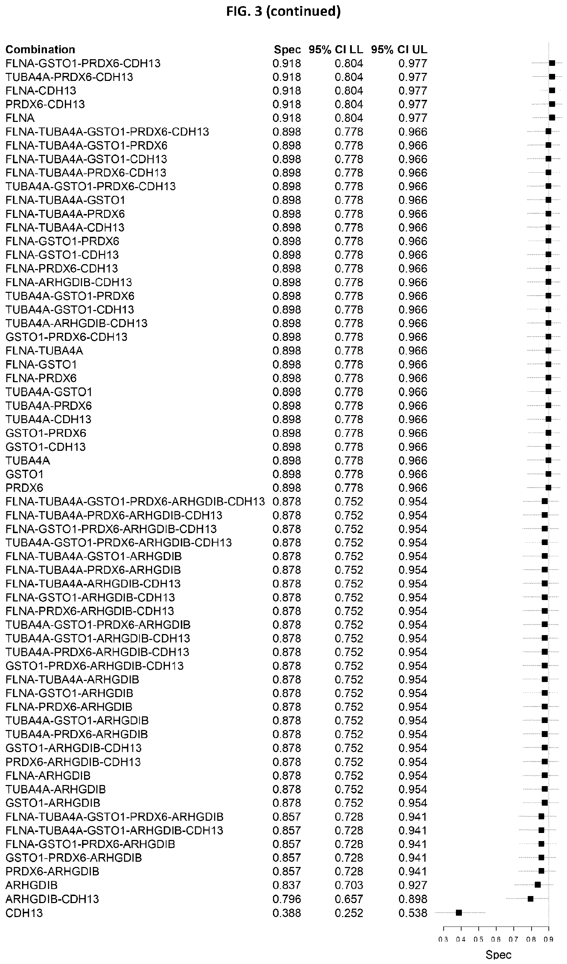

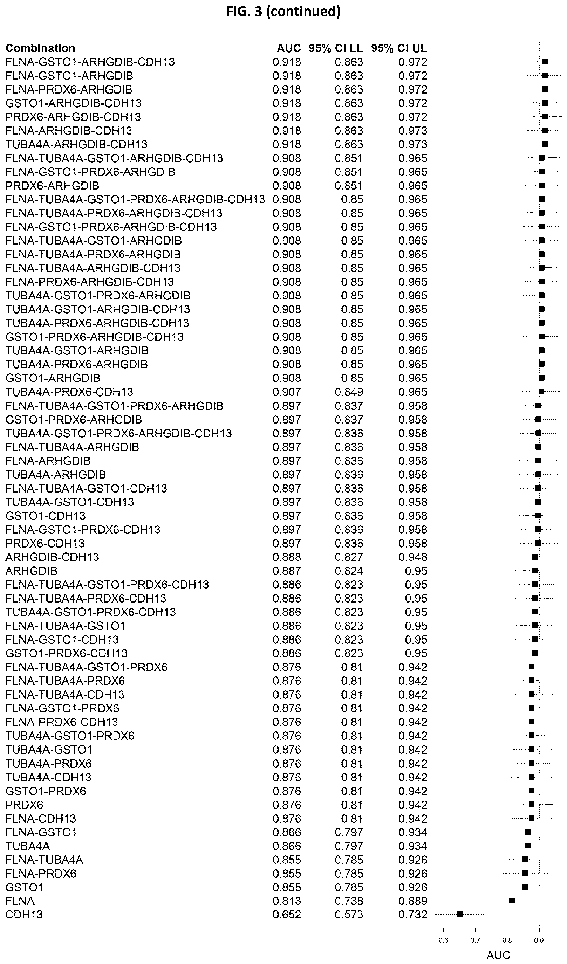

**Figure 3.** Forest plots of negative predictive value (NPV), positive predictive value (PPV), sensitivity ("sens"), specificity ("spec") and area under the receiver operating characteristic curve (AUC) for sub-combinations and single biomarkers of the 6-protein panel tested on the validation dataset.

## DESCRIPTION

As used herein, the singular forms "a", "an", and "the" include both singular and plural referents unless the context clearly dictates otherwise.

The terms "comprising", "comprises" and "comprised of" as used herein are synonymous with "including", "includes" or "containing", "contains", and are inclusive or open-ended and do not exclude additional, non-recited members, elements or method steps. The terms also encompass "consisting of' and "consisting essentially of", which enjoy well-established meanings in patent terminology.

The recitation of numerical ranges by endpoints includes all numbers and fractions subsumed within the respective ranges, as well as the recited endpoints.

The terms "about" or "approximately" as used herein when referring to a measurable value such as a parameter, an amount, a temporal duration, and the like, are meant to encompass variations of and from the specified value, such as variations of +/-10% or less, preferably +/-5% or less, more preferably +/-1% or less, and still more preferably +/-0.1% or less of and from the specified value, insofar such variations are appropriate to perform in the disclosed invention. It is to be understood that the value to which the modifier "about" refers is itself also specifically, and preferably, disclosed.

Whereas the terms "one or more" or "at least one", such as one or more members or at least one member of a group of members, is clear per se, by means of further exemplification, the term encompasses inter alia a reference to any one of said members, or to any two or more of said members, such as, e.g., any ≥3, ≥4, ≥5, ≥6 or ≥7 etc. of said members, and up to all said members. In another example, "one or more" or "at least one" may refer to 1, 2, 3, 4, 5, 6, 7 or more.

The discussion of the background to the invention herein is included to explain the context of the invention. This is not to be taken as an admission that any of the material referred to was published, known, or part of the common general knowledge in any country as of the priority date of any of the claims.

Throughout this disclosure, various publications, patents and published patent specifications are referenced by an identifying citation. All documents cited in the present specification are hereby incorporated by reference in their entirety. In particular, the teachings or sections of such documents herein specifically referred to are incorporated by reference.

Unless otherwise defined, all terms used in disclosing the invention, including technical and scientific terms, have the meaning as commonly understood by one of ordinary skill in the art to which this invention belongs. By means of further guidance, term definitions are included to better appreciate the teaching of the invention. When specific terms are defined in connection with a particular aspect of the invention or a particular embodiment of the invention, such connotation is meant to apply throughout this specification, i.e., also in the context of other aspects or embodiments of the invention, unless otherwise defined.

In the following passages, different aspects or embodiments of the invention are defined in more detail. Each aspect or embodiment so defined may be combined with any other aspect(s) or embodiment(s) unless clearly indicated to the contrary. In particular, any feature indicated as being preferred or advantageous may be combined with any other feature or features indicated as being preferred or advantageous.

Reference throughout this specification to "one embodiment", "an embodiment" means that a particular feature, structure or characteristic described in connection with the embodiment is included in at least one embodiment of the present invention. Thus, appearances of the phrases "in one embodiment" or "in an embodiment" in various places throughout this specification are not necessarily all referring to the same embodiment, but may. Furthermore, the particular features, structures or characteristics may be combined in any suitable manner, as would be apparent to a person skilled in the art from this disclosure, in one or more embodiments. Furthermore, while some embodiments described herein include some but not other features included in other embodiments, combinations of features of different embodiments are meant to be within the scope of the invention, and form different embodiments, as would be understood by those in the art. For example, in the appended claims, any of the claimed embodiments can be used in any combination.

The present inventors have identified a set of biomarkers which are of particular interest for the diagnosis of lung cancer, particularly early stage lung cancer. The expression of the biomarkers Rho GDP dissociation inhibitor beta (ARHGDIB), alpha-tubulin 4A (TUBA4A), glutathione S-transferase omega 1 (GSTO1), filamin A (FLNA), peroxiredoxin 6 (PRDX6) and cadherin 13 (CDH13) have been found to be particularly correlated with the occurrence of lung cancer and thus are suitable for diagnosis both individually and as part of a panel. While the detection of one of these markers already provides an important indication in many cases, the combination of two or more markers increases the accuracy and sensitivity of the diagnosis. Indeed, the inventors have found that the detection of at least one, preferably at least two or three, more preferably five or all six biomarker(s) selected from the group consisting of Rho GDP dissociation inhibitor beta (ARHGDIB), alpha-tubulin 4A (TUBA4A), glutathione S-transferase omega 1 (GSTO1), filamin A (FLNA), peroxiredoxin 6 (PRDX6) and cadherin 13 (CDH13) in a biological sample from a subject allows to accurately distinguish lung cancer patients from individuals who do not have lung cancer. The at least one biomarker selected from the group consisting of ARHGDIB, TUBA4A, GSTO1, FLNA, PRDX6 and CDH13 has an excellent performance in diagnosing lung cancer in a biological sample from a subject. This is supported by excellent performance measures including AUC, PPV, NPV, sensitivity and specificity. More particularly, the sensitivity of the present method is better than the described sensitivity of the blood test CancerSEEK (Cohen JD, Li L, Wang Y, Thoburn C, Afsari B, Danilova L, et al. Detection and localization of surgically resectable cancers with a multi-analyte blood test. Science. 2018; 359: 926-30) for lung cancer diagnosis.

Compared to imaging techniques, such as Lung cancer screening using low-dose CT (LDCT), the method as taught herein, which is a highly sensitive and highly specific method, reduces the percentage of false positive results (i.e. overdiagnosis rate) and avoids exposure of the tested subjects to radiation. The reduction of false positive cases also avoids additional invasive tests and unnecessary costs, as well as the exposure of the patients to physical and mental hardships. Furthermore, the use of one or more biomarker selected from the group consisting of ARHGDIB, TUBA4A, GSTO1, FLNA, PRDX6 and CDH13 allows to detect lung cancer in a non-invasive manner as the biomarker(s) can be detected in body fluid samples, such as in plasma samples. Moreover, the testing for one or more biomarker(s) selected from the group consisting of ARHGDIB, TUBA4A, GSTO1, FLNA, PRDX6 and CDH13 also allows to detect lung cancer independently of the disease stage, including stage I lung tumors. The earlier diagnosis of cancer during a regular screening offers the patients immediate treatment solutions. In addition, in order to make the present biomarker(s) easy to use by medical practitioners, present inventors have further also adopted a threshold-based approach, attributing a threshold value per biomarker, and optionally a score per sample, to classify the subject as having lung cancer or not.

In a first aspect, the present invention provides an *in vitro* method for diagnosing lung cancer in a subject, wherein the method comprises detecting Rho GDP dissociation inhibitor beta (ARHGDIB) and at least one biomarker (such as one, two, three, four or all five), such as at least two (such as two, three, four, five or six), at least three (such as three, four, five or six), at least four (such as four, five or six), or all five biomarkers, selected from the group consisting of alpha-tubulin 4A (TUBA4A), glutathione S-transferase omega 1 (GSTO1), filamin A (FLNA), peroxiredoxin 6 (PRDX6) and cadherin 13 (CDH13) in a biological sample from the subject.

Also disclosed herein but not specifically claimed is the use, preferably *in vitro* or *ex vivo* use, of at least one, such as at least two, at least three, at least four, at least five or all six, biomarker selected from the group consisting of ARHGDIB, TUBA4A, GSTO1, FLNA, PRDX6 and CDH13 as a biomarker for lung cancer in a subject.

The term "biomarker" is widespread in the art and may broadly denote a biological molecule and/or a detectable portion thereof whose qualitative and/or quantitative evaluation in a subject is predictive or informative (e.g., predictive, diagnostic and/or prognostic) with respect to one or more aspects of the subject's phenotype and/or genotype, such as, for example, with respect to the status of the subject as to a given disease or condition. Reference is made herein to a "biomarker panel" if more than one biomarker is being detected in the methods or uses as taught herein.

In certain embodiments, a biomarker as taught herein may be peptide-, polypeptide- and/or protein-based.

The reference to any marker, including any peptide, polypeptide or protein, corresponds to the marker, peptide, polypeptide, protein, commonly known under the respective designations in the art. The terms encompass such markers, peptides, polypeptides, or proteins of any organism where found, and particularly of animals, preferably warm-blooded animals, more preferably vertebrates, yet more preferably mammals, including humans and non-human mammals, still more preferably of humans. The terms particularly encompass such markers, peptides, polypeptides, or proteins, with a native sequence, i.e., ones of which the primary sequence is the same as that of the markers, peptides, polypeptides, or proteins found in or derived from nature. A skilled person understands that native sequences may differ between different species due to genetic divergence between such species. Moreover, native sequences may differ between or within different individuals of the same species due to normal genetic diversity (variation) within a given species. Also, native sequences may differ between or even within different individuals of the same species due to post-transcriptional or post-translational modifications. Any such variants or isoforms of markers, peptides, polypeptides or proteins are intended herein. Accordingly, all sequences of markers, peptides, polypeptides, or proteins found in or derived from nature are considered "native". The terms encompass the markers, peptides, polypeptides or proteins when forming a part of a living organism, organ, tissue or cell, when forming a part of a biological sample, as well as when at least partly isolated from such sources. The terms also encompass markers, peptides, polypeptides or proteins when produced by recombinant or synthetic means.

In certain embodiments, a biomarker as taught herein may be a human biomarker, such as human ARHGDIB (also known as Rho GDP-dissociation inhibitor 2 (RhoGDI2)), human TUBA4A, human GSTO1, human FLNA, human PRDX6 or human CDH13.

By means of an example, protein sequence of human ARHGDIB is annotated under NCBI Genbank (http://www.ncbi.nlm.nih.gov/) accession number NP_001308351.1 and under UniProtKB/Swiss-prot number P52566.3; protein sequence of human TUBA4A is annotated under NCBI Genbank accession number NP_005991.1 and under UniProtKB/Swiss-prot number P68366.1; protein sequence of human GSTO1 is annotated under NCBI Genbank accession number NP_004823.1 and under UniProtKB/Swiss-prot number P78417.2; protein sequence of human FLNA is annotated under NCBI Genbank accession number NP_001447.2 and under UniProtKB/Swiss-prot number P21333.4; protein sequence of human PRDX6 is annotated under NCBI Genbank accession number NP_004896.1 and under UniProtKB/Swiss-prot number P30041.3; and protein sequence of human CDH13 is annotated under NCBI Genbank accession number NP_001248.1 and under UniProtKB/Swiss-prot number P55290.1.

Unless otherwise apparent from the context, reference herein to any marker, peptide, polypeptide or protein, or fragment thereof may generally also encompass modified forms of said marker, peptide, polypeptide, or protein, or fragment thereof, such as bearing post-expression modifications including, for example, phosphorylation, glycosylation, lipidation, methylation, cysteinylation, sulphonation, glutathionylation, acetylation, oxidation of methionine to methionine sulphoxide or methionine sulphone, and the like.

The reference herein to any marker, peptide, polypeptide or protein also encompasses fragments thereof. Hence, the reference herein to measuring (or measuring the quantity of) any one marker, peptide, polypeptide or protein may encompass measuring the marker, peptide, polypeptide or protein, such as, e.g., measuring any mature and/or processed soluble/secreted form(s) thereof (e.g., plasma circulating form(s)) and/or measuring one or more fragments thereof.

For example, any marker, peptide, polypeptide or protein, and/or one or more fragments thereof may be measured collectively, such that the measured quantity corresponds to the sum amounts of the collectively measured species. In a further example, any marker, peptide, polypeptide or protein, and/or one or more fragments thereof may be measured each individually.

The term "fragment" with reference to a peptide, polypeptide, or protein generally denotes a N- and/or C-terminally truncated form of the peptide, polypeptide, or protein. Preferably, a fragment may comprise at least about 30%, e.g., at least about 50% or at least about 70%, preferably at least about 80%, e.g., at least about 85%, more preferably at least about 90%, and yet more preferably at least about 95% or even about 99% of the amino acid sequence length of said peptide, polypeptide, or protein. For example, insofar not exceeding the length of the full-length peptide, polypeptide, or protein, a fragment may include a sequence of ≥ 5 consecutive amino acids, or ≥ 10 consecutive amino acids, or ≥ 20 consecutive amino acids, or ≥ 30 consecutive amino acids, e.g., ≥40 consecutive amino acids, such as for example ≥ 50 consecutive amino acids, e.g., ≥ 60, ≥ 70, ≥ 80, ≥ 90, ≥ 100, ≥ 200, ≥ 300 or ≥ 400 consecutive amino acids of the corresponding full-length peptide, polypeptide, or protein.

For example, the biomarker may be measured by measuring a peptide fragment of the full length protein. For example, ARHGDIB may be measured by measuring YVQHTYR (SEQ ID NO: 1), TUBA4A may be measured by measuring EIIDPVLDR (SEQ ID NO: 2), GSTO1 may be measured by measuring GSAPPGPVPEGSIR (SEQ ID NO: 3), FLNA may be measured by measuring SPFSVAVSPSLDLSK (SEQ ID NO: 4), PRDX6 may be measured by measuring LSILYPATTGR (SEQ ID NO: 5) and CDH13 may be measured by measuring SIVVSPILIPENQR (SEQ ID NO: 6). Peptide YVQHTYR (SEQ ID NO: 1) typically has a mass/charge of 483.74 m/z, peptide EIIDPVLDR (SEQ ID NO: 2) typically has a mass/charge of 535.30 m/z, peptide GSAPPGPVPEGSIR (SEQ ID NO: 3) typically has a mass/charge of 660.85 m/z, peptide SPFSVAVSPSLDLSK (SEQ ID NO: 4) typically has a mass/charge of 767.41 m/z, peptide LSILYPATTGR (SEQ ID NO: 5) typically has a mass/charge of 596.34 m/z and peptide SIVVSPILIPENQR (SEQ ID NO: 6) typically has a mass/charge of 782.96 m/z.

The reference herein to any protein, polypeptide or peptide may also encompass variants thereof. The term "variant" of a protein, polypeptide or peptide refers to proteins, polypeptides or peptides the sequence (i.e., amino acid sequence) of which is substantially identical (i.e., largely but not wholly identical) to the sequence of said recited protein or polypeptide, e.g., at least about 80% identical or at least about 85% identical, e.g., preferably at least about 90% identical, e.g., at least 91% identical, 92% identical, more preferably at least about 93% identical, e.g., at least 94% identical, even more preferably at least about 95% identical, e.g., at least 96% identical, yet more preferably at least about 97% identical, e.g., at least 98% identical, and most preferably at least 99% identical. Preferably, a variant may display such degrees of identity to a recited protein, polypeptide or peptide when the whole sequence of the recited protein, polypeptide or peptide is queried in the sequence alignment (i.e., overall sequence identity).

Sequence identity may be determined using suitable algorithms for performing sequence alignments and determination of sequence identity as know *per se.* Exemplary but non-limiting algorithms include those based on the Basic Local Alignment Search Tool (BLAST) originally described by Altschul et al. 1990 (J Mol Biol 215: 403-10), such as the "Blast 2 sequences" algorithm described by Tatusova and Madden 1999 (FEMS Microbiol Lett 174: 247-250), for example using the published default settings or other suitable settings (such as, *e.g.,* for the BLASTN algorithm: cost to open a gap = 5, cost to extend a gap = 2, penalty for a mismatch = -2, reward for a match = 1, gap x_dropoff = 50, expectation value = 10.0, word size = 28; or for the BLASTP algorithm: matrix = Blosum62, cost to open a gap = 11, cost to extend a gap = 1, expectation value = 10.0, word size = 3).

A variant of a protein, polypeptide or peptide may be a homologue (e.g., orthologue or paralogue) of said protein, polypeptide or peptide. As used herein, the term "homology" generally denotes structural similarity between two macromolecules, particularly between two proteins or polypeptides, from same or different taxons, wherein said similarity is due to shared ancestry.

Where the present specification refers to or encompasses fragments and/or variants of proteins, polypeptides or peptides, this preferably denotes variants and/or fragments which are "functional", i.e., which at least partly retain the biological activity or intended functionality of the respective proteins, polypeptides or peptides. Preferably, a functional fragment and/or variant may retain at least about 20%, e.g., at least 30%, or at least about 40%, or at least about 50%, e.g., at least 60%, more preferably at least about 70%, e.g., at least 80%, yet more preferably at least about 85%, still more preferably at least about 90%, and most preferably at least about 95% or even about 100% or higher of the intended biological activity or functionality compared to the corresponding protein, polypeptide or peptide.

Also disclosed herein but not specifically claimed is an *in vitro* method for diagnosing lung cancer in a subject, wherein the method comprises detecting at least one, such as at least two, at least three, at least four, at least five or all six, biomarkers selected from the group consisting of ARHGDIB, TUBA4A, GSTO1, FLNA, PRDX6 and CDH13 in a biological sample from the subject.

Also disclosed herein but not claimed is a method of diagnosing and treating lung cancer in a subject, comprising

(a) detecting at least one, such as at least two, at least three, at least four, at least five or all six, biomarkers selected from the group consisting of ARHGDIB, TUBA4A, GSTO1, FLNA, PRDX6 and CDH13 in a biological sample from the subject;
(b) diagnosing lung cancer in the subject based on the detection of said at least one biomarker; and
(c) administering to said subject diagnosed with lung cancer a therapy for lung cancer, such as administering to said subject an effective amount of a therapeutic agent for lung cancer.

The individual biomarkers of the group consisting of ARHGDIB, TUBA4A, GSTO1, FLNA, PRDX6 and CDH13, and especially ARHGDIB, have an excellent predictive power for distinguishing lung cancer patients from healthy subjects.

More particularly, the performance metrics of ARHGDIB were excellent: a NPV of > 0.90, a sensitivity of > 0.90 and an AUC of ≥ 0.90 in the test cohort and a NPV of ≥ 0.90 and a sensitivity of ≥ 0.90 in the validation cohort. However ARHGDIB can be used in a panel with other known lung cancer biomarkers. Also disclosed herein but not specifically claimed is an *in vitro* method for diagnosing lung cancer in a subject comprises detecting at least two (such as two, three, four, five or six), at least three (such as three, four, five or six), at least four (such as four, five or six), at least five (such as five or six), or all six biomarkers, selected from the group consisting ARHGDIB, TUBA4A, GSTO1, FLNA, PRDX6 and CDH13 in a biological sample from the subject.

Present inventors have shown that biomarker combinations comprising CDH13 and/or ARHGDIB show particularly good performance measures for distinguishing lung cancer patients from healthy subjects. Accordingly, also disclosed herein but not specifically claimed is an *in vitro* method for diagnosing lung cancer in a subject comprising detecting a first biomarker selected from the group consisting of CDH13 and ARHGDIB, and optionally at least one (such as one, two, three, four or all five) additional biomarker selected from the group consisting of ARHGDIB, TUBA4A, GSTO1, FLNA and PRDX6, if the first biomarker is CDH13, and at least one (such as one, two, three, four or all five) additional biomarker selected from the group consisting of CDH13, TUBA4A, GSTO1, FLNA and PRDX6, if the first biomarker is ARHGDIB in a biological sample from the subject.

Of the biomarker combinations comprising CDH13 and/or ARHGDIB, 35 biomarker combinations show excellent performance metrics: a sensitivity ≥ 0.90 and a specificity of > 0.90 or a NPV of ≥0.90. Therefore, the *in vitro* method for diagnosing lung cancer in a subject may comprise detecting a biomarker panel comprising biomarkers (i) FLNA, TUBA4A, GSTO1, PRDX6, ARHGDIB and CDH13; (ii) FLNA, TUBA4A, GSTO1, PRDX6 and ARHGDIB; (iii) FLNA, TUBA4A, GSTO1, ARHGDIB and CDH13; (iv) FLNA, TUBA4A, PRDX6, ARHGDIB and CDH13; (v) FLNA, GSTO1, PRDX6, ARHGDIB and CDH13; (vi) TUBA4A, GSTO1, PRDX6, ARHGDIB and CDH13; (vii) FLNA, TUBA4A, GSTO1 and ARHGDIB; (viii) FLNA, TUBA4A, GSTO1 and CDH13 (not claimed); (ix) FLNA, TUBA4A, PRDX6 and ARHGDIB;

(x) FLNA, TUBA4A, ARHGDIB and CDH13; (xi) FLNA, GSTO1, PRDX6 and ARHGDIB; (xii) FLNA, GSTO1, ARHGDIB and CDH13; (xiii) FLNA, PRDX6, ARHGDIB and CDH13; (xiv) TUBA4A, GSTO1, PRDX6 and ARHGDIB; (xv) TUBA4A, GSTO1, ARHGDIB and CDH13; (xvi) TUBA4A, PRDX6, ARHGDIB and CDH13; (xvii) GSTO1, PRDX6, ARHGDIB and CDH13; (xviii) FLNA, TUBA4A and ARHGDIB; (xix) FLNA, GSTO1 and ARHGDIB; (xx) FLNA, PRDX6 and ARHGDIB; (xxi) FLNA, ARHGDIB and CDH13; (xxii) TUBA4A, GSTO1 and ARHGDIB; (xxiii) TUBA4A, GSTO1 and CDH13 (not claimed); (xxiv) TUBA4A, PRDX6 and ARHGDIB; (xxv) TUBA4A, PRDX6 and CDH13 (not claimed); (xxvi) TUBA4A, ARHGDIB and CDH13; (xxvii) GSTO1, PRDX6 and ARHGDIB; (xxviii) GSTO1, ARHGDIB and CDH13; (xxix) PRDX6, ARHGDIB and CDH13; (xxx) FLNA and ARHGDIB; (xxxi) TUBA4A and ARHGDIB; (xxxii) GSTO1 and ARHGDIB; (xxxiii) GSTO1 and CDH13 (not claimed); (xxxiv) PRDX6 and ARHGDIB; or (xxxv)ARHGDIB and CDH13, in a biological sample from the subject.

Of the 35 biomarker combinations comprising CDH13 and/or ARHGDIB, 6 biomarker combinations show even better performance metrics: a sensitivity ≥ 0.90, a specificity of ≥ 0.90 and a NPV of ≥0.90. Accordingly, the *in vitro* method for diagnosing lung cancer in a subject may comprise detecting a biomarker panel comprising biomarkers FLNA, TUBA4A, GSTO1 and CDH13 (not claimed); FLNA, ARHGDIB and CDH13; TUBA4A, GSTO1 and CDH13 (not claimed); TUBA4A, PRDX6 and CDH13 (not claimed); TUBA4A, ARHGDIB and CDH13; or GSTO1 and CDH13 (not claimed), in a biological sample from the subject.

Also disclosed herein but not specifically claimed is an *in vitro* method for diagnosing lung cancer in a subject comprising detecting CDH13 and at least one, such as one, two, three, four or all five, biomarker selected from the group consisting of ARHGDIB, TUBA4A, GSTO1, FLNA and PRDX6 in a biological sample from the subject. For example, such *in vitro* method for diagnosing lung cancer in a subject may comprise detecting a biomarker panel comprising biomarkers (i) CDH13, FLNA, TUBA4A, GSTO1, PRDX6 and ARHGDIB; CDH13, FLNA, TUBA4A, GSTO1 and ARHGDIB; (ii) CDH13, FLNA, TUBA4A, PRDX6 and ARHGDIB; (iii) CDH13, FLNA, GSTO1, PRDX6 and ARHGDIB; (iv) CDH13, TUBA4A, GSTO1, PRDX6 and ARHGDIB; (v) CDH13, FLNA, TUBA4A and GSTO1 (not claimed); (vi) CDH13, FLNA, TUBA4A and ARHGDIB; (vii) CDH13, FLNA, GSTO1 and ARHGDIB; (viii) CDH13, FLNA, PRDX6 and ARHGDIB; (ix) CDH13, TUBA4A, GSTO1 and ARHGDIB; (x) CDH13, TUBA4A, PRDX6 and ARHGDIB; (xi) CDH13, GSTO1, PRDX6 and ARHGDIB; (xii) CDH13, FLNA and ARHGDIB; (xiii) CDH13, TUBA4A and GSTO1 (not claimed); (xiv) CDH13, TUBA4A and PRDX6 (not claimed); (xv) CDH13, TUBA4A and ARHGDIB; (xvi) CDH13, GSTO1 and ARHGDIB; (xvii) CDH13, PRDX6 and ARHGDIB; (xviii) CDH13 and GSTO1 (not claimed); (xvix) CDH13 and ARHGDIB, preferably FLNA, TUBA4A, GSTO1 and CDH13 (not claimed); FLNA, ARHGDIB and CDH13; TUBA4A, GSTO1 and CDH13; TUBA4A, PRDX6 and CDH13 (not claimed); TUBA4A, ARHGDIB and CDH13; or GSTO1 and CDH13 (not claimed), in a biological sample from the subject. Also disclosed herein but not specifically claimed is an *in vitro* method for diagnosing lung cancer in a subject comprising detecting ARHGDIB in a biological sample from the subject. Also disclosed herein but not specifically claimed is an *in vitro* method for diagnosing lung cancer in a subject comprising detecting GSTO1, optionally in combination with at least one, preferably two biomarkers selected from the group consisting of TUBA4A, ARHGDIB, FLNA, and CDH13. Also disclosed herein but not specifically claimed is an *in vitro* method for diagnosing lung cancer in a subject comprising detecting CDH13, optionally in combination with at least one, preferably two biomarkers selected from the group consisting of TUBA4A, ARHGDIB, FLNA, PRDX6 and GSTO1. Also disclosed herein but not specifically claimed are methods which involve the detection of CDH13 and GSTO1 optionally in combination with one or more biomarkers selected from the group consisting of TUBA4A, ARHGDIB, FLNA and PRDX6.

In further particular embodiments, the *in vitro* method for diagnosing lung cancer in a subject comprises detecting a biomarker panel comprising biomarkers (i) ARHGDIB, FLNA, TUBA4A, GSTO1, PRDX6 and CDH13; (ii) ARHGDIB, FLNA, TUBA4A, GSTO1 and PRDX6; (iii) ARHGDIB, FLNA, TUBA4A, GSTO1 and CDH13; (iv) ARHGDIB, FLNA, TUBA4A, PRDX6 and CDH13; (v) ARHGDIB, FLNA, GSTO1, PRDX6 and CDH13; (vi) ARHGDIB, TUBA4A, GSTO1, PRDX6 and CDH13; (vii) ARHGDIB, FLNA, TUBA4A and GSTO1; (viii) ARHGDIB, FLNA, TUBA4A and PRDX6; (ix) ARHGDIB, FLNA, TUBA4A and CDH13; (x) ARHGDIB, FLNA, GSTO1 and PRDX6; (xi) ARHGDIB, FLNA, GSTO1 and CDH13; (xii) ARHGDIB, FLNA, PRDX6 and CDH13; (xiii) ARHGDIB, TUBA4A, GSTO1 and PRDX6; (xiv) ARHGDIB, TUBA4A, GSTO1 and CDH13; (xv) ARHGDIB, TUBA4A, PRDX6 and CDH13; (xvi) ARHGDIB, GSTO1, PRDX6 and CDH13; (xvii) ARHGDIB, FLNA and TUBA4A; (xviii) ARHGDIB, FLNA and GSTO1; (xix) ARHGDIB, FLNA and PRDX6; (xx) ARHGDIB, FLNA and CDH13; (xxi) ARHGDIB, TUBA4A and GSTO1; (xxii) ARHGDIB, TUBA4A and PRDX6; (xxiii) ARHGDIB, TUBA4A and CDH13; (xxiv) ARHGDIB, GSTO1 and PRDX6; (xxv) ARHGDIB, GSTO1 and CDH13; (xxvi) ARHGDIB, PRDX6 and CDH13; (xxvii) ARHGDIB and FLNA; (xxviii) ARHGDIB and TUBA4A; (xxvix) ARHGDIB and GSTO1; (xxx) ARHGDIB and PRDX6; or (xxxi) ARHGDIB and CDH13, preferably ARHGDIB, FLNA and CDH13; or ARHGDIB, TUBA4A and CDH13, in a biological sample from the subject. Alternatively envisaged panels are those additionally described for the methods described above.

Present inventors identified, in addition to the potent individual biomarkers, a 6-protein panel that displays excellent discriminative power in the logistic regression model, especially when compared to the commercially available Xpresys® Lung (XL) test (Biodesix, Boulder, CO) and univariable models: the lowest AIC (30.876), the highest AUC (0.999), the highest PPV (0.992), the highest NPV (0.989), the highest specificity (0.989) and the highest sensitivity (0.992) in the test cohort. In addition, the 6-protein panel allows to detect non-invasively lung cancer independently of the disease stage (including stage I tumors). Accordingly, the 6-protein panel has a particularly high potential as a screening tool.

Hence, in particular embodiments, the *in vitro* method for diagnosing lung cancer in a subject comprises detecting ARHGDIB, TUBA4A, GSTO1, FLNA, PRDX6 and CDH13 in a biological sample from the subject.

A molecule or analyte such as a marker, peptide, polypeptide, or protein, is "detected" in a sample when the presence or absence and/or quantity of said molecule or analyte is detected or determined in the sample, preferably substantially to the exclusion of other molecules and analytes.

Depending on factors that can be evaluated and decided on by a skilled person, such as *inter alia* the type of a biomarker (e.g., peptide, polypeptide, or protein), the type of a sample (e.g., whole blood, plasma, serum, lung tissue biopsy, histological section), the expected abundance of the biomarker in the sample, the type, robustness, sensitivity and/or specificity of the detection method used to detect the biomarker, etc., the biomarker may be measured directly in the sample, or the sample may be subjected to one or more processing steps aimed at achieving an adequate measurement of the biomarker.

By means of example, the sample may be subjected to one or more isolation or separation steps, aimed at whereby the biomarker is isolated from the sample or whereby a fraction of the sample is prepared which is enriched for the biomarker. For example, if the biomarker is a peptide, polypeptide, or protein, any known protein purification technique may be applied to the sample to isolate peptides, polypeptides, and proteins therefrom. Non-limiting examples of methods to purify peptides, polypeptides, or proteins, may include chromatography, preparative electrophoresis, centrifugation, precipitation, affinity purification, etc.

As used herein, the term "purified" with reference to markers, peptides, polypeptides, or proteins does not require absolute purity. Instead, it denotes that such markers, peptides, polypeptides, or proteins, are in a discrete environment in which their abundance (conveniently expressed in terms of mass or weight or concentration) relative to other analytes is greater than in the biological sample. A discrete environment denotes a single medium, such as for example a single solution, gel, precipitate, lyophilisate, etc. Purified proteins, polypeptides or peptides may be obtained by known methods including, for example, laboratory or recombinant synthesis, chromatography, preparative electrophoresis, centrifugation, precipitation, affinity purification, *etc.*

Purified markers, peptides, polypeptides, or proteins may preferably constitute by weight $\geq$ 10%, more preferably $\geq$ 50%, such as $\geq$ 60%, yet more preferably $\geq$ 70%, such as $\geq$ 80%, and still more preferably $\geq$ 90%, such as $\geq$ 95%, $\geq$ 96%, $\geq$ 97%, $\geq$ 98%, $\geq$ 99% or even 100%, of the protein content of the discrete environment. Protein content may be determined, e.g., by the Lowry method (Lowry et al. 1951. J Biol Chem 193: 265), optionally as described by Hartree 1972 (Anal Biochem 48: 422-427). Purity of peptides, polypeptides, or proteins may be determined by SDS-PAGE under reducing or non-reducing conditions using Coomassie blue or, preferably, silver stain.

Any existing, available or conventional separation, detection and quantification methods may be used herein to measure the presence or absence (e.g., readout being present vs. absent; or detectable amount vs. undetectable amount) and/or quantity (e.g., readout being an absolute or relative quantity, such as, for example, absolute or relative concentration) of markers, peptides, polypeptides, or proteins in samples.

For example, such methods may include mass spectrometry analysis methods, biochemical assay methods, immunoassay methods, or chromatography methods, or combinations thereof.

The term "immunoassay" generally refers to methods known as such for detecting one or more molecules or analytes of interest in a sample, wherein specificity of an immunoassay for the molecule(s) or analyte(s) of interest is conferred by specific binding between a specific-binding agent, commonly but without limitation an antibody, and the molecule(s) or analyte(s) of interest. Immunoassay technologies include without limitation immunohistochemistry, direct ELISA (enzyme-linked immunosorbent assay), indirect ELISA, sandwich ELISA, competitive ELISA, multiplex ELISA, radioimmunoassay (RIA), ELISPOT technologies, and other similar techniques known in the art.

Generally, any mass spectrometric (MS) techniques that are capable of obtaining precise information on the mass of peptides, and preferably also on fragmentation and/or (partial) amino acid sequence of selected peptides (e.g., in tandem mass spectrometry, MS/MS; or in post source decay, TOF MS), are useful herein. Suitable peptide MS and MS/MS techniques and systems are well-known *per se* (see, *e.g.,* Methods in Molecular Biology, vol. 146: "Mass Spectrometry of Proteins and Peptides", by Chapman, ed., Humana Press 2000, ISBN 089603609x; Biemann 1990. Methods Enzymol 193: 455-79; or Methods in Enzymology, vol. 402: "Biological Mass Spectrometry", by Burlingame, ed., Academic Press 2005, ISBN 9780121828073) and may be used herein. MS peptide analysis methods may be advantageously combined with upstream peptide or protein separation or fractionation methods, such as for example with chromatography. The data obtained from MS may be processed using software for data analysis of proteomics and/or metabolomics known in the art, such as the Skyline software (v19.1.0.193).

Chromatography may also be used for measuring biomarkers. As used herein, the term "chromatography" encompasses methods for separating chemical substances, referred to as such and vastly available in the art. Chromatography as used herein may be preferably columnar (i.e., wherein the stationary phase is deposited or packed in a column), preferably liquid chromatography, and yet more preferably HPLC. While particulars of chromatography are well known in the art, for further guidance see, e.g., Meyer M., 1998, ISBN: 047198373X, and "Practical HPLC Methodology and Applications", Bidlingmeyer, B. A., John Wiley & Sons Inc., 1993.

Further peptide or polypeptide separation, identification or quantification methods may be used, optionally in conjunction with any of the above described analysis methods, for measuring biomarkers in the present disclosure. Such methods include, without limitation, chemical extraction partitioning, isoelectric focusing (IEF) including capillary isoelectric focusing (CIEF), capillary isotachophoresis (CITP), capillary electrochromatography (CEC), and the like, one-dimensional polyacrylamide gel electrophoresis (PAGE), two-dimensional polyacrylamide gel electrophoresis (2D-PAGE), capillary gel electrophoresis (CGE), capillary zone electrophoresis (CZE), micellar electrokinetic chromatography (MEKC), free flow electrophoresis (FFE), etc.

Present inventors found that each of the biomarkers ARHGDIB, TUBA4A, GSTO1, FLNA, PRDX6 and CDH13 can be detected in an antibody-independent method, more particularly using parallel reaction monitoring (PRM)-based mass spectrometry, for example as described in Bourmaud A, Gallien S, Domon B. Parallel reaction monitoring using quadrupole-Orbitrap mass spectrometer: Principle and applications. Proteomics. 2016; 16: 2146-59..

In particular embodiments, the biomarkers are detected using mass spectrometry, preferably liquid chromatography-mass spectrometry (LC-MS). For example, the LC-MS may be performed using a LC-MS setup consisting of a Dionex U3000 RSLC liquid chromatography system operated in column switching mode coupled with Q Exactive Plus mass spectrometer.

The person skilled in the art will understand that prior to mass spectrometry the biological sample may be depleted and processed, e.g. (ultra)filtrated, denaturated, alkylated, digested and/or deglycosylated. In particular embodiments, detecting ARHGDIB and at least one biomarker selected from the group consisting of TUBA4A, GSTO1, FLNA, PRDX6 and CDH13 in a biological sample from the subject comprises measuring the quantity or expression level of said biomarkers in the biological sample from the subject.

The terms "quantity", "amount" and "level" are synonymous and generally well-understood in the art. The terms as used herein may particularly refer to an absolute quantification of a molecule or an analyte in a sample, or to a relative quantification of a molecule or analyte in a sample, i.e., relative to another value such as relative to a reference value as taught herein, or to a range of values indicating a base-line expression of the biomarker. These values or ranges can be obtained from a single patient or from a group of patients.

An absolute quantity of a molecule or analyte in a sample may be advantageously expressed as weight or as molar amount, or more commonly as a concentration, e.g., weight per volume or mol per volume. A relative quantity of a molecule or analyte in a sample may be advantageously expressed as an increase or decrease or as a fold-increase or fold-decrease relative to said another value, such as relative to a reference value as taught herein. Performing a relative comparison between first and second parameters (e.g., first and second quantities) may but need not require first to determine the absolute values of said first and second parameters. For example, a measurement method can produce quantifiable readouts (such as, e.g., signal intensities) for said first and second parameters, wherein said readouts are a function of the value of said parameters, and wherein said readouts can be directly compared to produce a relative value for the first parameter vs. the second parameter, without the actual need first to convert the readouts to absolute values of the respective parameters.

The terms "quantity" and "expression level" of said at least one biomarker selected from the group consisting of ARHGDIB, TUBA4A, GSTO1, FLNA, PRDX6 and CDH13 are used interchangeably in this specification to refer to the absolute and/or relative quantification, concentration level or amount of any such product in a sample. Preferably, the biomarker is a protein and the term "expression level" refers to the "protein expression level".

Present inventors found that biomarkers TUBA4A, GSTO1, FLNA, PRDX6, ARHGDIB and/or CDH13 are significantly differentially expressed in a biological sample, preferably a plasma sample, from patients suffering from lung cancer compared to healthy subjects.

In particular embodiments, the methods as taught herein may comprise the step of comparing the quantity or expression level of ARHGDIB and said at least one biomarker selected from the group consisting of TUBA4A, GSTO1, FLNA, PRDX6, and CDH13 in the biological sample of the subject with a reference or threshold quantity or expression level of said respective biomarker. Said reference or threshold quantity or expression level of said at least one biomarker may represent a known diagnosis of lung cancer.

In particular embodiments, the method comprises the steps of

(a) measuring the quantity or expression level of ARHGDIB and at least one, such as at least two, at least three, at least four, or all five, biomarker selected from the group consisting of TUBA4A, GSTO1, FLNA, PRDX6, and CDH13 in the biological sample from the subject;
(b) comparing the quantity or expression level of said biomarkers as measured in (a) with a reference value or a threshold value, said reference value or threshold value representing a known diagnosis of lung cancer;
(c) finding a deviation or no deviation of the quantity or expression level of said biomarkers as measured in (a) from said reference value or threshold value; and
(d) attributing said finding of deviation or no deviation to a particular diagnosis of lung cancer.

Also disclosed herein but not claimed is a method for diagnosing and treating lung cancer in a subject comprising:

(a) measuring the quantity or expression level of at least one, such as at least two, at least three, at least four, at least five or all six, biomarker selected from the group consisting of TUBA4A, GSTO1, FLNA, PRDX6, ARHGDIB and CDH13 in the biological sample from the subject;
(b) comparing the quantity or expression level of said at least one biomarker as measured in (a) with a reference value or a threshold value, said reference value or threshold value representing a known diagnosis of lung cancer;
(c) diagnosing lung cancer in the subject or diagnosing the subject as in need of treatment for lung cancer when said quantity or expression level of said at least one biomarker as measured in (a) deviates from said reference value or threshold value; and
(d) administering to said subject diagnosed with lung cancer a therapy or treatment for lung cancer, such as administering to said subject an effective amount of a therapeutic agent for lung cancer.

Present inventors found increased levels TUBA4A, GSTO1, FLNA, PRDX6 and ARHGDIB and decreased levels of CDH13 in biological samples, preferably plasma samples, from lung cancer patients (independently of the lung cancer stage) compared to healthy subjects.

Accordingly, in particular embodiments, if the reference value or threshold value represents a subject or a group of subjects not affected by lung cancer,

- an elevated quantity or expression level of ARHGDIB (e.g. elevated by at least about 10% (about 1.1-fold or more), or by at least about 20% (about 1.2-fold or more), or by at least about 30% (about 1.3-fold or more), or by at least about 40% (about 1.4-fold or more), or by at least about 50% (about 1.5-fold or more), or by at least about 60% (about 1.6-fold or more), or by at least about 70% (about 1.7-fold or more), or by at least about 80% (about 1.8-fold or more), or by at least about 90% (about 1.9-fold or more), or by at least about 100% (about 2-fold or more), or by at least about 400% (about 5-fold or more), or by at least about 900% (about 10-fold or more)) in the sample of the subject as compared to the reference value or threshold value;
- an elevated quantity or expression level of TUBA4A (e.g. elevated by at least about 10% (about 1.1-fold or more), or by at least about 20% (about 1.2-fold or more), or by at least about 30% (about 1.3-fold or more), or by at least about 40% (about 1.4-fold or more), or by at least about 50% (about 1.5-fold or more), or by at least about 60% (about 1.6-fold or more), or by at least about 70% (about 1.7-fold or more), or by at least about 80% (about 1.8-fold or more), or by at least about 90% (about 1.9-fold or more), or by at least about 100% (about 2-fold or more), or by at least about 200% (about 3-fold or more), or by at least about 300% (about 4-fold or more), or by at least about 400% (about 5-fold or more)) in the sample of the subject as compared to the reference value or threshold value;
- an elevated quantity or expression level of GSTO1 (e.g. elevated by at least about 10% (about 1.1-fold or more), or by at least about 20% (about 1.2-fold or more), or by at least about 30% (about 1.3-fold or more), or by at least about 40% (about 1.4-fold or more), or by at least about 50% (about 1.5-fold or more), or by at least about 60% (about 1.6-fold or more), or by at least about 70% (about 1.7-fold or more), or by at least about 80% (about 1.8-fold or more), or by at least about 90% (about 1.9-fold or more), or by at least about 100% (about 2-fold or more), or by at least about 200% (about 3-fold or more), or by at least about 300% (about 4-fold or more), or by at least about 400% (about 5-fold or more), or by at least about 900% (about 10-fold or more)) in the sample of the subject as compared to the reference value or threshold value;
- an elevated quantity or expression level of PRDX6 (e.g. elevated by at least about 10% (about 1.1-fold or more), or by at least about 20% (about 1.2-fold or more), or by at least about 30% (about 1.3-fold or more), or by at least about 40% (about 1.4-fold or more), or by at least about 50% (about 1.5-fold or more), or by at least about 60% (about 1.6-fold or more), or by at least about 70% (about 1.7-fold or more), or by at least about 80% (about 1.8-fold or more), or by at least about 90% (about 1.9-fold or more), or by at least about 100% (about 2-fold or more), or by at least about 200% (about 3-fold or more), or by at least about 300% (about 4-fold or more), or by at least about 400% (about 5-fold or more), or by at least about 900% (about 10-fold or more)) in the sample of the subject as compared to the reference value or threshold value;
- an elevated quantity or expression level of FLNA (e.g. elevated by at least about 10% (about 1.1-fold or more), or by at least about 20% (about 1.2-fold or more), or by at least about 30% (about 1.3-fold or more), or by at least about 40% (about 1.4-fold or more), or by at least about 50% (about 1.5-fold or more), or by at least about 60% (about 1.6-fold or more), or by at least about 70% (about 1.7-fold or more), or by at least about 80% (about 1.8-fold or more), or by at least about 90% (about 1.9-fold or more), or by at least about 100% (about 2-fold or more), or by at least about 200% (about 3-fold or more), or by at least about 300% (about 4-fold or more), or by at least about 400% (about 5-fold or more), or by at least about 900% (about 10-fold or more), or by at least about 1400% (about 15-fold or more), or by at least about 1900% (about 20-fold or more)) in the sample of the subject as compared to the reference value or threshold value; and/or

- a decreased quantity or expression level of CDH13 (e.g. decreased by at least about 10% (about 0.9-fold or less), or by at least about 20% (about 0.8-fold or less), or by at least about 30% (about 0.7-fold or less), or by at least about 40% (about 0.6-fold or less), or by at least about 50% (about 0.5-fold or less), or by at least about 60% (about 0.4-fold or less), or by at least about 70% (about 0.3-fold or less), or by at least about 80% (about 0.2-fold or less), or by at least about 90% (about 0.1-fold or less)) in the sample of the subject as compared to the reference value or threshold value,

allows for the diagnosis of lung cancer in the subject. In particular embodiments, if the method comprises measuring the quantity or expression level of TUBA4A, GSTO1, FLNA, PRDX6, ARHGDIB and CDH13 in a biological sample from the subject and if the reference value or threshold value represents a subject or a group of subjects not affected by lung cancer, the subject is diagnosed with lung cancer if:

- the quantity or expression level of ARHGDIB is elevated in the sample of the subject as compared to the reference value or threshold value,
- the quantity or expression level of TUBA4A is elevated in the sample of the subject as compared to the reference value or threshold value,
- the quantity or expression level of GSTO1 is elevated in the sample of the subject as compared to the reference value or threshold value,
- the quantity or expression level of PRDX6 is elevated in the sample of the subject as compared to the reference value or threshold value,
- the quantity or expression level of FLNA is elevated in the sample of the subject as compared to the reference value or threshold value, and
- the quantity or expression level of CDH13 is decreased in the sample of the subject as compared to the reference value or threshold value.

Distinct reference or threshold values may represent the diagnosis of lung cancer *vs.* the absence of a lung cancer (such as, e.g., healthy or recovered from lung cancer). In another example, distinct reference values or threshold values may represent the diagnosis of a lung cancer of varying severity.

In yet another example, distinct reference or threshold values may represent the need of a subject for a therapeutic treatment of lung cancer *vs.* no need of a subject for a therapeutic treatment of a lung cancer. Such comparison may generally include any means to determine the presence or absence of at least one difference and optionally of the size of such difference between values or profiles being compared. A comparison may include a visual inspection, an arithmetical or statistical comparison of measurements. Such statistical comparisons include, but are not limited to, applying an algorithm. If the values or biomarker profiles comprise at least one standard, the comparison to determine a difference in said values or biomarker profiles may also include measurements of these standards, such that measurements of the biomarker are correlated to measurements of the internal standards.

Reference values or threshold values for the quantity or expression level of said biomarker selected from the group consisting of ARHGDIB, TUBA4A, GSTO1, FLNA, PRDX6 and CDH13 may be established according to known procedures previously employed for other biomarkers.

For example, a reference value of the amount of said biomarker selected from the group consisting of ARHGDIB, TUBA4A, GSTO1, FLNA, PRDX6 and CDH13 for a particular diagnosis of lung cancer as taught herein may be established by determining the quantity or expression level of said at least one biomarker in a biological sample(s) from one individual or from a population (e.g. group) of individuals characterized by said particular diagnosis of said disease or condition. Such population may comprise without limitation $\geq 2$, $\geq 10$, $\geq 100$, or even several hundred individuals or more.

Hence, by means of an illustrative example, reference values of the quantity or expression level of said biomarker selected from the group consisting of ARHGDIB, TUBA4A, GSTO1, FLNA, PRDX6 and CDH13 for the diagnosis of lung cancer *vs.* no such disease or condition may be established by determining the quantity or expression level of said biomarker in sample(s) from one individual or from a population of individuals diagnosed (e.g., based on other adequately conclusive means, such as, for example, clinical signs and symptoms, imaging, etc.) as, respectively, having or not having lung cancer.

Measuring the quantity or expression level of said biomarker selected from the group consisting of ARHGDIB, TUBA4A, GSTO1, FLNA, PRDX6 and CDH13 for the same patient at different time points may in such a case thus enable the continuous monitoring of the status of the patient and may lead to prediction of worsening or improvement of the patient's condition with regard to a given disease or condition as taught herein. Tools such as the kits described herein below can be developed to ensure this type of monitoring. One or more reference values, threshold values or ranges for said biomarker quantities or expression levels linked to the development of lung cancer can, e.g., be determined beforehand or during the monitoring process over a certain period of time in said subject. Alternatively, these reference values or ranges can be established through data sets of several patients with highly similar disease phenotypes, e.g., from subjects not developing lung cancer. A sudden deviation of the levels of said biomarker from said reference value, threshold value, or range can predict the worsening of the condition of the patient (e.g., at home or in the clinic) before the (often severe) symptoms actually can be felt or observed. Monitoring may be applied in the course of a medical treatment of a subject, preferably medical treatment aimed at alleviating the so-monitored disease or condition. Such monitoring may be comprised, e.g., in decision making whether a patient may be discharged, needs a change in treatment or needs further hospitalisation.

Accordingly, also provided herein is an *in vitro* method for monitoring lung cancer in a subject, wherein the method comprises detecting ARHGDIB and at least one, such as at least two, at least three, at least four, or all five, biomarkers selected from the group consisting of TUBA4A, GSTO1, FLNA, PRDX6 and CDH13 in a biological sample from the subject.

Furthermore, disclosed herein but not claimed is an *in vitro* method for monitoring lung cancer in a subject, wherein the method comprises detecting at least one, such as at least two, at least three, at least four, at least five or all six, biomarkers selected from the group consisting of ARHGDIB, TUBA4A, GSTO1, FLNA, PRDX6 and CDH13 in a biological sample from the subject.

In an embodiment, reference value(s) or threshold value(s) as intended herein may convey absolute quantities of the biomarker as intended herein. In another embodiment, the quantity of the biomarker in a sample from a tested subject may be determined directly relative to the reference value (e.g., in terms of increase or decrease, or fold-increase or fold-decrease). Advantageously, this may allow the comparison of the quantity or expression level of the biomarker in the sample from the subject with the reference value (in other words to measure the relative quantity of the biomarker in the sample from the subject vis-à-vis the reference value) without the need first to determine the respective absolute quantities of the biomarker.

As explained, the present methods, uses, or products may involve finding a deviation or no deviation between the quantity or expression level of said biomarkers as taught herein measured in a sample from a subject and a given reference value or threshold value.

A "deviation" of a first value from a second value or a "difference" between a first value and a second value may generally encompass any direction (e.g., increase: first value > second value; or decrease: first value < second value) and any extent of alteration.

For example, a deviation or a difference may encompass a decrease in a first value by, without limitation, at least about 10% (about 0.9-fold or less), or by at least about 20% (about 0.8-fold or less), or by at least about 30% (about 0.7-fold or less), or by at least about 40% (about 0.6-fold or less), or by at least about 50% (about 0.5-fold or less), or by at least about 60% (about 0.4-fold or less), or by at least about 70% (about 0.3-fold or less), or by at least about 80% (about 0.2-fold or less), or by at least about 90% (about 0.1-fold or less), relative to a second value with which a comparison is being made. For example, a deviation or a difference may encompass an increase of a first value by, without limitation, at least about 10% (about 1.1-fold or more), or by at least about 20% (about 1.2-fold or more), or by at least about 30% (about 1.3-fold or more), or by at least about 40% (about 1.4-fold or more), or by at least about 50% (about 1.5-fold or more), or by at least about 60% (about 1.6-fold or more), or by at least about 70% (about 1.7-fold or more), or by at least about 80% (about 1.8-fold or more), or by at least about 90% (about 1.9-fold or more), or by at least about 100% (about 2-fold or more), or by at least about 150% (about 2.5-fold or more), or by at least about 200% (about 3-fold or more), or by at least about 500% (about 6-fold or more), or by at least about 700% (about 8-fold or more), or like, relative to a second value with which a comparison is being made.

Preferably, a deviation or a difference may refer to a statistically significant observed alteration. For example, a deviation or a difference may refer to an observed alteration which falls outside of error margins of reference values in a given population (as expressed, for example, by standard deviation or standard error, or by a predetermined multiple thereof, e.g., ±1xSD or ±2xSD or ±3xSD, or ±1xSE or ±2xSE or ±3xSE). Deviation or a difference may also refer to a value falling outside of a reference range defined by values in a given population (for example, outside of a range which comprises ≥40%, ≥ 50%, ≥60%, ≥70%, ≥75% or ≥80% or ≥85% or ≥90% or ≥95% or even ≥100% of values in said population).

In a further embodiment, a deviation or a difference may be concluded if an observed alteration is beyond a given threshold or cut-off. Such threshold or cut-off may be selected as generally known in the art to provide for a chosen accuracy, sensitivity and/or specificity of the prediction methods, e.g., accuracy, sensitivity and/or specificity of at least 50%, or at least 60%, or at least 70%, or at least 80%, or at least 85%, or at least 90%, or at least 95%.

For example, receiver-operating characteristic (ROC) curve analysis can be used to select an optimal threshold or cut-off value of the quantity of a given biomarker for clinical use of the present diagnostic tests, based on acceptable global accuracy, sensitivity and/or specificity, or related performance measures which are well-known *per se,* such as positive predictive value (PPV), negative predictive value (NPV), positive likelihood ratio (LR+), negative likelihood ratio (LR-), Youden index, or similar. For example, an optimal threshold or cut-off value may be selected for each individual biomarker as a local extremum of the receiver operating characteristic (ROC) curve, i.e. a point of local maximum distance to the diagonal line, as described in Robin X., PanelomiX: a threshold-based algorithm to create panels of biomarkers, 2013, Translational Proteomics, 1(1):57-64.

The person skilled in the art will understand that it is not relevant to give an exact threshold or cut-off value. A relevant threshold or cut-off value can be obtained by correlating the sensitivity and specificity and the sensitivity/specificity for any threshold or cut-off value.

It is to the diagnostic engineers to determine which level of positive predictive value/negative predictive value/sensitivity/specificity is desirable and how much loss in positive or negative predictive value is tolerable. The chosen threshold or cut-off level could be dependent on other diagnostic parameters used in combination with the present method by the diagnostic engineers.

The present methods, uses, or products may further involve attributing any finding of a deviation or no deviation between the quantity or expression level of said biomarkers as taught herein measured in a biological sample from a subject and a given reference value or threshold to the presence or absence of lung cancer.

In the methods provided herein the observation of a deviation between the quantity or expression level of said biomarkers as taught herein and a reference value or threshold value can lead to the conclusion that the diagnosis of lung cancer in said subject is different from that represented by said reference value or threshold value. Similarly, when no deviation is found between the quantity or expression level of said biomarkers as taught herein in a sample from a subject and a reference value or threshold value, the absence of such deviation can lead to the conclusion that the diagnosis of said lung cancer in said subject is substantially the same as that represented by said reference value or threshold value.

In particular embodiments, the reference value or threshold value as used in the methods according to the invention is determined from a biological sample from a subject or a group of subjects not affected by lung cancer, such as a healthy subject or a group of healthy subjects. The healthy subject or group of healthy subjects may be at high or average risk of developing lung cancer, for example, the healthy subject or group of healty subjects may be a smoker or a group of smokers. The quantity or expression level of said biomarker selected from the group consisting of ARHGDIB, TUBA4A, GSTO1, FLNA, PRDX6 and CDH13 in a biological sample from a subject (preferably but without limitation, subject with lung cancer) may be elevated (e.g. if the biomarker is ARHGDIB, TUBA4A, GSTO1, FLNA or PRDX6) or decreased (e.g. if the biomarker is CDH13) compared to (i.e., relative to) a reference value or threshold value representing the quantity or expression level of said at least one biomarker selected from the group consisting of ARHGDIB, TUBA4A, GSTO1, FLNA, PRDX6 and CDH13 in a biological sample from a subject or a group of subjects not affected by lung cancer, such as a healthy subject or a group of healthy subjects. The so-elevated or decreased quantity or expression level may allow for the diagnosis of a lung cancer in the subject.

In order to make the diagnostic method as taught herein easy to use by medical practitioners, present inventors adopted a threshold-based approach, attributing a cut-off value per biomarker, wherein the cut-off value per biomarker is a local extremum of the receiver operating characteristic (ROC) curve, i.e. a point of local maximum distance to the diagonal line, as described in Robin X., PanelomiX: a threshold-based algorithm to create panels of biomarkers, 2013, Translational Proteomics, 1(1):57-64. In particular embodiments of the methods as taught herein, if a particular high global diagnostic accuracy of the method is desired (including a high sensitivity (e.g. higher or equal than 95%) and/or specificity (e.g. higher or equal than 95%) and the method comprises detecting ARHGDIB, TUBA4A, GSTO1, FLNA, PRDX6 and CDH13 in a biological sample, said reference value or threshold value for TUBA4A is 1.69, said reference value or threshold value for GSTO1 is 5.36, said reference value or threshold value for FLNA is 0.48, said reference value or threshold value for PRDX6 is 6.0, said reference value or threshold value for ARHGDIB is 0.51, and said reference value or threshold value for CDH13 is 69.83.

In further particular embodiments of the methods as taught herein, if a particular high sensitivity (e.g. higher or equal than 95%) and/or specificity (e.g. higher or equal than 95%) of the method is desired and the method comprises detecting ARHGDIB, TUBA4A, GSTO1, FLNA, PRDX6 and CDH13 in a biological sample, said reference value or threshold value for TUBA4A is 0.19, said reference value or threshold value for GSTO1 is 5.36, said reference value or threshold value for FLNA is 0.48, said reference value or threshold value for PRDX6 is 4.04, said reference value or threshold value for ARHGDIB is 0.51, and said reference value or threshold value for CDH13 is 148.16.

To make the diagnostic method as taught herein even more practical for medical practitioners, present inventors also adopted a single score per sample of a subject at risk of lung cancer to classify the sample as lung cancer or healthy, wherein the single score represents the number of biomarkers selected from the group consisting of ARHGDIB, TUBA4A, GSTO1, FLNA, PRDX6 and CDH13 which are differentially expressed in the sample of said subject compared to healthy individuals.

Accordingly, in particular embodiments, the method as taught herein comprises the steps of

(a) measuring the quantity or expression level of ARHGDIB and at least two, at least three, at least four or all five,

biomarkers selected from the group consisting of TUBA4A, GSTO1, FLNA, PRDX6 and CDH13 in the biological sample from the subject;

(b) calculating a score based on the quantity or expression levels of said biomarkers as measured in (a);

(c) comparing the score calculated in (b) with a reference or threshold score; and

(d) diagnosing the subject with lung cancer if the score calculated in (b) is equal to or higher than the reference or threshold score.

In particular embodiments, the method as taught herein comprises the steps of

(a) measuring the quantity or expression level of ARHGDIB and at least two, at least three, at least four or all five biomarkers selected from the group consisting of TUBA4A, GSTO1, FLNA, PRDX6 and CDH13 in the biological sample from the subject;

(b) comparing the quantity or expression level of said biomarkers as measured in (a) with a reference value or threshold value;

(c) finding a deviation or no deviation of the quantity or expression level of said biomarkers as measured in (a) from said reference value or threshold value;

(d) calculating a score representing the number of biomarkers measured in step (a) that were found to deviate from said reference value or threshold value in step (c);

(e) comparing the score calculated in (b) with a reference or threshold score; and

(f) diagnosing the subject with lung cancer if the score calculated in (b) is equal to or higher than said second reference or threshold score.

In particular embodiments, the score representing the number of biomarkers measured in step (a) that were found to deviate from said reference value or threshold value in step (c) can be represented as

$$\mathrm{S}_p = \sum_{i=1}^{n} \mathrm{I}\left(\mathrm{X}_{ip} \geq \mathrm{T}_i\right)$$

wherein $S_p$ is the score for patient p, n is the number of biomarkers measured in step (a), $X_{ip}$ is the quantity or expression level of the $i^{th}$ biomarker in subject p, $T_i$ is the reference value or threshold value for the $i^{th}$ biomarker, and I(x) is an indicator function which takes the value of 1 for x=true and 0 otherwise, for example as described in Robin X., PanelomiX: a threshold-based algorithm to create panels of biomarkers, 2013, Translational Proteomics, 1(1):57-64.

In particular embodiments, if a particular high global diagnostic accuracy of the method is desired (including a high sensitivity (e.g. higher or equal than 95%) and/or specificity (e.g. higher or equal than 95%), the method as taught herein comprises the steps of

(a) measuring the quantity or expression level of ARHGDIB, TUBA4A, GSTO1, FLNA, PRDX6 and CDH13 in the sample from the subject;

(b) comparing the quantity or expression level of said biomarkers as measured in (a) with a reference value or threshold value,

wherein said reference value or threshold value for TUBA4A is 1.69, said reference value or threshold value for GSTO1 is 5.36, said reference value or threshold value for FLNA is 0.48, said reference value or threshold value for PRDX6 is 6.0, said reference value or threshold value for ARHGDIB is 0.51, and said reference value or threshold value for CDH13 is 69.83;

(c) finding a deviation or no deviation of the quantity or expression level of said biomarkers as measured in (a) from said reference value or threshold value;

(d) diagnosing the subject with lung cancer if the quantity or expression level of at least three of the biomarkers as measured in (a) deviate from said reference value or threshold value.

In particular embodiments, if a particular high sensitivity (e.g. higher or equal than 95%) and/or specificity (e.g. higher or equal than 95%) of the method is desired, the method as taught herein comprises the steps of

(a) measuring the quantity or expression level of ARHGDIB, TUBA4A, GSTO1, FLNA, PRDX6 and CDH13 in the sample from the subject;

(b) comparing the quantity or expression level of said biomarkers as measured in (a) with a reference value or threshold value,

wherein said reference value or threshold value for TUBA4A is 0.19, said reference value or threshold value for GSTO1 is 5.36, said reference value or threshold value for FLNA is 0.48, said reference value or threshold value for PRDX6 is 4.04, said reference value or threshold value for ARHGDIB is 0.51, and/or said reference value or threshold value for CDH13 is 148.16;

(c) finding a deviation or no deviation of the quantity or expression level of said biomarkers as measured in (a) from said reference value or threshold value;

(d) diagnosing the subject with lung cancer if the quantity or expression level of at least five of the biomarkers as measured in (a) deviate from said reference value or threshold value.

The terms "sample" or "biological sample" as used herein include any biological specimen obtained and isolated from a subject. Samples may include, without limitation, organ tissue (i.e., lung tissue), whole blood, plasma, serum, whole blood cells, red blood cells, white blood cells (e.g., peripheral blood mononuclear cells), saliva, urine, stool (i.e., faeces), tears, sweat, sebum, nipple aspirate, ductal lavage, tumour exudates, synovial fluid, cerebrospinal fluid, lymph, fine needle aspirate, amniotic fluid, any other bodily fluid, cell lysates, cellular secretion products, inflammation fluid, semen and vaginal secretions. Preferably, a sample may be readily obtainable by minimally invasive methods, such as blood collection, allowing the removal / isolation / provision of the sample from the subject. The term "tissue" as used herein encompasses all types of cells of the human body including cells of organs but also including blood and other body fluids recited above.

In particular embodiments, the sample is a body fluid sample, preferably a body fluid sample selected from the group consisting of plasma, serum, whole blood, urine, tissue lysate, cerebrospinal fluid (CSF), saliva and sweat.

Identifying solid cancers by a simple blood analysis has been a long-standing goal in cancer research as the detection of cancer during the regular screening can offer the patients immediate treatment solutions. If combined with a highly accurate measurement method, blood samples may represent an ideal minimally invasive, easily collected material for cancer diagnostics.

While blood-based early diagnostics for cancer still remains a challenge, several proteins circulating in the blood have been useful for monitoring treatment response and/or tumor recurrence. So far, only prostate-specific antigen is routinely measured in blood for early diagnosis of cancer. Recently, Cohen and colleagues published the results of CancerSeek, a blood test that assesses the presence of 8 protein markers and 1933 genetic alterations in cell-free DNAs to diagnose common solid tumors. While the results were promising, the utility of this assay to advance cancer management has not yet garnered widespread adoption. The median sensitivity of CancerSeek in lung cancer was ~ 59%, the second lowest among the 8 cancer types investigated.

Present inventors found that each of the biomarkers selected from the group consisting of ARHGDIB, TUBA4A, GSTO1, FLNA, PRDX6 and CDH13 can be detected in plasma, for example by mass spectrometry, and that detection of at least one, preferably at least two, more preferably at least six, biomarker selected from the group consisting of ARHGDIB, TUBA4A, GSTO1, FLNA, PRDX6 and CDH13 in a plasma sample allows to differentiate lung cancer patients from subjects not suffering from lung cancer. Accordingly, the method of present invention allows diagnosis of lung cancer in a minimally invasive or even non-invasive manner, as the method as taught herein can be performed on the basis of a body fluid sample, particularly a plasma sample, which can be easily collected.

In particular embodiments, the sample is a plasma sample.

The terms "diagnosing" or "diagnosis" generally refer to the process or act of recognising, deciding on or concluding on a disease or condition in a subject on the basis of symptoms and signs and/or from results of various diagnostic procedures (such as, for example, from knowing the presence, absence and/or quantity of one or more biomarkers characteristic of the diagnosed disease or condition). As used herein, "diagnosis of" the diseases or conditions as taught herein in a subject may particularly mean that the subject has such, hence, is diagnosed as having such. "Diagnosis of no" diseases or conditions as taught herein in a subject may particularly mean that the subject does not have such, hence, is diagnosed as not having such. A subject may be diagnosed as not having such despite displaying one or more conventional symptoms or signs reminiscent of such.

The term "lung cancer", as used herein, refers to a malignant tumor occurring in the lung, and includes both small cell lung cancer and non-small cell lung cancer including adenocarcinoma, squamous cell carcinoma and large cell carcinoma.

Present inventors identified biomarkers which have a strong diagnostic performance in lung cancer and especially early-stage lung cancer, and which allow to diagnose lung cancer independently of the disease stage (including stage I tumors). As a result thereof, the present biomarkers are a very interesting tool to screen patients for lung cancer, even at an early stage of the disease.

In particular embodiments, the lung cancer is a stage I (e.g. stage IA or IB), stage II (e.g. stage IIA or IIB), stage III (e.g. stage IIIA, IIIB or IIIC) or stage IV (e.g. stage IVA or IVB) lung cancer, preferably a stage I or stage II lung cancer, more preferably stage I lung cancer.

The term "subject" or "patient" as used herein typically and preferably denotes humans, but may also encompass reference to non-human animals, preferably warm-blooded animals, more preferably vertebrates, even more preferably mammals, such as, e.g., non-human primates, rodents, canines, felines, equines, ovines, porcines, and the like. Particularly intended are subjects known or suspected to have lung cancer. Suitable subjects may include ones presenting to a physician for a screening for lung cancer and/or with symptoms and signs indicative of lung cancer.

In particular embodiments, the subject is a subject at a risk of lung cancer, such as a subject at average or high risk of lung cancer. Non-limiting examples of risk factors for lung cancer include genetic susceptibility, diet, occupational exposures (e.g. asbestos, metals, silica, polycyclic aromatic hydrocarbons, diesel exhaust), air pollution and tobacco smoking.

In more particular embodiments, the subject is a tobacco smoker or a former tobacco smoker.

The diagnostic method as taught herein can be used to efficiently complement imaging techniques in lung cancer screening.

In particular embodiments, the subject is a subject diagnosed with lung cancer, for example by imaging techniques.

As used herein, a phrase such as "a subject in need of treatment" includes subjects that would benefit from treatment of a given condition, particularly lung cancer. Such subjects may include, without limitation, those that have been diagnosed with said condition.

The terms "treat" or "treatment" encompass both the therapeutic treatment of an already developed disease or condition, such as the therapy of an already developed proliferative disease, as well as prophylactic or preventive measures, wherein the aim is to prevent or lessen the chances of incidence of an undesired affliction, such as to prevent progression of lung cancer. Beneficial or desired clinical results may include, without limitation, alleviation of one or more symptoms or one or more biological markers, diminishment of extent of disease, stabilised (i.e., not worsening) state of disease, delay or slowing of disease progression, amelioration or palliation of the disease state, and the like. "Treatment" can also mean prolonging survival as compared to expected survival if not receiving treatment. Non-limiting examples of therapeutic treatment of lung cancer are radiotherapy, chemotherapy, targeted drug therapy, immunotherapy and surgery.

In particular embodiments, the treatment is selected from the group consisting of radiotherapy, chemotherapy, targeted therapy, immunotherapy and surgery.

In particular embodiments, the treatment comprises the administration of an effective amount of a therapeutic agent selected from the group consisting of Abraxane, Afatinib Dimaleate, Afinitor, Afinitor Disperz, Alecensa, Alectinib, Alimta, Alunbrig Atezolizumab, Avastin, Bevacizumab, Brigatinib, Carboplatin, Ceritinib, Crizotinib, Cyramza, Dabrafenib Mesylate, Dacomitinib, Docetaxel, Doxorubicin Hydrochloride, Durvalumab, Entrictinib, Erlotinib Hydrochloride, Everolimus, Etopophus, Etopside, Etoposide Phosphate, Gefitinib, Gilotrif, Gemcitabine, Gemzar, Hycamtin, Imfinzi, Iressa, Keytruda, Lorbrena, Lrlatinib, Mechlorethamine Hydrocloride, Mekinist, Methotrexate, Mustargen, Mvasi, Navelbine, Necitumumab, Nivolumab, Opdivo, Osimertinib Mesylate, Paclitaxel, Pactilaxel Albumin-stabilized Nanoparticle formulation, Paraplat, Paraplatin, Pembrolizumab, Pemetrexed Disodium, Portrazza, Ramucirumab, Rozlytrek, Tafinlar, Tagrisso, Tarceva, Taxol, TAxotere, Tecentriq, Topotecan Hydrochloride, Trametinib, Trexall, Vizimpro, Vinorelbine Tartrate, Xalkori, Zykadia, carboplatin-taxol and gemcitabine-cisplatin.

The term "effective amount" as used herein may refer to a prophylactically effective amount, which is an amount of an active compound or pharmaceutical agent, more particularly a prophylactic agent, that inhibits or delays in a subject the onset of a disorder as being sought by a researcher, veterinarian, medical doctor or other clinician, or may refer to a therapeutically effective amount, which is an amount of active compound or pharmaceutical agent, more particularly a therapeutic agent, that elicits the biological or medicinal response in a subject that is being sought by a researcher, veterinarian, medical doctor or other clinician, which may include *inter alia* alleviation of the symptoms of the disease or condition being treated. Methods are known in the art for determining therapeutically and prophylactically effective doses for the agents as taught herein.

The term "administration" or "administering" as used herein refers to the giving of a certain treatment of lung cancer to a subject in need of such a treatment. Such a treatment can be a therapeutic agent as described elsewhere herein. The route of administration may be essentially any route of administration, such as without limitation, oral administration (such as, *e.g.,* oral ingestion or inhalation), intranasal administration (such as, e.g., intranasal inhalation or intranasal mucosal application), parenteral administration (such as, e.g., subcutaneous, intravenous, intramuscular, intraperitoneal or intrasternal injection or infusion), transdermal or transmucosal (such as, e.g., oral, sublingual, intranasal) administration, topical administration, rectal, vaginal or intra-tracheal instillation, and the like. In this way, the therapeutic effects attainable by the methods of the invention can be, for example, systemic, local, tissue-specific, etc., depending of the specific needs of a given application of the invention.

The present methods for the diagnosis of lung cancer may be adequately qualified as *in vitro* methods in that they apply one or more *in vitro* processing and/or analysis steps to a sample removed from the subject. The term *"in vitro"* generally denotes outside, or external to, a body, e.g., an animal or human body. Detecting or measuring said at least one biomarker selected from the group consisting of ARHGDIB, TUBA4A, GSTO1, FLNA, PRDX6 and CDH13 in a biological sample from a subject may ordinarily imply that the examination phase of the present methods comprises measuring the quantity of said at least one biomarker in the sample from the subject. One understands that the present methods may generally comprise an examination phase in which data is collected from and/or about the subject. A further aspect provides the use of a kit, the kit consisting of:

(a) means for measuring the quantity or expression level of ARHGDIB, and at least one biomarker selected from the group consisting of TUBA4A, GSTO1, FLNA, PRDX6 and CDH13 in a sample from a subject; and
(b) a reference value or threshold value for each of said biomarkers or means for establishing said reference value or threshold value, wherein said reference value or threshold value represents a known diagnosis of lung cancer, in the diagnosis of lung cancer based on the detection of said biomarkers in a biological sample of a subject.

In particular embodiments, the means for measuring biomarkers are specifically adapted for said biomarkers. For instance, various techniques for measuring biomarkers may employ binding agents for said respective biomarkers. Hence, the means for measuring the quantity or expression level of said biomarkers in a sample from a subject may comprise binding agents capable of specifically binding to said respective biomarkers, e.g. an antibody or antibody fragment, aptamer, photoaptamer, protein, peptide, peptidomimetic, or a small molecule, and/or carriers which allow visualization and/or a qualitative read-out of the measurement, for example, by spectrophotometry. In some embodiments, binding agents as taught herein may comprise a detectable label. In some embodiments, binding agents may be provided with a tag that permits detection with another agent (e.g., with a probe binding partner). The biomarker - binding agent conjugate may be associated with or attached to a detection agent to facilitate detection.
Additionally or alternatively, the binding agents may detect the expression of said biomarkers at an RNA level. The most commonly used methods for RNA detection include northern blots, the polymerase chain reaction (PCR), RNA in situ hybridization, cDNA microarrays, and high-throughput sequencing techniques.
In particular embodiments, the means for measuring the quantity or expression level of said biomarkers in a sample from a subject comprise one or more antibodies specifically binding to said respective biomarkers. Numerous antibodies to ARHGDIB, TUBA4A, GSTO1, FLNA, PRDX6 or CDH13 are commercially available from a variety of vendors. This information can be obtained from the respective vendors, and is also conveniently catalogued and can be queried in publically available databases, such as the GeneCards® database maintained by the Weizmann Institute (www.genecards.org), field "Antibody products".
In particular embodiments, the means for measuring the quantity or expression level of said biomarkers in a sample from a subject comprise one or more probes or primers specifically binding to the RNA encoding each of said biomarkers.
In particular embodiments, the reference value or threshold value is a reference value or threshold value for the quantity or expression level of said biomarker, wherein said reference value or threshold value corresponds to the quantity or expression level of said biomarker in a sample from a subject not affected by lung cancer, such as a sample from a healthy subject or a group of healthy subjects, or wherein said reference value or threshold value corresponds to the quantity or expression level of said biomarker in a sample from a subject or a group of subjects affected by lung cancer.
In particular embodiments, the kit further comprises a reference or threshold score representing the number of biomarkers selected from the group consisting of ARHGDIB, TUBA4A, GSTO1, FLNA, PRDX6 and CDH13 that were found to deviate from their respective reference value or threshold value. The kit for diagnosing lung cancer in a subject may further comprise ready-to use substrate solutions, wash solutions, dilution buffers and instructions. The diagnostic kit may also comprise positive and/or negative control samples.
Preferably, the instructions included in the diagnostic kit are unambiguous, concise and comprehensible to those skilled in the art. The instructions typically provide information on kit contents, how to collect the tissue sample, methodology, experimental read-outs and interpretation thereof and cautions and warnings.
The terms "kit of parts" and "kit" as used throughout this specification refer to a product containing components necessary for carrying out the specified methods (e.g., methods for the diagnosis of lung cancer in a subject or for determining whether a subject is in need of therapeutic treatment of lung cancer as taught herein), packed so as to allow their transport and storage. Materials suitable for packing the components comprised in a kit include crystal, plastic (e.g., polyethylene, polypropylene, polycarbonate), bottles, flasks, vials, ampules, paper, envelopes, or other types of containers, carriers or supports. Where a kit comprises a plurality of components, at least a subset of the components (e.g., two or more of the plurality of components) or all of the components may be physically separated, e.g., comprised in or on separate containers, carriers or supports. The components comprised in a kit may be sufficient or may not be sufficient for carrying out the specified methods, such that external reagents or substances may not be necessary or may be necessary for performing the methods, respectively. Typically, kits are employed in conjunction with standard laboratory equipment, such as liquid handling equipment, environment (e.g., temperature) controlling equipment, analytical instruments, etc. In addition to the recited binding agents(s) as taught herein, such as for example, antibodies, hybridisation probes, amplification and/or sequencing primers, optionally provided on arrays or microarrays, the present kits may also include some or all of solvents, buffers (such as for example but without limitation histidine-buffers, citrate-buffers, succinate-buffers, acetate-buffers, phosphate-buffers, formate buffers, benzoate buffers, TRIS (Tris(hydroxymethyl)-aminomethan) buffers or maleate buffers, or mixtures thereof), enzymes (such as for example but without limitation thermostable DNA polymerase), detectable labels, detection reagents, and control formulations (positive and/or negative), useful in the specified methods. Typically, the kits may also include instructions for use thereof, such as on a printed insert or on a computer readable medium. The terms may be used interchangeably with the term "article of manufacture", which broadly encompasses any man-made tangible structural product, when used in the present context.

In particular embodiments, the kit further comprises a computer readable storage medium having recorded thereon one or more programs for carrying out the method as taught herein.

Also disclosed herein but not claimed is a kit, in particular a kit for diagnosing or monitoring lung cancer, the kit comprising:

(a) means for measuring the quantity or expression level of at least one, at least two, at least three, at least four, at least five or all six, preferably at least two, biomarkers selected from the group consisting of ARHGDIB, TUBA4A, GSTO1, FLNA, PRDX6 and CDH13 in a sample from a subject; and
(b) a reference value or threshold value for each of said at least one, at least two, at least three, at least four, at least five or all six, preferably at least two, biomarkers or means for establishing said reference value or threshold value, wherein said reference value or threshold value represents a known diagnosis of lung cancer.

Also disclosed herein but not specifically claimed is the use of such kit for the diagnosis of lung cancer or for monitoring lung cancer based on the detection of said biomarkers in a sample of a subject.

Furthermore, present inventors found that specific methods can successfully be used to identify and validate suitable biomarker panels.

Accordingly, also disclosed herein but not claimed is a method for identifying and validating biomarker panels for a certain disease or disorder, preferably lung cancer, comprising

(a) measuring the quantity or expression level of a pre-determined set of proteins in biological samples of a first group of patients diagnosed with said disease or condition and a first group of healthy patients;
(b) identifying the proteins of which the quantity or expression levels significantly differ between the group of patients diagnosed with said disease or condition and the group of healthy patients;
(c) performing a hierarchical clustering on the proteins identified in (b) and selecting one protein per group of correlated proteins;
(d) applying absolute shrinkage and selection operator (LASSO) in combination with bootstrap sampling for the proteins selected in (c) to obtain the best protein biomarker panel for outcome prediction of the disease or condition;
(e) optionally identifying the optimal threshold for the each biomarker of the protein biomarker panel obtained in (d); and
(f) validating each biomarker of the protein biomarker panel obtained in (d) on an a second group of patients diagnosed with said disease or condition and a second group of healthy patients.

The pre-determined set of proteins may comprise proteins which are known to be associated with the disease or disorder and optionally which are known to be measurable within the biological sample. For example, if the biological sample is human blood, the pre-determined set of proteins only comprises proteins which are detectable in the human blood. If the biological sample is human blood, the pre-determined set of proteins may further comprise well-known blood proteins, such as well-known plasma proteins.

The proteins known to be associated with the disease or disorder may comprise proteins identified using genomic analysis of diseased and non-diseased human tissue (e.g. differentially expressed genes between tumor vs. tumor normal specimens with at least 2-fold difference ($p < 0.05$)), proteins identified based on mice xenograft studies (e.g. human protein candidates identified in the plasma of mice xenografted with human lung cancer cells proteins), and/or proteins identified based on published literature.

The group of patients diagnosed with said disease or condition and the group of healthy patients may be selected in such a way that they are a good representation of the intended population in which the biomarker panel will be used for screening.

The step for identifying the proteins of which the quantity or expression level significantly differs between the group of patients diagnosed with said disease or condition and the group of healthy patients may be performed using non-parametric Kruskal-Wallis test and Bonferroni adjusted P-values. For example, proteins are considered to significantly differ between the group of patients diagnosed with said disease or condition and the group of healthy patients if $P < 0.00014$ (= 0.05/351; Bonferroni corrected). A hierarchical clustering may be performed on the proteins identified in (b) using Spearman's correlation coefficient. For example, hierarchical clustering of proteins can be performed using a dissimilarity function (= 1 - absolute value of correlation) to discriminate all correlated groups. The one protein per group of correlated proteins may be selected to represent the group of correlated proteins, for example, based on high intensity, lower missing values in the biological samples and/or absence of interference in PRM signals, if LC-PRM-MS method is used to measure the quantity or expression level of a pre-determined set of proteins in the biological samples.

The step of performing a hierarchical clustering on the proteins identified in (b) may allow to exclude highly correlated proteins before applying LASSO in combination with bootstrap sampling. This prevents that the LASSO randomly chooses one protein of a group of highly correlated proteins.

The best protein biomarker panel for outcome prediction of the disease or condition may be selected based on the frequence of retaining a certain combination of proteins.

The optimal threshold for each biomarker of the protein biomarker panel obtained in (d) may be determined using the Panelomix platform, for example as described in Robin X., PanelomiX: a threshold-based algorithm to create panels of biomarkers, 2013, Translational Proteomics, 1(1):57-64.

The above aspects and embodiments are further supported by the following non-limiting examples.

**EXAMPLES**

Example 1: use of the biomarker panel as taught herein allows to diagnose lung cancer in a plasma sample from the patient

I.Materials and methods

**1.1** Study cohort.

The training cohort consisted of 128 lung cancer patients and 93 healthy donors followed within Luxembourg's hospitals. The validation cohort comprised 48 patients and 49 age-, sex and smoking status-matched non-cancer subjects, not included in the training cohort. All the participants provided blood samples following informed consent according to the Helsinki Declaration. The study was approved by the national research ethics committee "Comité National d'Ethique de Recherche" and the national commission for data protection "Commission Nationale pour la Protection des Données". Blood samples were collected and processed following the standard operating procedures of the Integrated Biobank of Luxembourg to prepare plasma samples. Diagnosis, staging and grading of the disease were done by experienced pathologists, according to the IASLC/ATS/ERS histological classification of lung tumors (2011) and TNM classification of lung carcinoma (2009). The clinicopathological features of the subjects are summarized in Tables 1 and 2.

**Table 1 . Clinicopathological features of lung cancer patients and healthy donors in the training cohort.**

| **Features** | | **Lung cancer diagnosis** | | | **No cancer diagnosis** |
|---|---|---|---|---|---|
| | | ADC | SCC | Others* | |
| N° of subjects | | 73 | 24 | 31 | 93 |
| Gender | Female | 35 | 4 | 12 | 42 |
| | Male | 38 | 21 | 18 | 51 |
| Age at blood collection (y) [median value (range)] | | 63 (28-85) | 71.5 (59-86) | 62.5 (41-77) | 62 (32-85) |
| Smoking history | Never smokers | 15 | 0 | 4 | 14 |
| | Former smokers | 37 | 20 | 17 | 54 |
| | Current smokers | 21 | 5 | 9 | 25 |
| Stage | I | 13 | 8 | 2 | |
| | II | 3 | 6 | 2 | |
| | III | 4 | 5 | 10 | |
| | IV | 46 | 4 | 14 | |

(continued)

| Features | | Lung cancer diagnosis | | | No cancer diagnosis |
|---|---|---|---|---|---|
| | Not available | 7 | 2 | 2 | |
| Grade** | I | 11 | 11 | 1 | |
| | II | 22 | 3 | 2 | |
| | II~III | 3 | 0 | 0 | |
| | III and IV | 15 | 8 | 23 | |
| | Not available | 22 | 3 | 4 | |
| Anticancer treatment status before blood collection | Untreated | 52 | 23 | 24 | |
| | Treated | 21 | 2 | 6 | |

*Other lung cancer types and subtypes including NSCLC not otherwise specified (NOS) with or without evidence of neuroendocrine differentiation, adenosquamous carcinoma, large cell carcinoma (LCC), large cell neuroendocrine carcinoma (LCNEC), small cell lung cancer (SCLC), atypical carcinoid and mixed types.
Diagnosis and staging of lung cancer patients were done by experienced pathologists following the IASLC/ATS/ERS histological classification of lung tumours (2011) and TNM classification of lung carcinoma (2009).
**In mixed tumors, grading was determined according to the most predominant pattern. If not specified by the pathologist, atypical carcinoids were inherently graded as moderately differentiated, and SCLC and LCC as poorly differentiated tumors.
Grade I = well-differentiated; Grade II = moderately-differentiated; Grade III = poorly-differentiated; Grade IV = undifferentiated

**Table 2. Clinicopathological features of lung cancer patients and healthy donors in the validation cohort.**

| Features | | Lung cancer diagnosis | | | No cancer diagnosis |
|---|---|---|---|---|---|
| | | ADC | SCC | Others* | |
| N° of subjects | | 26 | 11 | 11 | 49 |
| Gender | Female | 11 | 1 | 4 | 17 |
| | Male | 15 | 10 | 7 | 32 |
| Age at blood collection (y) [median value (range)] | | 68 (49-85) | 63 (47-83) | 70 (65-83) | 67 (32-82) |
| Smoking history | Never smokers | 5 | 0 | 1 | 8 |
| | Former smokers | 18 | 7 | 9 | 33 |
| | Current smokers | 3 | 4 | 1 | 8 |
| Stage | I | 9 | 1 | 1 | |
| | II | 1 | 1 | 1 | |
| | III | 5 | 4 | 3 | |
| | IV | 10 | 4 | 6 | |
| | Not available | 1 | 1 | 0 | |

(continued)

| Features | | Lung cancer diagnosis | | | No cancer diagnosis |
|---|---|---|---|---|---|
| Grade** | I | 7 | 2 | 0 | |
| | II | 9 | 6 | 0 | |
| | III and IV | 3 | 2 | 7 | |
| | Not available | 7 | 1 | 4 | |
| Anticancer treatment status before blood collection | Untreated | 23 | 6 | 10 | |
| | Treated | 2 | 3 | 1 | |
| | Not available | 1 | 2 | 0 | |

*Other lung cancer types and subtypes including NSCLC not otherwise specified (NOS) with or without evidence of neuroendocrine differentiation, adenosquamous carcinoma, large cell carcinoma (LCC), large cell neuroendocrine carcinoma (LCNEC), small cell lung cancer (SCLC), carcinoid tumors and mixed types.

Diagnosis and staging of lung cancer patients were done by experienced pathologists following the IASLC/ATS/ERS histological classification of lung tumors (2011) and TNM classification of lung carcinoma (2009).

**In mixed tumors, grading was determined according to the most predominant pattern. If not specified by the pathologist, SCLC and LCC were inherently graded as poorly differentiated tumors.

Grade I = well-differentiated; Grade II = moderately-differentiated; Grade III = poorly-differentiated; Grade IV = undifferentiated

**1.2 Plasma depletion and processing.**

*Training cohort*

High abundance proteins were removed from 40 μL of plasma using an Agilent 1260 Infinity Bio-inert LC system equipped with a Human 14 Multiple Affinity Removal Column (4.6 × 100 mm) (Agilent Technologies, Diegem, Belgium) according to the manufacturer's procedure. After elution, buffer A was exchanged to 100 mM $NH_4HCO_3$ / 10% ACN (pH 8) and the volume was reduced to 100 μL using a spin concentrator 5K (Agilent). Proteins were denatured with 1% sodium deoxycholate (SDC), reduced with 10 mM dithiothreitol for 30 min at 37°C, alkylated with 25 mM iodoacetamide for 30 min at room temperature followed by quenching with 10 mM n-acetyl-L-cysteine. All reagents were prepared in 50 mM tris buffer. The processed sample was diluted to reduce the SDC concentration to 0.5% and incubated with 13 μg of sequencing grade trypsin (Promega, Leiden, The Netherlands) for 16 h at 37°C, then with 10 U of PNGase F for 1 h at 37°C followed by additional 2 μg of trypsin for 3 h at 37°C. SDC was removed by precipitation with 1% formic acid and centrifugation. Digested samples were cleaned up with Sep-Pak C18 cartridges (Waters, Milford, MA, USA) and dried *in vacuo.* Samples were reconstituted with 200 μL of 0.1% formic acid/ 4% acetonitrile.

*Validation cohort*

Aliquots of 40 μl of plasma samples from patient and healthy donors were depleted using a LC1260 chromatography system coupled with a Mars14 column (Agilent) depletion column. The depleted plasma samples were buffer exchanged with 50mM ammonium bicarbonate buffer by ultrafiltration onto Agilent spin filters (5KDa MWCO). An aliquot of 10% of the samples were then denatured for 1H at 37°C with sodium deoxycholate (SDC) 1% (w/v) and 10mM DTT. Samples were then alkylated for 30 min in the dark at room temperature with iodoacetamide at a final concentration of 25mM. The reaction was stopped by addition of acetyl cysteine at a final concentration 10mM. The samples were diluted to 1% (w/v) SDC with the ammonium bicarbonate buffer and then digested overnight by sequencing grade trypsin (Promega). Afterwards, the samples were deglycosylated by incubation with of PNGase F for 1H at 37°C followed by a second tryptic digestion for 3H hours at 37°C. The SDC was precipitated by addition of formic acid to 1% final and removed by centrifugation. The supernatants were then purified by solid phase extraction (Sep Pak C18, Waters). Eluted samples were dried in vacuum centrifuge and finally suspended in 50μL of 1% acetonitrile and 0.05% trifluoroacetic acid in water.

A mixture of quantified synthetic peptides (aqua quant pro, Thermo scientific) labeled with heavy C-terminal lysine or arginine (C-terminal arginine, $^{13}C_6$, $^{15}N_4$, $\Delta m$ = 10 Da,C-terminal lysine $^{13}C_6$, $^{15}N_2$, $\Delta m$ = 8 Da) was aliquoted for single use and stored at -80°C. The mixture was spiked in the digested plasma sample before LC-MS analysis.

### 1.3 LC-PRM analysis.

*Training cohort*

Stable isotope labeled (SIL) ($^{13}C_6{}^{15}N_4$ for the C-terminal arginine and $^{13}C_6{}^{15}N_2$ for the C-terminal lysine) synthetic peptides were used as internal standards (AQUA QuantPro grade, Thermo Fisher Scientific, Bremen, Germany). For each peptide, LC-MS attributes (retention time, precursor m/z and the most intense fragment ions) were determined to build the LC-PRM method. Samples were analysed using scheduled LC-PRM assays for 351 peptides. An Ultimate 3000 RSLCnano system coupled to a Q-Exactive Plus mass spectrometer (Thermo Fisher Scientific) was used as described previously in Kim YJ, et al. Quantification of SAA1 and SAA2 in lung cancer plasma using the isotype-specific PRM assays. Proteomics 2015;15:3116-3125. Precise, relative quantification was obtained from the intensity ratio of light and SIL peptides.

*Validation cohort*

The LC-MS setup consisted in a Dionex U3000 RSLC liquid chromatography system operated in column switching mode coupled with Q Exactive Plus mass spectrometer. The A and B mobile phases of the liquid chromatography consisted of water with 0.1% formic acid and acetonitrile with 0.1% formic acid, respectively. The loading phase consisted in 1% acetonitrile and 0.05% trifluoroacetic acid in water. The samples were loaded onto a trap column (75 $\mu$m $\times$ 20 mm, $C_{18}$ pepmap 100, 3 $\mu$m) by the loading phase at a flow rate of 5 $\mu$l/min. The samples were then eluted from the trap to the analytical column (75 $\mu$m $\times$ 150 mm, C18 for pepmap 100, 2 $\mu$m) by a linear gradient ranging from 2% A to 35% B in 66 min. The MS acquisition was performed on a Q Exactive Plus, Thermo Scientific) operated in parallel reaction monitoring mode (PRM). The acquisition loop consisted in a time scheduled targeted PRM acquisition performed at a resolution of 70,000 at 200 m/z. The isolation windows of the targeted peptide ions was set to 1 m/z, the normalized collision energy to 25 and the maximum fill time to 240ms. The duration of the time scheduled windows for each pairs of endogenous and isotope labelled peptides was set to 5 min and centred on their retention times.

### 1.4 Model development and statistical analysis (training cohort).

The LC-PRM signal was converted into protein concentration in finol/$\mu$L based on the internal standard peptides. Values of undetected proteins were replaced by minimal protein concentration/$\sqrt{2}$. Non-parametric Kruskal-Wallis test and Bonferroni adjusted P-values were used to compare protein concentrations in lung cancer and healthy samples. Proteins with P-value < 0.00014 (= 0.05/351; Bonferroni corrected) were further considered for analysis. Correlations between proteins were investigated using Spearman's correlation coefficient. Hierarchical clustering of proteins was performed using a dissimilarity function (= 1 - absolute value of correlation) to discriminate all correlated groups. One protein per group of highly correlated proteins was selected to represent the group, based on high intensity, lower missing values in lung cancer samples and absence of interference in PRM signals.

Bootstrap sampling and least absolute shrinkage and selection operator (LASSO) penalisation were used to find the best combination of proteins for outcome prediction. LASSO with 10-fold cross-validation was performed on 4,500,000 bootstrapped datasets, using the "glmnet" package of R. To assess the predictive power of proteins and protein combinations, the negative predictive value (NPV), positive predictive value (PPV), sensitivity, specificity, area under the receiver operating characteristic curve (AUC) and the Akaike Information Criterion (AIC) of the logistic regression models were calculated on the original dataset. A bootstrap test was used to compare AUC of different models. For comparing sensitivities and specificities, the McNemar $\chi^2$ test was used, as recommended.[17] For model validation, sensitivity, specificity, NPV, PPV, AUC and their 95% confidence intervals (CI) were calculated on the validation dataset.

Multinomial logistic regression was used to predict the probability of each cancer stage (6 levels including 4 cancer stages, one unknown stage and one healthy condition) using the 6-protein panel. The level with the highest probability was chosen as the final predicted cancer stage (or healthy condition). The Cohen's kappa test was used to evaluate the degree of agreement between clinically annotated and predicted staging.

Continuous variables were compared using the Kruskal- Wallis test. Binary or categorical variables were compared using Pearson's Chi-Squared test.

### 1.5 MS data processing of validation cohort

Data were processed with Skyline software (v19.1.0.193). The MS signal of the four most intense and least interfered product ions of each pair of endogenous and isotope labelled peptides were extracted as ion chromatograms (XIC). The relative dot product between each pair of endogenous and isotope labelled peptides fragment ions XICs were calculated using Skyline embedded calculator and the peptides pairs with a score below 0.99 were rejected for the quantitative analysis. The quantitative values were extracted as the sum of the endogenous fragment ions XICs areas divided by the sum of the XICs areas of the corresponding isotope labelled peptide.

MS signal was converted in concentration by the following formula:

$$[endogeneous\ peptide](fmole)/\mu L\ of\ plasma = \frac{[isotope\ labeled\ peptide](fmole) \times XIC\ area\ ratio}{\mu L\ of\ plasma}$$

### 1.6 Use of PanelomiX for threshold selection (training cohort).

The PanelomiX platform as described in Robin X. PanelomiX for the Combination of Biomarkers. Methods Mol Biol 2019;1959:261-273 was used to select thresholds for the candidate biomarkers to have the optimal classification performance of the combination. First, a threshold value was defined for each of the proteins, then a score was assigned to each subject. A patient's score is the number of biomarkers fulfilling the disease condition (referred to as "positive" biomarker). A subject was classified as lung cancer patient if their score was at least equal to a panel threshold score identified by Panelomix. Thresholds obtained from the training set were applied to the validation set for cancer prediction and the performance metrics were calculated.

### 1.7 Discovery summary

***Genomic analysis for human lung tissue***

DNA copy number / changes of 19 tumor samples were determined from 38 lung cancer specimen using Agilent Sure Print Human CGH Microarray 244K. Copy number variations (CNV) specific for each tumor sample was determined as log-fold change relative to a human reference (Promega, Madison, WI) using DNA Analytics. For gene expression analysis, Agilent SurePrint G3 Human Exon 2X400K Microarray was used. Of the 19 matched healthy and tumor paired specimens, 16 matched samples had high quality RNA (RIN > 7.5) for analysis. A two-sided t-test was used to identify differentially expressed genes between tumor vs. tumor normal specimens with at least 2-fold difference ($p < 0.05$) using GeneSpring Software. Seventy-eight genes were commonly overexpressed in the tumor samples when compared to tumor normal lung specimens, whereas 81 genes were underexpressed in the tumor samples as compared to tumor normal. Candidate gene biomarkers were prioritized as previously described (Salhia B, Kiefer J, Ross JT, et al. Integrated genomic and epigenomic analysis of breast cancer brain metastasis. PLoS One 2014;9:e85448).

***Mice xenograft study***

Orthotopic lung cancer models were developed from human lung cancer cell lines (H2009 and H1975) in the lungs of immuno-compromised mice as described in the previous study. After, two weeks of implantation, mouse plasma was collected from 21 mice that developed a tumor to identify circulating human proteins by LC-MS/MS based proteomics. Shotgun-based proteomic analysis was performed on these plasma samples to identify human proteins secreted from the lung tumor into the mouse blood circulation. Bioinformatics analysis distinguished 436 human-specific proteins in the mice plasma, including proteins previously implicated in the development and progression of lung cancer.

***Integration of the discovery data and candidate prioritization***

For additional discovery, we exhaustively searched the published literature to identify high quality - omics datasets of lung cancer and curated the 40 datasets that met stringent quality standards. These datasets were combined with our own discovery data to develop a comprehensive human tissue candidate database. The 4000 top-ranked candidates from these four datasets (proteomics, aCGH, gene expression, and literature curation) were combined with the human protein candidates identified in the plasma of mice xenografted with human lung cancer cells to yield a total of 4254 unique biomarker candidates for consideration in the next prioritization step.

In order to prioritize the candidates based on our ability to detect them in plasma, two-pronged analyses were performed. First, in-depth shotgun analysis of a pool of human plasma derived from 13 late-stage lung cancer patients was performed, resulting in the detection of 1245 unique human proteins, 520 overlapping with the prioritized tissue-based candidate list. Secondly, to help detect candidates that potentially were not evident from the shotgun proteomics we applied Accurate Inclusion Mass Screening (AIMS). Using empirically observed peptides and publically available databases, we assembled a list of 29270 peptides representative of 3573 of our protein biomarker candidates. These peptides were subset into 20 separate inclusion lists and divided equally between FHCRC and TGen for analysis in depleted plasma by two-dimensional separation LC-MS/MS. Biomarker candidates observed by shotgun or AIMS analysis of depleted human plasma were used to prioritize the biomarker candidates for the verification study, resulting in 559 proteins.

**2. Results**

**2.1 Patient and healthy donor demographics.**

The cohort was composed of 57.92 % male, 42.08 % female with 14.93 % non-smokers, 57.92 % former smokers and 27.15 % current smokers. The mean age was 63.56 (±10.03) and the median age was 63 (Table 3). No significant differences in age, gender and smoking status were found between healthy and cancer individuals.

**2.2 Broad selection of potential tumour predictors in plasma.**

Previous multi-omics discovery efforts performed in the present inventors' laboratories suggested 559 proteins to be associated with lung cancer and potentially detectable in human blood (see Discovery Summary in section 1.8 of the Materials and Methods ) (Zhang H, Kennedy J, Lee LW, et al. Integrated Strategy for Lung Cancer Biomarker Candidate Discovery by Quantitative Proteomics Profiling on Tumor and Adjacent Normal Lung Tissue (abstract). 59 th ASMS Conference on Mass Spectrometry and Allied Topics Denver, Colorado: 2011:Abstract nr MP 679 and Zhang H, Whiteaker J, Lin C, et al. Prioritization of Plasma-Based Predictive Markers for Chemotherapy in Lung Cancer Using Fractionation and Targeted Mass Spectrometry (abstract). 61st ASMS Conference on Mass Spectrometry and Allied Topics. Minneapolis, Minnesota: 2013:Abstract nr MP 541). The detectabiliy of each protein in human plasma was previously verified resulting in a set of 323 proteins to be further verified in a larger cohort. In this study, the plasma levels of the 323 proteins were quantified by LC-PRM in plasma from lung cancer patients and healthy donors. An additional 28 well-known plasma proteins were also screened.Differential analysis of the PRM data indicated that plasma levels of 229 proteins were significantly different between lung cancer and healthy groups (data not shown).

**Table 3. Patient and healthy donor demographics**

| | **All subjects** | | | **Healthy donors** | | |
|---|---|---|---|---|---|---|
| | **N (%)** | **Mean (±SD)** | **Median** | **N (%)** | **N (%)** | **Median** |
| **Gender** | | | | | | |
| Male | 128 (57.92) | | | 51 (54.84) | 51 (54.84) | |
| Female | 93 (42.08) | | | 42 (45.16) | 42 (45.16) | |
| Total | 221 | | | 93 | 93 | |
| **Age** | | 63.56 (±10.03) | 63 | 93 | 93 | 62 |
| **Smoking Status** | | | | | | |
| Non-Smoker | 33 (14.93) | | | 14 (15.05) | 14 (15.05) | |

| Former Smoker | 128 (57.92) | | | 54 (58.06) | 54 (58.06) | |
|---|---|---|---|---|---|---|
| Current Smoker | 60 (27.15) | | | 25 (26.88) | 25 (26.88) | |

| | **Cancer patients** | | | |
|---|---|---|---|---|
| | **N (%)** | **Mean (±SD)** | **Median** | **P-value*** |
| **Gender** | **N (%)** | | | 0.51 |
| Male | | | | |
| Female | 77 (60.16) | | | |
| Total | 51 (39.84) | | | |
| **Age** | 128 | 64.45 (±9.96) | 64 | 0.09 |
| **Smoking Status** | 128 | | | 1.00 |
| Non-Smoker | | | | |
| Former Smoker | 19 (14.84) | | | |
| Current Smoker | 60 (27.15) | | | 25 (26.88) |

* The ages of healthy donors and cancer patients were compared using non-parametric Kruskal- Wallis Test. "Gender" and "Smoking Status" in both sub-populations were compared using Pearson's Chi-Squared Test.

### 2.3 Refinement of biomarker selection

From the 229 differentially abundant proteins in plasma from lung cancer and healthy subjects, 90 proteins showed a correlation ≥0.9 or ≤-0.9 with one or more proteins, whereas 139 proteins displayed weaker correlations. When a threshold of dissimilarity or "distance" between proteins was set to 0.1 (as an absolute value), 19 groups with highly correlated proteins were identified. Accordingly, 19 surrogate proteins were chosen (see Materials and Methods for details) and 71 proteins were excluded from further analysis.

LASSO variable selection was implemented with 158 proteins. The combination that was retained the most (23 times) was FLNA, TUBA4A, GSTO1, PRDX6, ARHGDIB and CDH13 (hereafter referred to as 6-protein combination/panel/classifier) (Table 4). The concentrations of the 6 proteins were significantly different in plasma from lung cancer patients and healthy donors (Figure 1). The PRM readouts of the proteins measured in samples from one lung cancer patient and one healthy donor, compared to the internal standards, are shown in Figure 2. These proteins were individually selected as the most predictive ones, independently of the combination, in 74.51% of the cases for FLNA, 76.91% for TUBA4A, 44.42% for GSTO1, 54.74% for PRDX6 , 45.11% for ARHGDIB and 81.43% for CDH13 (Table 4). The proteins that were selected as predictive in more than 75% of all combinations were TUBA4A, TFPI and CDH13 (hereafter referred to as 3-protein combination).

**Table 4. Protein combinations selected more than 10 times in LASSO as the most predictive ones in distinguishing lung cancer from healthy samples, and the percentage of appearance of individual proteins in 4500000 bootstrapped datasets.**

| **Appearance (x= times)** | 74.51% | 76.91% | 44.42% | 54.74% | 45.11% | 81.43% | 52.94% |
|---|---|---|---|---|---|---|---|
| **23 x** | FLNA | TUBA4A | GSTO1 | PRDX6 | ARHGDIB | CDH13 | |
| **18 x** | FLNA | TUBA4A | GSTO1 | PRDX6 | | CDH13 | HSPB1 |

(continued)

| Appearance (x= times) | 74.51% | 76.91% | 44.42% | 54.74% | 45.11% | 81.43% | 52.94% |
|---|---|---|---|---|---|---|---|
| **15 x** | FLNA | TUBA4A | GSTO1 | PRDX6 | | CDH13 | HSPB1 |
| | FLNA | TUBA4A | GSTO1 | PRDX6 | | CDH13 | |
| **14 x** | FLNA | TUBA4A | GSTO1 | PRDX6 | | CDH13 | |
| **13 x** | FLNA | TUBA4A | GSTO1 | PRDX6 | | CDH13 | HSPB1 |
| **12 x** | FLNA | TUBA4A | | PRDX6 | ARHGDIB | CDH13 | |
| | FLNA | TUBA4A | GSTO1 | PRDX6 | | CDH13 | HSPB1 |
| | FLNA | TUBA4A | GSTO1 | PRDX6 | | CDH13 | HSPB1 |
| **Appearance (x= times)** | 85.22% | 68.68% | 61.64% | 56.63% | 57.73% | 52.59% | 39.87% |
| **23 x** | | | | | | | |
| **18 x** | TFPI | GSN | GPX3 | IGFBP3 | F7 | | |
| **15 x** | | GSN | | | | | |
| **14 x** | | | | | | PEBP1 | |
| **13 x** | TFPI | GSN | | IGFBP3 | | | |
| **12 x** | TFPI | | GPX3 | | | PEPB1 | ENPEP |
| | TFPI | | | | | | |

**2.4 Performance analysis of the models.**

Present inventors compared the performance of the models towards the commercially available Xpresys® Lung (XL) test (Biodesix, Boulder, CO) that consists of five diagnostic proteins. XL test originally designed to differentiate benign from malignant lung nodules among indeterminate pulmonary nodules. The present biomarker panel was compared to the five diagnostic protein-panel included in the Xpresys® Lung XL test since the XL test is the only currently commercially available plasma protein classifier in the lung cancer diagnostics market. However, some caution should be exercised in the interpretation of the comparative results: Xpresys® Lung XL was developed to help identify likely benign nodules following a chest computerized tomography (CT) scan. Accordingly, it was validated in a subject cohort presenting with 8 to 30 mm lung nodules and a diagnosis of non-small cell lung cancer (i.e. Stage IA) or without evidence of cancer. In the present study, Xpresys® Lung XL was studied in a cohort of patients with mixed lung cancer types and stages in addition to the healthy population. Since the primary objective and the targeted population of the present study differ from those of Xpresys® Lung XL investigations, the comparison between the 6-biomarker panel and the Xpresys® Lung XL is just an indication of the good performance of the present classifier and should not be interpreted as a comparison of the biomaker panels' utility. Nevertheless, while limited, based on different primary objectives and target populations, direct comparison with the XL test in the same pool of plasma samples can provide a useful benchmark for the present panel. The values of the performance indicators were the best with the 6-protein combination compared to the 3-protein combination, XL panel and the univariable models (Table 5): the lowest AIC (30.876), the highest AUC (0.999) (shared with the 3-protein combination), the highest PPV (0.992), the highest NPV (0.989), the highest specificity (0.989) (shared with ARHGDIB) and the highest sensitivity (0.992). The use of TUBA4A, TFPI and CDH13, as a classifier, showed a slightly higher AIC (31.402) and slightly lower PPV (0.984), NPV (0.968), specificity (0.978) and sensitivity (0.977) values. When considering FLNA, TUBA4A, GSTO1, PRDX6 and ARHGDIB as sole classifiers, the performance indicators showed also excellent predictive power. Only CDH13 and TFPI performed worse but still with a good predictive power (AUC = 0.845 and 0.851, respectively).

Compared to the 6- protein model, the logistic regression model derived using the proteins of the XL panel had a higher AIC (45.592) suggesting a worse fitness to the data. In addition, the PPV, NPV, specificity and sensitivity were lower than the ones of the 6-protein and 3-protein models (Table 5). Next, the ability of the 6-protein panel to predict cancer stage was tested. As shown in Table 6, the 6-protein panel distinguished between healthy and lung cancer individuals but could not predict cancer stage. An unweighted Cohen's Kappa of 0.59 (95% CI, 0.52-0.66) and a weighted Cohen's Kappa of 0.73 (95% CI, 0.73-0.73) were found, suggesting a weak degree of agreement between predicted and clinically annotated stages. Importantly, the 6-protein panel classified 22 out of 23 stage I patients as lung cancer individuals, demonstrating its strong diagnostic performance in early-stage cases.

**Table 5. Performance of the logistic regression models in lung cancer prediction.**

| Model | AIC | AUC | PPV | NPV | Specificit y | Sensitivit y |
|---|---|---|---|---|---|---|
| **6- protein combination** | 30.876 | 0.999 | 0.992 | 0.989 | 0.989 | 0.992 |
| **3- protein combination** | 31.402 | 0.999 | 0.984 | 0.968 | 0.978 | 0.977 |
| **FLNA** | 65.647 | 0.990 | 0.967 | 0.908 | 0.957 | 0.930 |
| **TUBA4A** | 41.556 | 0.997 | 0.984 | 0.948 | 0.978 | 0.961 |
| **GSTO1** | 45.427 | 0.996 | 0.976 | 0.947 | 0.968 | 0.961 |
| **PRDX6** | 51.763 | 0.993 | 0.976 | 0.957 | 0.968 | 0.969 |
| **ARHGDIB** | 54.303 | 0.981 | 0.992 | 0.929 | 0.989 | 0.945 |
| **CDH13** | 219.090 | 0.845 | 0.791 | 0.747 | 0.699 | 0.828 |
| **TFPI** | 204.860 | 0.851 | 0.836 | 0.737 | 0.785 | 0.797 |
| **Xpresys® XL panel** | 45.592 | 0.996 | 0.969 | 0.957 | 0.957 | 0.969 |
| **ALDOA** | 43.946 | 0.994 | 0.969 | 0.947 | 0.957 | 0.961 |
| **COL18A1** | 250.790 | 0.767 | 0.752 | 0.630 | 0.677 | 0.711 |
| **FTL** | 297.720 | 0.554 | 0.579 | NaN* | 0.000 | 1.000 |
| **LGALS3BP** | 295.220 | 0.601 | 0.601 | 0.500 | 0.258 | 0.813 |
| **THBS1** | 161.780 | 0.924 | 0.871 | 0.794 | 0.828 | 0.844 |
| *NaN cannot be calculated since no patient was classified as not having a cancer | | | | | | |

**Table 6. Number of clinically annotated and predicted healthy and cancer patients, including their stages, as obtained using the 6-protein classifier.**

| | | Clinically annotated stages | | | | | |
|---|---|---|---|---|---|---|---|
| | | **No cancer** | **Stage NA*** | **Stage I** | **Stage II** | **Stage III** | **Stage IV** |
| **Predicted stages** | **No cancer** | 92 | 1 | 1 | 0 | 1 | 0 |
| | **Stage NA*** | 0 | 2 | 0 | 1 | 0 | 0 |
| | **Stage I** | 0 | 2 | 9 | 1 | 2 | 6 |
| | **Stage II** | 0 | 0 | 0 | 0 | 0 | 1 |
| | **Stage III** | 0 | 0 | 0 | 1 | 0 | 0 |
| | **Stage IV** | 1 | 6 | 13 | 8 | 16 | 57 |
| | **Sum** | 93 | 11 | 23 | 11 | 19 | 64 |

*NA = not available.

**2.5 Determination of biomarker thresholds for sample classification.**

The PanelomiX platform was used to select the best thresholds for the 6 biomarkers identified. Three panel optimization options were used: optimizing the sensitivity at ≥95% specificity, optimizing the specificity at ≥95% sensitivity and optimizing global accuracy. When choosing to optimize the accuracy or the specificity, only one threshold per biomarker was selected by Panelomix, resulting in one combination per optimization. When optimizing the sensitivity, 19644 combinations were found, with the first one being the same as the threshold combination selected when optimizing the specificity. Therefore, two threshold combinations were considered: The one obtained when optimizing the panel accuracy ($T_A$ combination) and the combination common to sensitivity and specificity optimization ($T_S$ combination) (Table 7). If any 3 proteins were positive using $T_A$ thresholds, then the subject was classified as having lung cancer. For

$T_S$, any 5 of the 6 proteins have to be positive in order to classify an individual as having lung cancer.

Applying the thresholds on the original dataset, the performance metrics of the panel were excellent: a sensitivity of 0.992 and a specificity of 0.989 for $T_A$ combination, and a sensitivity of 0.977 and a specificity of 1.0 for $T_S$ combination.

**Table 7. Threshold values and positivity of the biomarkers when optimizing the global accuracy ($T_A$) or the sensitivity or specificity ($T_S$) of the panel, as defined by PanelomiX platform.**

| Protein biomarker | $T_A$ | $T_S$ |
|---|---|---|
| FLNA | > 0.48091298 | > 0.48091298 |
| TUBA4A | > 1.6875327 | > 0.18983749 |
| GSTO1 | > 5.363042 | > 5.363042 |
| PRDX6 | > 5.9975386 | > 4.038682 |
| ARHGDIB | > 0.5091874 | > 0.5091874 |
| CDH13 | < 69.826614 | < 148.1571 |

**2.6 Panel performance on the validation dataset.**

The models were then tested on a validation dataset using plasma from 48 lung cancer patients and 49 healthy donors. The models' estimates of the logistic regression and Panelomix thresholds obtained from the training set were applied to the validation set for cancer prediction. NPV, PPV, sensitivity and specificity of the XL and the 6-protein panels were calculated for the new dataset (Table 8). When comparing the results obtained from the logistic regression models, values of all the performance metrics of the 6-protein combination were at least as high as the values of the XL panel. Interestingly, the highest specificity (0.918) was obtained for the 6- protein panel as predicted by the $T_S$ thresholds. All the possible sub-combinations of the 6-protein panel were also tested on the validation dataset. Many of them displayed excellent performance as shown by the forest plots of NPV, PPV, sensitivity, specificity and AUC (Figure 3). The sub-combinations of biomarkers were filtered for sub-combinations having a sensitivity of ≥0.90 and NPV≥0.90 or a specificity of ≥0.90. 36 sub-combinations as shown in Table 9 were obtained after filtration and have the best performance. Of these 36 sub-combinations, 6 combinations have a sensitivity ≥0.90, a NPV ≥0.90 and a specificity ≥0.90, namely FLNA-TUBA4A-GSTO1-CDH13 (i.e. combination 8 in Table 9), FLNA-ARHGDIB-CDH13 (i.e. combination 21 in Table 9), TUBA4A-GSTO1-CDH13 (i.e. combination 23 in Table 9), TUBA4A-PRDX6-CDH13 (i.e. combination 25 in Table 9), TUBA4A-ARHGDIB-CDH13 (i.e. combination 26 in Table 9), and GSTO1-CDH13 (i.e. combination 33 in Table 9).

**Table 8. Performance of the classification models on the validation dataset**

| | **6- protein panel** | | | **Xpresys® XL panel** |
|---|---|---|---|---|
| | $T_A$ thresholds | $T_S$ thresholds | logistic regression | logistic regression |
| **NPV (95% CI)** | 0.840 (0.709-0.928) | 0.849 (0.724-0.933) | 0.935 (0.821-0.986) | 0.930 (0.809-0.985) |
| **PPV (95% CI)** | 0.851 (0.717-0.938) | 0.909 (0.783-0.975) | 0.882 (0.761-0.956) | 0.833 (0.707-0.921) |
| **Sensitivity (95% CI)** | 0.833 (0.698-0.925) | 0.833 (0.698-0.925) | 0.938 (0.828-0.987) | 0.938 (0.828-0.987) |
| **Specificity (95% CI)** | 0.857 (0.728-0.941) | 0.918 (0.804-0.977) | 0.878 (0.752-0.954) | 0.816 (0.680-0.912) |
| **AUC (95% CI)** | 0.845 (0.773-0.918) | 0.876 (0.810-0.942) | 0.908 (0.850-0.965) | 0.877 (0.812-0.942) |

**Table 9. Performance of the best sub-combinations on the validation dataset**

| # | Biomarker(s) | PPV (95% CI) | NPV (95% CI) | Spec (95% CI) | Sens (95% CI) | AUC (95% CI) |
|---|---|---|---|---|---|---|
| 1 | FLNA-TUBA4A-GSTO1- PRDX6-ARHGDIB-CDH13 | 0.882 (0.761-0.956) | 0.935 (0.821-0.986) | 0.878 (0.752-0.954) | 0.938 (0.828-0.987) | 0.908 (0.850-0.965) |
| 2 | FLNA-TUBA4A-GSTO1-PRDX6-ARHGDIB | 0.865 (0.742-0.944) | 0.933 (0.817-0.986) | 0.857 (0.728-0.941) | 0.938 (0.828-0.987) | 0.897 (0.837-0.958) |
| 3 | FLNA-TUBA4A-GSTO1-ARHGDIB-CDH13 | 0.868 (0.747-0.945) | 0.955 (0.845-0.994) | 0.857 (0.728-0.941) | 0.958 (0.857-0.995) | 0.908 (0.851-0.965) |
| 4 | FLNA-TUBA4A-PRDX6-ARHGDIB-CDH13 | 0.882 (0.761-0.956) | 0.935 (0.821-0.986) | 0.878 (0.752-0.954) | 0.938 (0.828-0.987) | 0.908 (0.850-0.965) |
| 5 | FLNA-GSTO1-PRDX6-ARHGDIB-CDH13 | 0.882 (0.761-0.956) | 0.935 (0.821-0.986) | 0.878 (0.752-0.954) | 0.938 (0.828-0.987) | 0.908 (0.850-0.965) |
| 6 | TUBA4A-GSTO1-PRDX6-ARHGDIB-CDH13 | 0.880 (0.757-0.955) | 0.915 (0.796-0.976) | 0.878 (0.752-0.954) | 0.917 (0.800-0.977) | 0.897 (0.836-0.958) |
| 7 | FLNA-TUBA4A-GSTO1-ARHGDIB | 0.882 (0.761-0.956) | 0.935 (0.821-0.986) | 0.878 (0.752-0.954) | 0.938 (0.828-0.987) | 0.908 (0.850-0.965) |
| 8 | FLNA-TUBA4A-GSTO1-CDH13 (not claimed) | 0.896 (0.773-0.965) | 0.898 (0.778-0.966) | 0.898 (0.778-0.966) | 0.896 (0.773-0.965) | 0.897 (0.836-0.958) |
| 9 | FLNA-TUBA4A-PRDX6-ARHGDIB | 0.882 (0.761-0.956) | 0.935 (0.821-0.986) | 0.878 (0.752-0.954) | 0.938 (0.828-0.987) | 0.908 (0.850-0.965) |
| 10 | FLNA-TUBA4A-ARHGDIB-CDH13 | 0.882 (0.761-0.956) | 0.935 (0.821-0.986) | 0.878 (0.752-0.954) | 0.938 (0.828-0.987) | 0.908 (0.850-0.965) |
| 11 | FLNA-GSTO1-PRDX6-ARHGDIB | 0.868 (0.747-0.945) | 0.955 (0.845-0.994) | 0.857 (0.728-0.941) | 0.958 (0.857-0.995) | 0.908 (0.851-0.965) |
| 12 | FLNA-GSTO1-ARHGDIB-CDH13 | 0.885 (0.766-0.956) | 0.956 (0.849-0.995) | 0.878 (0.752-0.954) | 0.958 (0.857-0.995) | 0.918 (0.863-0.972) |
| 13 | FLNA-PRDX6-ARHGDIB-CDH13 | 0.882 (0.761-0.956) | 0.935 (0.821-0.986) | 0.878 (0.752-0.954) | 0.938 (0.828-0.987) | 0.908 (0.850-0.965) |
| 14 | TUBA4A-GSTO1-PRDX6-ARHGDIB | 0.882 (0.761-0.956) | 0.935 (0.821-0.986) | 0.878 (0.752-0.954) | 0.938 (0.828-0.987) | 0.908 (0.850-0.965) |
| 15 | TUBA4A-GSTO1-ARHGDIB-CDH13 | 0.882 (0.761-0.956) | 0.935 (0.821-0.986) | 0.878 (0.752-0.954) | 0.938 (0.828-0.987) | 0.908 (0.850-0.965) |
| 16 | TUBA4A-PRDX6-ARHGDIB-CDH13 | 0.882 (0.761-0.956) | 0.935 (0.821-0.986) | 0.878 (0.752-0.954) | 0.938 (0.828-0.987) | 0.908 (0.850-0.965) |
| 17 | GSTO1-PRDX6-ARHGDIB-CDH13 | 0.882 (0.761-0.956) | 0.935 (0.821-0.986) | 0.878 (0.752-0.954) | 0.938 (0.828-0.987) | 0.908 (0.850-0.965) |
| 18 | FLNA-TUBA4A-ARHGDIB | 0.880 (0.757-0.955) | 0.915 (0.796-0.976) | 0.878 (0.752-0.954) | 0.917 (0.800-0.977) | 0.897 (0.836-0.958) |
| 19 | FLNA-GSTO1-ARHGDIB | 0.885 (0.766-0.956) | 0.956 (0.849-0.995) | 0.878 (0.752-0.954) | 0.958 (0.857-0.995) | 0.918 (0.863-0.972) |

(continued)

| # | Biomarker(s) | PPV (95% CI) | NPV (95% CI) | Spec (95% CI) | Sens (95% CI) | AUC (95% CI) |
|---|---|---|---|---|---|---|
| 20 | FLNA-PRDX6-ARHGDIB | 0.885 (0.766-0.956) | 0.956 (0.849-0.995) | 0.878 (0.752-0.954) | 0.958 (0.857-0.995) | 0.918 (0.863-0.972) |
| 21 | FLNA-ARHGDIB-CDH13 | 0.900 (0.782-0.967) | 0.936 (0.825-0.987) | 0.898 (0.778-0.966) | 0.938 (0.828-0.987) | 0.918 (0.863-0.973) |
| 22 | TUBA4A-GSTO1-ARHGDIB | 0.882 (0.761-0.956) | 0.935 (0.821-0.986) | 0.878 (0.752-0.954) | 0.938 (0.828-0.987) | 0.908 (0.850-0.965) |
| 23 | TUBA4A-GSTO1-CDH13 (not claimed) | 0.896 (0.773-0.965) | 0.898 (0.778-0.966) | 0.898 (0.778-0.966) | 0.896 (0.773-0.965) | 0.897 (0.836-0.958) |
| 24 | TUBA4A-PRDX6-ARHGDIB | 0.882 (0.761-0.956) | 0.935 (0.821-0.986) | 0.878 (0.752-0.954) | 0.938 (0.828-0.987) | 0.908 (0.850-0.965) |
| 25 | TUBA4A-PRDX6-CDH13 (not claimed) | 0.915 (0.796-0.976) | 0.900 (0.782-0.967) | 0.918 (0.804-0.977) | 0.896 (0.773-0.965) | 0.907 (0.849-0.965) |
| 26 | TUBA4A-ARHGDIB-CDH13 | 0.900 (0.782-0.967) | 0.936 (0.825-0.987) | 0.898 (0.778-0.966) | 0.938 (0.828-0.987) | 0.918 (0.863-0.973) |
| 27 | GSTO1-PRDX6-ARHGDIB | 0.865 (0.742-0.944) | 0.933 (0.817-0.986) | 0.857 (0.728-0.941) | 0.938 (0.828-0.987) | 0.897 (0.837-0.958) |
| 28 | GSTO1-ARHGDIB-CDH13 | 0.885 (0.766-0.956) | 0.956 (0.849-0.995) | 0.878 (0.752-0.954) | 0.958 (0.857-0.995) | 0.918 (0.863-0.972) |
| 29 | PRDX6-ARHGDIB-CDH13 | 0.885 (0.766-0.956) | 0.956 (0.849-0.995) | 0.878 (0.752-0.954) | 0.958 (0.857-0.995) | 0.918 (0.863-0.972) |
| 30 | FLNA-ARHGDIB | 0.880 (0.757-0.955) | 0.915 (0.796-0.976) | 0.878 (0.752-0.954) | 0.917 (0.800-0.977) | 0.897 (0.836-0.958) |
| 31 | TUBA4A-ARHGDIB | 0.880 (0.757-0.955) | 0.915 (0.796-0.976) | 0.878 (0.752-0.954) | 0.917 (0.800-0.977) | 0.897 (0.836-0.958) |
| 32 | GSTO1-ARHGDIB | 0.882 (0.761-0.956) | 0.935 (0.821-0.986) | 0.878 (0.752-0.954) | 0.938 (0.828-0.987) | 0.908 (0.850-0.965) |
| 33 | GSTO1-CDH13 (not claimed) | 0.896 (0.773-0.965) | 0.898 (0.778-0.966) | 0.898 (0.778-0.966) | 0.896 (0.773-0.965) | 0.897 (0.836-0.958) |
| 34 | PRDX6-ARHGDIB | 0.868 (0.747-0.945) | 0.955 (0.845-0.994) | 0.857 (0.728-0.941) | 0.958 (0.857-0.995) | 0.908 (0.851-0.965) |
| 35 | ARHGDIB-CDH13 | 0.825 (0.701-0.913) | 0.975 (0.868-0.999) | 0.796 (0.657-0.898) | 0.979 (0.889-0.999) | 0.888 (0.827-0.948) |
| 36 | ARHGDIB (not claimed) | 0.849 (0.724-0.933) | 0.932 (0.813-0.986) | 0.837 (0.703-0.927) | 0.938 (0.828-0.987) | 0.887 (0.824-0.950) |

### 3. Discussion

The purpose of this study was to identify a panel of protein biomarkers to be used as a non-invasive diagnostic tool in lung cancer. For this purpose, 351 potential biomarkers were screened, that have been discovered and preliminarily verified in human plasma. Here, based on PRM measurement followed by logistic regression analysis, present inventors identified a blood-based 6-protein panel as a potential diagnostic tool in lung cancer. In order to make this panel easy to use by medical practitioners, present inventors also adopted a threshold-based approach, attributing a cut-off value per biomarker, then a score per sample to classify it as lung cancer or healthy.

The biomarker panel displayed excellent performance in the test cohort, supported by the AUC (0.999), PPV (0.992), NPV (0.989), specificity (0.989) and sensitivity (0.992) values. The results were confirmed in a validation dataset which also showed that other sub-combinations of these 6 proteins displayed excellent discriminative power. Importantly, the ability of the 6-protein panel to detect non-invasively lung cancer independently of the disease stage (including stage I tumors) suggests its high potential as a screening tool.

The performance of the 6-protein biomarker panel as described herein was compared to a commercially available, MS-based lung cancer diagnostic test, Xpresys® Lung (XL) test. While limited, based on different primary objectives and target populations, direct comparison with the XL test in the same pool of plasma samples can provide a useful benchmark for the 6-protein biomarker panel as described herein. In the training set, the values of all performance metrics tended to be better with the 6-protein biomarker panel as described herein and showed that the present panels displayed an excellent diagnostic accuracy in the present cohort.

The biomarker panel is further validated in independent cohorts which include patients with different cancer types (e.g. colon cancer) and donors with and without underlying non-malignant lung diseases (e.g. chronic obstructive pulmonary disease). Analysis thereof confirms the specificity of the biomarker panel for lung cancer.

In conclusion, present inventors identified a protein-based diagnostic panel to detect lung cancer in blood. If used as a routine test for high- and average-risk individuals (e.g. smokers and former smokers), it may efficiently complement LDCT in lung cancer screening. This would reduce the number of false-positive cases that often lead to additional invasive tests and unnecessary costs and expose the patients to physical and mental hardships.

## Claims

**1.** An *in vitro* method for diagnosing lung cancer in a subject, wherein the method comprises detecting Rho GDP dissociation inhibitor beta (ARHGDIB) and at least one biomarker selected from the group consisting of alpha-tubulin 4A (TUBA4A), glutathione S-transferase omega 1 (GSTO1), filamin A (FLNA), peroxiredoxin 6 (PRDX6) and cadherin 13 (CDH13) in a biological sample from the subject.

**2.** The method according to claim 1, wherein the method comprises detecting ARHGDIB and at least two biomarkers selected from the group consisting of TUBA4A, GSTO1, FLNA and PRDX6 and CDH13.

**3.** The method according to claim 1 or 2, comprising detecting at least three, at least four, or at least five biomarkers selected from the group consisting of ARHGDIB, TUBA4A, GSTO1, FLNA, PRDX6 and CDH13.

**4.** The method according to claim 1 or 2, comprising detecting ARHGDIB and TUBA4A; ARHGDIB and GST01; ARHGDIB and FLNA; ARHGDIB and PRDX6 or ARHGDIB and CDH13.

**5.** The method according to any one of claims 1 to 4, comprising detecting

(a) ARHGDIB, TUBA4A and GSTO1;
(b) ARHGDIB, TUBA4A and FLNA;
(c) ARHGDIB, TUBA4A and PRDX6; or
(d) ARHGDIB, TUBA4A and CDH13.

**6.** The method according to any one of claims 1 to 5, comprising detecting

(a) ARHGDIB, TUBA4A, GSTO1 and FLNA;
(b) ARHGDIB, TUBA4A, FLNA and PRDX6;
(c) ARHGDIB, TUBA4A, GSTO1 and PRDX6.
(d) ARHGDIB, TUBA4A, FLNA and CDH13;
(e) ARHGDIB, TUBA4A, GSTO1 and CDH13; or
(f) ARHGDIB, TUBA4A, PRDX6 and CDH13.

**7.** The method according to any of claims 1 to 6, comprising detecting

(a) ARHGDIB, TUBA4A, GST01, FLNA and PRDX6;
(b) ARHGDIB, TUBA4A, GSTO1, FLNA and CDH13;
(c) ARHGDIB, TUBA4A, FLNA, PRDX6 and CDH13; or
(d) ARHGDIB, TUBA4A, GSTO1, PRDX6 and CDH13.

**8.** The method according to any one of claims 1 to 7, comprising detecting ARHGDIB, TUBA4A, GSTO1, FLNA, PRDX6 and CDH13.

9. The method according to any one of claims 1 to 8, wherein the biological sample is a body fluid sample; preferably a body fluid sample selected from the group consisting of plasma, serum, whole blood, urine, tissue lysate, cerebrospinal fluid (CSF), saliva and sweat; more preferably wherein the biological sample is a plasma sample.

10. The method according to any one of claims 1 to 9, wherein the at least two biomarkers are detected using mass spectrometry analysis methods, biochemical assay methods, immunoassay methods, chromatography methods, or combinations thereof.

11. The method according to any one of claims 1 to 10, comprising the steps of

(a) measuring the quantity or expression levels of at least ARHGDIB and at least one biomarker selected from the group consisting of TUBA4A, GSTO1, FLNA, PRDX6 and CDH13 in the biological sample from the subject;
(b) calculating a score based on the quantity or expression levels of said biomarkers measured in (a);
(c) comparing the score calculated in (b) with a threshold score; and
(d) diagnosing the subject with lung cancer if the score calculated in (b) is equal to or higher than the threshold score.

12. Use of a kit,

the kit consisting of:

(a) means specifically adapted for measuring the quantity or expression levels of ARHGDIB and at least one biomarker selected from the group consisting of TUBA4A, GSTO1, FLNA, PRDX6 and CDH13 in a biological sample from a subject; and
(b) a threshold value for each of said biomarkers or means for establishing said threshold value, wherein said threshold value represents a known diagnosis of lung cancer,

in the diagnosis of lung cancer based on the detection of said biomarkers in a biological sample of a subject.

13. The use according to claim 12, wherein said means is a binding agent specifically binding to said protein or to RNA encoding said protein.

14. The use according to claim 12 or 13 in the method according to any one of claims 1 to 11.

**Patentansprüche**

1. *In-vitro*-Verfahren zum Diagnostizieren von Lungenkrebs in einem Subjekt, wobei das Verfahren das Nachweisen von Rho-GDP-Dissoziationshemmer beta (ARHGDIB) und von mindestens einem Biomarker umfasst, ausgewählt aus der Gruppe bestehend aus Alpha-tubulin 4A (TUBA4A), Glutathion-S-transferase omega 1 (GSTO1), Filamin A (FLNA), Peroxiredoxin 6 (PRDX6) und Cadherin 13 (CDH13) in einer biologischen Probe von dem Subjekt .

2. Verfahren nach Anspruch 1, wobei das Verfahren das Nachweisen von ARHGDIB und mindestens zwei Biomarkern umfasst, ausgewählt aus der Gruppe bestehend aus TUBA4A, GSTO1, FLNA und PRDX6 und CDH13.

3. Verfahren nach Anspruch 1 oder 2, umfassend das Nachweisen von mindestens drei, mindestens vier oder mindestens fünf Biomarkern, ausgewählt aus der Gruppe bestehend aus ARHGDIB, TUBA4A, GSTO1, FLNA, PRDX6 und CDH13.

4. Verfahren nach Anspruch 1 oder 2, umfassend das Nachweisen von ARHGDIB und TUBA4A; ARHGDIB und GSTO1; ARHGDIB und FLNA; ARHGDIB und PRDX6 oder ARHGDIB und CDH13.

5. Verfahren nach einem der Ansprüche 1 bis 4, umfassend das Nachweisen von

(a) ARHGDIB, TUBA4A und GSTO1;
(b) ARHGDIB, TUBA4A und FLNA;

(c) ARHGDIB, TUBA4A und PRDX6; or
(d) ARHGDIB, TUBA4A und CDH13.

**6.** Verfahren nach einem der Ansprüche 1 bis 5, umfassend das Nachweisen von

(a) ARHGDIB, TUBA4A, GSTO1 und FLNA;
(b) ARHGDIB, TUBA4A, FLNA und PRDX6;
(c) ARHGDIB, TUBA4A, GSTO1 und PRDX6.
(d) ARHGDIB, TUBA4A, FLNA und CDH13;
(e) ARHGDIB, TUBA4A, GSTO1 und CDH13; oder
(f) ARHGDIB, TUBA4A, PRDX6 und CDH13.

**7.** Verfahren nach einem der Ansprüche 1 bis 6, umfassend das Nachweisen von

(a) ARHGDIB, TUBA4A, GSTO1, FLNA und PRDX6;
(b) ARHGDIB, TUBA4A, GSTO1, FLNA und CDH13;
(c) ARHGDIB, TUBA4A, FLNA, PRDX6 und CDH13; oder
(d) ARHGDIB, TUBA4A, GSTO1, PRDX6 und CDH13.

**8.** Verfahren nach einem der Ansprüche 1 bis 7, umfassend das Nachweisen von ARHGDIB, TUBA4A, GSTO1, FLNA, PRDX6 und CDH13.

**9.** Verfahren nach einem der Ansprüche 1 bis 8, wobei die biologische Probe eine Körperflüssigkeitsprobe ist; vorzugsweise eine Körperflüssigkeitsprobe, ausgewählt aus der Gruppe bestehend aus Plasma, Serum, Vollblut, Urin, Gewebelysat, Zerebrospinalflüssigkeit (CSF), Speichel und Schweiß; stärker bevorzugt wobei die biologische Probe eine Plasmaprobe ist.

**10.** Verfahren nach einem der Ansprüche 1 bis 9, wobei die mindestens zwei Biomarker mittels massenspektrometrischen Analyseverfahren, biochemischen Assay-Verfahren, Immunoassay-Verfahren, Chromatographieverfahren oder Kombinationen daraus nachgewiesen werden.

**11.** Verfahren nach einem der Ansprüche 1 bis 10, umfassend die Schritte

(a) Messen der Menge oder der Expressionspegel von mindestens ARHGDIB und mindestens einem Biomarker, ausgewählt aus der Gruppe bestehend aus TUBA4A, GSTO1, FLNA, PRDX6 und CDH13, in der biologischen Probe des Subjekts;
(b) Berechnen eines Scores basierend auf der Menge oder dem Expressionspegel der Biomarker, gemessen in (a);
(c) Vergleichen des in (b) berechneten Scores mit einem Schwellenwert-Score; und
(d) Diagnostizieren von Lungenkrebs bei dem Subjekt, wenn der in (b) berechnete Score gleich oder höher als dem Schwellenwert-Score ist.

**12.** Verwendung eines Kits,

wobei das Kit besteht aus:

(a) Mitteln, die speziell angepasst sind zum Messen der Menge oder der Expressionspegel von ARHGDIB und mindestens einem Biomarker, ausgewählt aus der Gruppe bestehend aus TUBA4A, GSTO1, FLNA, PRDX6 und CDH13, in einer biologischen Probe eines Subjekts; und
(b) einem Schwellenwert für jeden/jedes der Biomarker oder Mittel zum Festlegen des Schwellenwerts, wobei der Schwellenwert eine bekannte Diagnose von Lungenkrebs darstellt,

bei der Diagnose von Lungenkrebs basierend auf dem Nachweis dieser Biomarker in einer biologischen Probe eines Subjekts.

**13.** Verwendung nach Anspruch 12, wobei das Mittel ein Bindemittel ist, das spezifisch an das Protein oder an RNA, die für das Protein kodiert, bindet.

14. Verwendung nach Anspruch 12 oder 13 in dem Verfahren nach einem der Ansprüche 1 bis 11.

## Revendications

1. Procédé *in vitro* pour le diagnostic du cancer du poumon chez un sujet, dans lequel le procédé comprend la détection de l'inhibiteur bêta de la dissociation de Rho GDP (ARHGDIB) et d'au moins un biomarqueur sélectionné dans le groupe constitué par l'alpha-tubuline 4A (TUBA4A), la glutathion S-transférase Oméga 1 (GSTO1), la filamine A (FLNA), la peroxyredoxine 6 (PRDX6) et la cadhérine 13 (CDH13) dans un échantillon biologique du sujet.

2. Procédé selon la revendication 1, dans lequel le procédé comprend la détection d'ARHGDIB et d'au moins deux biomarqueurs sélectionnés dans le groupe constitué par TUBA4A, GSTO1, FLNA et PRDX6 et CDH13.

3. Procédé selon la revendication 1 ou 2, comprenant la détection d'au moins trois, d'au moins quatre ou d'au moins cinq biomarqueurs sélectionnés dans le groupe constitué par ARHGDIB, TUBA4A, GSTO1, FLNA, PRDX6 et CDH13.

4. Procédé selon la revendication 1 ou 2, comprenant la détection d'ARHGDIB et TUBA4A ; ARHGDIB et GSTO1 ; ARHGDIB et FLNA ; ARHGDIB et PRDX6 ou ARHGDIB et CDH13.

5. Procédé selon l'une quelconque des revendications 1 à 4, comprenant la détection de

   (a) ARHGDIB, TUBA4A et GSTO1 ;
   (b) ARHGDIB, TUBA4A et FLNA ;
   (c) ARHGDIB, TUBA4A et PRDX6 ; ou
   (d) ARHGDIB, TUBA4A et CDH13.

6. Procédé selon l'une quelconque des revendications 1 à 5, comprenant la détection de

   (a) ARHGDIB, TUBA4A, GSTO1 et FLNA ;
   (b) ARHGDIB, TUBA4A, FLNA et PRDX6 ;
   (c) ARHGDIB, TUBA4A, GSTO1 et PRDX6.
   (d) ARHGDIB, TUBA4A, FLNA et CDH13 ;
   (e) ARHGDIB, TUBA4A, GSTO1 et CDH13 ; ou
   (f) ARHGDIB, TUBA4A, PRDX6 et CDH13.

7. Procédé selon l'une quelconque des revendications 1 à 6, comprenant la détection de

   (a) ARHGDIB, TUBA4A, GSTO1, FLNA et PRDX6 ;
   (b) ARHGDIB, TUBA4A, GSTO1, FLNA et CDH13 ;
   (c) ARHGDIB, TUBA4A, FLNA, PRDX6 et CDH13 ; ou
   (d) ARHGDIB, TUBA4A, GSTO1, PRDX6 et CDH13.

8. Procédé selon l'une quelconque des revendications 1 à 7, comprenant la détection d'ARHGDIB, TUBA4A, GSTO1, FLNA, PRDX6 et CDH13.

9. Procédé selon l'une quelconque des revendications 1 à 8, dans lequel l'échantillon biologique est un échantillon de fluide corporel ; de préférence un échantillon de fluide corporel sélectionné dans le groupe constitué par le plasma, le sérum, le sang total, l'urine, un lysat de tissu, le liquide céphalorachidien (LCR), la salive et la sueur ; plus préférablement dans lequel l'échantillon biologique est un échantillon de plasma.

10. Procédé selon l'une quelconque des revendications 1 à 9, dans lequel les au moins deux biomarqueurs sont détectés à l'aide de procédés d'analyse par spectrométrie de masse, de procédés de dosage biochimique, de procédés d'immunodosage, de procédés de chromatographie ou de combinaisons correspondantes.

11. Procédé selon l'une quelconque des revendications 1 à 10, comprenant les étapes de

   (a) mesure de la quantité ou des niveaux d'expression d'au moins ARHGDIB et d'au moins un biomarqueur sélectionné dans le groupe constitué par TUBA4A, GSTO1, FLNA, PRDX6 et CDH13 dans l'échantillon biologique du sujet ;
(b) calcul d'un score basé sur la quantité ou les niveaux d'expression desdits biomarqueurs mesurés en (a) ;
(c) comparaison du score calculé en (b) avec un score seuil ; et
(d) diagnostic du sujet atteint d'un cancer du poumon si le score calculé en (b) est supérieur ou égal au score seuil.

**12.** Utilisation d'un kit,

le kit étant constitué de :

(a) un moyen spécifiquement adapté pour mesurer la quantité ou les niveaux d'expression d'ARHGDIB et d'au moins un biomarqueur sélectionné dans le groupe constitué par TUBA4A, GSTO1, FLNA, PRDX6 et CDH13 dans un échantillon biologique d'un sujet ; et
(b) une valeur seuil pour chacun desdits biomarqueurs ou un moyen pour établir ladite valeur seuil, dans laquelle ladite valeur seuil représente un diagnostic connu de cancer du poumon,

dans le diagnostic du cancer du poumon basé sur la détection desdits biomarqueurs dans un échantillon biologique d'un sujet.

**13.** Utilisation selon la revendication 12, dans laquelle ledit moyen est un agent de liaison se liant spécifiquement à ladite protéine ou à un ARN codant pour ladite protéine.

**14.** Utilisation selon à la revendication 12 ou 13 dans le procédé selon l'une quelconque des revendications 1 à 11.

FIG 1.

A
Plasma FLNA concentration (fmol/uL)
100
80
60
40
20
0
****
Lung cancer
No cancer
B
Plasma TUBA4A concentration (fmol/uL)
20
15
10
5
0
****
Lung cancer
No cancer
C
Plasma GSTO1 concentration (fmol/uL)
40
30
20
10
0
****
Lung cancer
No cancer
D
Plasma PRDX6 concentration (fmol/uL)
40
30
20
10
0
****
Lung cancer
No cancer
E
Plasma ARHGDIB concentration (fmol/uL)
40
30
20
10
0
****
Lung cancer
No cancer
F
Plasma CDH13 concentration (fmol/uL)
250
200
150
100
50
0
****
Lung cancer
No cancer

FIG 2.

FLNA: SPFSVAVSPSLDLSK
SEQ ID NO: 4
Lung Cancer
Healthy
Intensity
1.5e+05
1.0e+05
5.0e+04
0.0e+00
3e+05
2e+05
1e+05
0e+00
Target
Internal Standard
43
44
45
44
45
RT
Ion type
y−10.1
y−8.1
y−9.1
y−7.1
y−12.1
b−3.1

TUBA4A: EIIDPVLDR
SEQ ID NO: 2
Lung Cancer
Healthy
Intensity
5e+05
4e+05
3e+05
2e+05
1e+05
0e+00
1e+06
5e+05
0e+00
Target
Internal Standard
35.5
36.0
36.5
37.0
35.5
36.0
36.5
37.0
RT
Ion type
y−5.1
y−6.1
y−5.2
y−7.1
y−3.1
y−4.1

FIG 2. (continued)

GSTO1: GSAPPGPVPEGSIR
SEQ ID NO: 3
Lung Cancer
Healthy
Intensity
4e+05
3e+05
2e+05
1e+05
0e+00
1e+06
5e+05
0e+00
26
27
RT
Target
Internal Standard
Ion type
y−11.2
y−10.1
y−10.2
y−6.1
y−8.1
y−9.1
PRDX6: LSILYPATTGR
SEQ ID NO: 5
Lung Cancer
Healthy
Intensity
3e+05
2e+05
1e+05
0e+00
36
37
38
RT
Target
Internal Standard
Ion type
y−6.1
y−7.1
y−8.1
b−3.1
y−6.2
b−4.1

FIG 2. (continued)

ARHGDIB: YVQHTYR
SEQ ID NO: 1
Lung Cancer
Healthy
Intensity
Target
Internal Standard
RT
Ion type
y−5.1
y−4.1
y−3.1
y−6.1
y−6.2
y−2.1
CDH13: SIVVSPILIPENQR
SEQ ID NO: 6
Lung Cancer
Healthy
Intensity
Target
Internal Standard
RT
Ion type
y−9.1
y−5.1
b−3.1
y−10.1
y−6.1
y−7.1

FIG. 3

| Combination | NPV | 95% CI LL | 95% CI UL |
|---|---|---|---|
| ARHGDIB-CDH13 | 0.975 | 0.868 | 0.999 |
| FLNA-GSTO1-ARHGDIB-CDH13 | 0.956 | 0.849 | 0.995 |
| FLNA-GSTO1-ARHGDIB | 0.956 | 0.849 | 0.995 |
| FLNA-PRDX6-ARHGDIB | 0.956 | 0.849 | 0.995 |
| GSTO1-ARHGDIB-CDH13 | 0.956 | 0.849 | 0.995 |
| PRDX6-ARHGDIB-CDH13 | 0.956 | 0.849 | 0.995 |
| FLNA-TUBA4A-GSTO1-ARHGDIB-CDH13 | 0.955 | 0.845 | 0.994 |
| FLNA-GSTO1-PRDX6-ARHGDIB | 0.955 | 0.845 | 0.994 |
| PRDX6-ARHGDIB | 0.955 | 0.845 | 0.994 |
| FLNA-ARHGDIB-CDH13 | 0.936 | 0.825 | 0.987 |
| TUBA4A-ARHGDIB-CDH13 | 0.936 | 0.825 | 0.987 |
| FLNA-TUBA4A-GSTO1-PRDX6-ARHGDIB-CDH13 | 0.935 | 0.821 | 0.986 |
| FLNA-TUBA4A-PRDX6-ARHGDIB-CDH13 | 0.935 | 0.821 | 0.986 |
| FLNA-GSTO1-PRDX6-ARHGDIB-CDH13 | 0.935 | 0.821 | 0.986 |
| FLNA-TUBA4A-GSTO1-ARHGDIB | 0.935 | 0.821 | 0.986 |
| FLNA-TUBA4A-PRDX6-ARHGDIB | 0.935 | 0.821 | 0.986 |
| FLNA-TUBA4A-ARHGDIB-CDH13 | 0.935 | 0.821 | 0.986 |
| FLNA-PRDX6-ARHGDIB-CDH13 | 0.935 | 0.821 | 0.986 |
| TUBA4A-GSTO1-PRDX6-ARHGDIB | 0.935 | 0.821 | 0.986 |
| TUBA4A-GSTO1-ARHGDIB-CDH13 | 0.935 | 0.821 | 0.986 |
| TUBA4A-PRDX6-ARHGDIB-CDH13 | 0.935 | 0.821 | 0.986 |
| GSTO1-PRDX6-ARHGDIB-CDH13 | 0.935 | 0.821 | 0.986 |
| TUBA4A-GSTO1-ARHGDIB | 0.935 | 0.821 | 0.986 |
| TUBA4A-PRDX6-ARHGDIB | 0.935 | 0.821 | 0.986 |
| GSTO1-ARHGDIB | 0.935 | 0.821 | 0.986 |
| FLNA-TUBA4A-GSTO1-PRDX6-ARHGDIB | 0.933 | 0.817 | 0.986 |
| GSTO1-PRDX6-ARHGDIB | 0.933 | 0.817 | 0.986 |
| ARHGDIB | 0.932 | 0.813 | 0.986 |
| TUBA4A-GSTO1-PRDX6-ARHGDIB-CDH13 | 0.915 | 0.796 | 0.976 |
| FLNA-TUBA4A-ARHGDIB | 0.915 | 0.796 | 0.976 |
| FLNA-ARHGDIB | 0.915 | 0.796 | 0.976 |
| TUBA4A-ARHGDIB | 0.915 | 0.796 | 0.976 |
| TUBA4A-PRDX6-CDH13 | 0.9 | 0.782 | 0.967 |
| FLNA-TUBA4A-GSTO1-CDH13 | 0.898 | 0.778 | 0.966 |
| TUBA4A-GSTO1-CDH13 | 0.898 | 0.778 | 0.966 |
| GSTO1-CDH13 | 0.898 | 0.778 | 0.966 |
| FLNA-GSTO1-PRDX6-CDH13 | 0.882 | 0.761 | 0.956 |
| PRDX6-CDH13 | 0.882 | 0.761 | 0.956 |
| FLNA-TUBA4A-GSTO1-PRDX6-CDH13 | 0.88 | 0.757 | 0.955 |
| FLNA-TUBA4A-PRDX6-CDH13 | 0.88 | 0.757 | 0.955 |
| TUBA4A-GSTO1-PRDX6-CDH13 | 0.88 | 0.757 | 0.955 |
| FLNA-TUBA4A-GSTO1 | 0.88 | 0.757 | 0.955 |
| FLNA-GSTO1-CDH13 | 0.88 | 0.757 | 0.955 |
| GSTO1-PRDX6-CDH13 | 0.88 | 0.757 | 0.955 |
| FLNA-TUBA4A-GSTO1-PRDX6 | 0.863 | 0.737 | 0.943 |
| FLNA-TUBA4A-PRDX6 | 0.863 | 0.737 | 0.943 |
| FLNA-TUBA4A-CDH13 | 0.863 | 0.737 | 0.943 |
| FLNA-GSTO1-PRDX6 | 0.863 | 0.737 | 0.943 |
| FLNA-PRDX6-CDH13 | 0.863 | 0.737 | 0.943 |
| TUBA4A-GSTO1-PRDX6 | 0.863 | 0.737 | 0.943 |
| TUBA4A-GSTO1 | 0.863 | 0.737 | 0.943 |
| TUBA4A-PRDX6 | 0.863 | 0.737 | 0.943 |
| TUBA4A-CDH13 | 0.863 | 0.737 | 0.943 |
| GSTO1-PRDX6 | 0.863 | 0.737 | 0.943 |
| PRDX6 | 0.863 | 0.737 | 0.943 |
| FLNA-CDH13 | 0.849 | 0.724 | 0.933 |
| FLNA-GSTO1 | 0.846 | 0.719 | 0.931 |
| TUBA4A | 0.846 | 0.719 | 0.931 |
| FLNA-TUBA4A | 0.83 | 0.702 | 0.919 |
| FLNA-PRDX6 | 0.83 | 0.702 | 0.919 |
| GSTO1 | 0.83 | 0.702 | 0.919 |
| CDH13 | 0.826 | 0.612 | 0.95 |
| FLNA | 0.763 | 0.634 | 0.864 |

0.65 0.7 0.75 0.8 0.85 0.9 0.95

NPV

FIG. 3 (continued)

| Combination | PPV | 95% CI LL | 95% CI UL |
|---|---|---|---|
| TUBA4A-PRDX6-CDH13 | 0.915 | 0.796 | 0.976 |
| FLNA-GSTO1-PRDX6-CDH13 | 0.913 | 0.792 | 0.976 |
| PRDX6-CDH13 | 0.913 | 0.792 | 0.976 |
| FLNA-CDH13 | 0.909 | 0.783 | 0.975 |
| FLNA-ARHGDIB-CDH13 | 0.9 | 0.782 | 0.967 |
| TUBA4A-ARHGDIB-CDH13 | 0.9 | 0.782 | 0.967 |
| FLNA-TUBA4A-GSTO1-CDH13 | 0.896 | 0.773 | 0.965 |
| TUBA4A-GSTO1-CDH13 | 0.896 | 0.773 | 0.965 |
| GSTO1-CDH13 | 0.896 | 0.773 | 0.965 |
| FLNA | 0.895 | 0.752 | 0.971 |
| FLNA-TUBA4A-GSTO1-PRDX6-CDH13 | 0.894 | 0.769 | 0.965 |
| FLNA-TUBA4A-PRDX6-CDH13 | 0.894 | 0.769 | 0.965 |
| TUBA4A-GSTO1-PRDX6-CDH13 | 0.894 | 0.769 | 0.965 |
| FLNA-TUBA4A-GSTO1 | 0.894 | 0.769 | 0.965 |
| FLNA-GSTO1-CDH13 | 0.894 | 0.769 | 0.965 |
| GSTO1-PRDX6-CDH13 | 0.894 | 0.769 | 0.965 |
| FLNA-TUBA4A-GSTO1-PRDX6 | 0.891 | 0.764 | 0.964 |
| FLNA-TUBA4A-PRDX6 | 0.891 | 0.764 | 0.964 |
| FLNA-TUBA4A-CDH13 | 0.891 | 0.764 | 0.964 |
| FLNA-GSTO1-PRDX6 | 0.891 | 0.764 | 0.964 |
| FLNA-PRDX6-CDH13 | 0.891 | 0.764 | 0.964 |
| TUBA4A-GSTO1-PRDX6 | 0.891 | 0.764 | 0.964 |
| TUBA4A-GSTO1 | 0.891 | 0.764 | 0.964 |
| TUBA4A-PRDX6 | 0.891 | 0.764 | 0.964 |
| TUBA4A-CDH13 | 0.891 | 0.764 | 0.964 |
| GSTO1-PRDX6 | 0.891 | 0.764 | 0.964 |
| PRDX6 | 0.891 | 0.764 | 0.964 |
| FLNA-GSTO1 | 0.889 | 0.759 | 0.963 |
| TUBA4A | 0.889 | 0.759 | 0.963 |
| FLNA-TUBA4A | 0.886 | 0.754 | 0.962 |
| FLNA-PRDX6 | 0.886 | 0.754 | 0.962 |
| GSTO1 | 0.886 | 0.754 | 0.962 |
| FLNA-GSTO1-ARHGDIB-CDH13 | 0.885 | 0.766 | 0.956 |
| FLNA-GSTO1-ARHGDIB | 0.885 | 0.766 | 0.956 |
| FLNA-PRDX6-ARHGDIB | 0.885 | 0.766 | 0.956 |
| GSTO1-ARHGDIB-CDH13 | 0.885 | 0.766 | 0.956 |
| PRDX6-ARHGDIB-CDH13 | 0.885 | 0.766 | 0.956 |
| FLNA-TUBA4A-GSTO1-PRDX6-ARHGDIB-CDH13 | 0.882 | 0.761 | 0.956 |
| FLNA-TUBA4A-PRDX6-ARHGDIB-CDH13 | 0.882 | 0.761 | 0.956 |
| FLNA-GSTO1-PRDX6-ARHGDIB-CDH13 | 0.882 | 0.761 | 0.956 |
| FLNA-TUBA4A-GSTO1-ARHGDIB | 0.882 | 0.761 | 0.956 |
| FLNA-TUBA4A-PRDX6-ARHGDIB | 0.882 | 0.761 | 0.956 |
| FLNA-TUBA4A-ARHGDIB-CDH13 | 0.882 | 0.761 | 0.956 |
| FLNA-PRDX6-ARHGDIB-CDH13 | 0.882 | 0.761 | 0.956 |
| TUBA4A-GSTO1-PRDX6-ARHGDIB | 0.882 | 0.761 | 0.956 |
| TUBA4A-GSTO1-ARHGDIB-CDH13 | 0.882 | 0.761 | 0.956 |
| TUBA4A-PRDX6-ARHGDIB-CDH13 | 0.882 | 0.761 | 0.956 |
| GSTO1-PRDX6-ARHGDIB-CDH13 | 0.882 | 0.761 | 0.956 |
| TUBA4A-GSTO1-ARHGDIB | 0.882 | 0.761 | 0.956 |
| TUBA4A-PRDX6-ARHGDIB | 0.882 | 0.761 | 0.956 |
| GSTO1-ARHGDIB | 0.882 | 0.761 | 0.956 |
| TUBA4A-GSTO1-PRDX6-ARHGDIB-CDH13 | 0.88 | 0.757 | 0.955 |
| FLNA-TUBA4A-ARHGDIB | 0.88 | 0.757 | 0.955 |
| FLNA-ARHGDIB | 0.88 | 0.757 | 0.955 |
| TUBA4A-ARHGDIB | 0.88 | 0.757 | 0.955 |
| FLNA-TUBA4A-GSTO1-ARHGDIB-CDH13 | 0.868 | 0.747 | 0.945 |
| FLNA-GSTO1-PRDX6-ARHGDIB | 0.868 | 0.747 | 0.945 |
| PRDX6-ARHGDIB | 0.868 | 0.747 | 0.945 |
| FLNA-TUBA4A-GSTO1-PRDX6-ARHGDIB | 0.865 | 0.742 | 0.944 |
| GSTO1-PRDX6-ARHGDIB | 0.865 | 0.742 | 0.944 |
| ARHGDIB | 0.849 | 0.724 | 0.933 |
| ARHGDIB-CDH13 | 0.825 | 0.701 | 0.913 |
| CDH13 | 0.595 | 0.474 | 0.707 |

0.5 0.6 0.7 0.8 0.9

PPV

FIG. 3 (continued)

| Combination | Sens | 95% CI LL | 95% CI UL |
|---|---|---|---|
| ARHGDIB-CDH13 | 0.979 | 0.889 | 0.999 |
| FLNA-TUBA4A-GSTO1-ARHGDIB-CDH13 | 0.958 | 0.857 | 0.995 |
| FLNA-GSTO1-PRDX6-ARHGDIB | 0.958 | 0.857 | 0.995 |
| FLNA-GSTO1-ARHGDIB-CDH13 | 0.958 | 0.857 | 0.995 |
| FLNA-GSTO1-ARHGDIB | 0.958 | 0.857 | 0.995 |
| FLNA-PRDX6-ARHGDIB | 0.958 | 0.857 | 0.995 |
| GSTO1-ARHGDIB-CDH13 | 0.958 | 0.857 | 0.995 |
| PRDX6-ARHGDIB-CDH13 | 0.958 | 0.857 | 0.995 |
| PRDX6-ARHGDIB | 0.958 | 0.857 | 0.995 |
| FLNA-TUBA4A-GSTO1-PRDX6-ARHGDIB-CDH13 | 0.938 | 0.828 | 0.987 |
| FLNA-TUBA4A-GSTO1-PRDX6-ARHGDIB | 0.938 | 0.828 | 0.987 |
| FLNA-TUBA4A-PRDX6-ARHGDIB-CDH13 | 0.938 | 0.828 | 0.987 |
| FLNA-GSTO1-PRDX6-ARHGDIB-CDH13 | 0.938 | 0.828 | 0.987 |
| FLNA-TUBA4A-GSTO1-ARHGDIB | 0.938 | 0.828 | 0.987 |
| FLNA-TUBA4A-PRDX6-ARHGDIB | 0.938 | 0.828 | 0.987 |
| FLNA-TUBA4A-ARHGDIB-CDH13 | 0.938 | 0.828 | 0.987 |
| FLNA-PRDX6-ARHGDIB-CDH13 | 0.938 | 0.828 | 0.987 |
| TUBA4A-GSTO1-PRDX6-ARHGDIB | 0.938 | 0.828 | 0.987 |
| TUBA4A-GSTO1-ARHGDIB-CDH13 | 0.938 | 0.828 | 0.987 |
| TUBA4A-PRDX6-ARHGDIB-CDH13 | 0.938 | 0.828 | 0.987 |
| GSTO1-PRDX6-ARHGDIB-CDH13 | 0.938 | 0.828 | 0.987 |
| FLNA-ARHGDIB-CDH13 | 0.938 | 0.828 | 0.987 |
| TUBA4A-GSTO1-ARHGDIB | 0.938 | 0.828 | 0.987 |
| TUBA4A-PRDX6-ARHGDIB | 0.938 | 0.828 | 0.987 |
| TUBA4A-ARHGDIB-CDH13 | 0.938 | 0.828 | 0.987 |
| GSTO1-PRDX6-ARHGDIB | 0.938 | 0.828 | 0.987 |
| GSTO1-ARHGDIB | 0.938 | 0.828 | 0.987 |
| ARHGDIB | 0.938 | 0.828 | 0.987 |
| TUBA4A-GSTO1-PRDX6-ARHGDIB-CDH13 | 0.917 | 0.8 | 0.977 |
| FLNA-TUBA4A-ARHGDIB | 0.917 | 0.8 | 0.977 |
| FLNA-ARHGDIB | 0.917 | 0.8 | 0.977 |
| TUBA4A-ARHGDIB | 0.917 | 0.8 | 0.977 |
| CDH13 | 0.917 | 0.8 | 0.977 |
| FLNA-TUBA4A-GSTO1-CDH13 | 0.896 | 0.773 | 0.965 |
| TUBA4A-GSTO1-CDH13 | 0.896 | 0.773 | 0.965 |
| TUBA4A-PRDX6-CDH13 | 0.896 | 0.773 | 0.965 |
| GSTO1-CDH13 | 0.896 | 0.773 | 0.965 |
| FLNA-TUBA4A-GSTO1-PRDX6-CDH13 | 0.875 | 0.748 | 0.953 |
| FLNA-TUBA4A-PRDX6-CDH13 | 0.875 | 0.748 | 0.953 |
| FLNA-GSTO1-PRDX6-CDH13 | 0.875 | 0.748 | 0.953 |
| TUBA4A-GSTO1-PRDX6-CDH13 | 0.875 | 0.748 | 0.953 |
| FLNA-TUBA4A-GSTO1 | 0.875 | 0.748 | 0.953 |
| FLNA-GSTO1-CDH13 | 0.875 | 0.748 | 0.953 |
| GSTO1-PRDX6-CDH13 | 0.875 | 0.748 | 0.953 |
| PRDX6-CDH13 | 0.875 | 0.748 | 0.953 |
| FLNA-TUBA4A-GSTO1-PRDX6 | 0.854 | 0.722 | 0.939 |
| FLNA-TUBA4A-PRDX6 | 0.854 | 0.722 | 0.939 |
| FLNA-TUBA4A-CDH13 | 0.854 | 0.722 | 0.939 |
| FLNA-GSTO1-PRDX6 | 0.854 | 0.722 | 0.939 |
| FLNA-PRDX6-CDH13 | 0.854 | 0.722 | 0.939 |
| TUBA4A-GSTO1-PRDX6 | 0.854 | 0.722 | 0.939 |
| TUBA4A-GSTO1 | 0.854 | 0.722 | 0.939 |
| TUBA4A-PRDX6 | 0.854 | 0.722 | 0.939 |
| TUBA4A-CDH13 | 0.854 | 0.722 | 0.939 |
| GSTO1-PRDX6 | 0.854 | 0.722 | 0.939 |
| PRDX6 | 0.854 | 0.722 | 0.939 |
| FLNA-GSTO1 | 0.833 | 0.698 | 0.925 |
| FLNA-CDH13 | 0.833 | 0.698 | 0.925 |
| TUBA4A | 0.833 | 0.698 | 0.925 |
| FLNA-TUBA4A | 0.812 | 0.674 | 0.911 |
| FLNA-PRDX6 | 0.812 | 0.674 | 0.911 |
| GSTO1 | 0.812 | 0.674 | 0.911 |
| FLNA | 0.708 | 0.559 | 0.83 |

0.6
0.7
0.8
0.9
Sens

FIG. 3 (continued)

| Combination | Spec | 95% CI LL | 95% CI UL |
|---|---|---|---|
| FLNA-GSTO1-PRDX6-CDH13 | 0.918 | 0.804 | 0.977 |
| TUBA4A-PRDX6-CDH13 | 0.918 | 0.804 | 0.977 |
| FLNA-CDH13 | 0.918 | 0.804 | 0.977 |
| PRDX6-CDH13 | 0.918 | 0.804 | 0.977 |
| FLNA | 0.918 | 0.804 | 0.977 |
| FLNA-TUBA4A-GSTO1-PRDX6-CDH13 | 0.898 | 0.778 | 0.966 |
| FLNA-TUBA4A-GSTO1-PRDX6 | 0.898 | 0.778 | 0.966 |
| FLNA-TUBA4A-GSTO1-CDH13 | 0.898 | 0.778 | 0.966 |
| FLNA-TUBA4A-PRDX6-CDH13 | 0.898 | 0.778 | 0.966 |
| TUBA4A-GSTO1-PRDX6-CDH13 | 0.898 | 0.778 | 0.966 |
| FLNA-TUBA4A-GSTO1 | 0.898 | 0.778 | 0.966 |
| FLNA-TUBA4A-PRDX6 | 0.898 | 0.778 | 0.966 |
| FLNA-TUBA4A-CDH13 | 0.898 | 0.778 | 0.966 |
| FLNA-GSTO1-PRDX6 | 0.898 | 0.778 | 0.966 |
| FLNA-GSTO1-CDH13 | 0.898 | 0.778 | 0.966 |
| FLNA-PRDX6-CDH13 | 0.898 | 0.778 | 0.966 |
| FLNA-ARHGDIB-CDH13 | 0.898 | 0.778 | 0.966 |
| TUBA4A-GSTO1-PRDX6 | 0.898 | 0.778 | 0.966 |
| TUBA4A-GSTO1-CDH13 | 0.898 | 0.778 | 0.966 |
| TUBA4A-ARHGDIB-CDH13 | 0.898 | 0.778 | 0.966 |
| GSTO1-PRDX6-CDH13 | 0.898 | 0.778 | 0.966 |
| FLNA-TUBA4A | 0.898 | 0.778 | 0.966 |
| FLNA-GSTO1 | 0.898 | 0.778 | 0.966 |
| FLNA-PRDX6 | 0.898 | 0.778 | 0.966 |
| TUBA4A-GSTO1 | 0.898 | 0.778 | 0.966 |
| TUBA4A-PRDX6 | 0.898 | 0.778 | 0.966 |
| TUBA4A-CDH13 | 0.898 | 0.778 | 0.966 |
| GSTO1-PRDX6 | 0.898 | 0.778 | 0.966 |
| GSTO1-CDH13 | 0.898 | 0.778 | 0.966 |
| TUBA4A | 0.898 | 0.778 | 0.966 |
| GSTO1 | 0.898 | 0.778 | 0.966 |
| PRDX6 | 0.898 | 0.778 | 0.966 |
| FLNA-TUBA4A-GSTO1-PRDX6-ARHGDIB-CDH13 | 0.878 | 0.752 | 0.954 |
| FLNA-TUBA4A-PRDX6-ARHGDIB-CDH13 | 0.878 | 0.752 | 0.954 |
| FLNA-GSTO1-PRDX6-ARHGDIB-CDH13 | 0.878 | 0.752 | 0.954 |
| TUBA4A-GSTO1-PRDX6-ARHGDIB-CDH13 | 0.878 | 0.752 | 0.954 |
| FLNA-TUBA4A-GSTO1-ARHGDIB | 0.878 | 0.752 | 0.954 |
| FLNA-TUBA4A-PRDX6-ARHGDIB | 0.878 | 0.752 | 0.954 |
| FLNA-TUBA4A-ARHGDIB-CDH13 | 0.878 | 0.752 | 0.954 |
| FLNA-GSTO1-ARHGDIB-CDH13 | 0.878 | 0.752 | 0.954 |
| FLNA-PRDX6-ARHGDIB-CDH13 | 0.878 | 0.752 | 0.954 |
| TUBA4A-GSTO1-PRDX6-ARHGDIB | 0.878 | 0.752 | 0.954 |
| TUBA4A-GSTO1-ARHGDIB-CDH13 | 0.878 | 0.752 | 0.954 |
| TUBA4A-PRDX6-ARHGDIB-CDH13 | 0.878 | 0.752 | 0.954 |
| GSTO1-PRDX6-ARHGDIB-CDH13 | 0.878 | 0.752 | 0.954 |
| FLNA-TUBA4A-ARHGDIB | 0.878 | 0.752 | 0.954 |
| FLNA-GSTO1-ARHGDIB | 0.878 | 0.752 | 0.954 |
| FLNA-PRDX6-ARHGDIB | 0.878 | 0.752 | 0.954 |
| TUBA4A-GSTO1-ARHGDIB | 0.878 | 0.752 | 0.954 |
| TUBA4A-PRDX6-ARHGDIB | 0.878 | 0.752 | 0.954 |
| GSTO1-ARHGDIB-CDH13 | 0.878 | 0.752 | 0.954 |
| PRDX6-ARHGDIB-CDH13 | 0.878 | 0.752 | 0.954 |
| FLNA-ARHGDIB | 0.878 | 0.752 | 0.954 |
| TUBA4A-ARHGDIB | 0.878 | 0.752 | 0.954 |
| GSTO1-ARHGDIB | 0.878 | 0.752 | 0.954 |
| FLNA-TUBA4A-GSTO1-PRDX6-ARHGDIB | 0.857 | 0.728 | 0.941 |
| FLNA-TUBA4A-GSTO1-ARHGDIB-CDH13 | 0.857 | 0.728 | 0.941 |
| FLNA-GSTO1-PRDX6-ARHGDIB | 0.857 | 0.728 | 0.941 |
| GSTO1-PRDX6-ARHGDIB | 0.857 | 0.728 | 0.941 |
| PRDX6-ARHGDIB | 0.857 | 0.728 | 0.941 |
| ARHGDIB | 0.837 | 0.703 | 0.927 |
| ARHGDIB-CDH13 | 0.796 | 0.657 | 0.898 |
| CDH13 | 0.388 | 0.252 | 0.538 |

0.3 0.4 0.5 0.6 0.7 0.8 0.9
Spec

FIG. 3 (continued)

| Combination | AUC | 95% CI LL | 95% CI UL |
|---|---|---|---|
| FLNA-GSTO1-ARHGDIB-CDH13 | 0.918 | 0.863 | 0.972 |
| FLNA-GSTO1-ARHGDIB | 0.918 | 0.863 | 0.972 |
| FLNA-PRDX6-ARHGDIB | 0.918 | 0.863 | 0.972 |
| GSTO1-ARHGDIB-CDH13 | 0.918 | 0.863 | 0.972 |
| PRDX6-ARHGDIB-CDH13 | 0.918 | 0.863 | 0.972 |
| FLNA-ARHGDIB-CDH13 | 0.918 | 0.863 | 0.973 |
| TUBA4A-ARHGDIB-CDH13 | 0.918 | 0.863 | 0.973 |
| FLNA-TUBA4A-GSTO1-ARHGDIB-CDH13 | 0.908 | 0.851 | 0.965 |
| FLNA-GSTO1-PRDX6-ARHGDIB | 0.908 | 0.851 | 0.965 |
| PRDX6-ARHGDIB | 0.908 | 0.851 | 0.965 |
| FLNA-TUBA4A-GSTO1-PRDX6-ARHGDIB-CDH13 | 0.908 | 0.85 | 0.965 |
| FLNA-TUBA4A-PRDX6-ARHGDIB-CDH13 | 0.908 | 0.85 | 0.965 |
| FLNA-GSTO1-PRDX6-ARHGDIB-CDH13 | 0.908 | 0.85 | 0.965 |
| FLNA-TUBA4A-GSTO1-ARHGDIB | 0.908 | 0.85 | 0.965 |
| FLNA-TUBA4A-PRDX6-ARHGDIB | 0.908 | 0.85 | 0.965 |
| FLNA-TUBA4A-ARHGDIB-CDH13 | 0.908 | 0.85 | 0.965 |
| FLNA-PRDX6-ARHGDIB-CDH13 | 0.908 | 0.85 | 0.965 |
| TUBA4A-GSTO1-PRDX6-ARHGDIB | 0.908 | 0.85 | 0.965 |
| TUBA4A-GSTO1-ARHGDIB-CDH13 | 0.908 | 0.85 | 0.965 |
| TUBA4A-PRDX6-ARHGDIB-CDH13 | 0.908 | 0.85 | 0.965 |
| GSTO1-PRDX6-ARHGDIB-CDH13 | 0.908 | 0.85 | 0.965 |
| TUBA4A-GSTO1-ARHGDIB | 0.908 | 0.85 | 0.965 |
| TUBA4A-PRDX6-ARHGDIB | 0.908 | 0.85 | 0.965 |
| GSTO1-ARHGDIB | 0.908 | 0.85 | 0.965 |
| TUBA4A-PRDX6-CDH13 | 0.907 | 0.849 | 0.965 |
| FLNA-TUBA4A-GSTO1-PRDX6-ARHGDIB | 0.897 | 0.837 | 0.958 |
| GSTO1-PRDX6-ARHGDIB | 0.897 | 0.837 | 0.958 |
| TUBA4A-GSTO1-PRDX6-ARHGDIB-CDH13 | 0.897 | 0.836 | 0.958 |
| FLNA-TUBA4A-ARHGDIB | 0.897 | 0.836 | 0.958 |
| FLNA-ARHGDIB | 0.897 | 0.836 | 0.958 |
| TUBA4A-ARHGDIB | 0.897 | 0.836 | 0.958 |
| FLNA-TUBA4A-GSTO1-CDH13 | 0.897 | 0.836 | 0.958 |
| TUBA4A-GSTO1-CDH13 | 0.897 | 0.836 | 0.958 |
| GSTO1-CDH13 | 0.897 | 0.836 | 0.958 |
| FLNA-GSTO1-PRDX6-CDH13 | 0.897 | 0.836 | 0.958 |
| PRDX6-CDH13 | 0.897 | 0.836 | 0.958 |
| ARHGDIB-CDH13 | 0.888 | 0.827 | 0.948 |
| ARHGDIB | 0.887 | 0.824 | 0.95 |
| FLNA-TUBA4A-GSTO1-PRDX6-CDH13 | 0.886 | 0.823 | 0.95 |
| FLNA-TUBA4A-PRDX6-CDH13 | 0.886 | 0.823 | 0.95 |
| TUBA4A-GSTO1-PRDX6-CDH13 | 0.886 | 0.823 | 0.95 |
| FLNA-TUBA4A-GSTO1 | 0.886 | 0.823 | 0.95 |
| FLNA-GSTO1-CDH13 | 0.886 | 0.823 | 0.95 |
| GSTO1-PRDX6-CDH13 | 0.886 | 0.823 | 0.95 |
| FLNA-TUBA4A-GSTO1-PRDX6 | 0.876 | 0.81 | 0.942 |
| FLNA-TUBA4A-PRDX6 | 0.876 | 0.81 | 0.942 |
| FLNA-TUBA4A-CDH13 | 0.876 | 0.81 | 0.942 |
| FLNA-GSTO1-PRDX6 | 0.876 | 0.81 | 0.942 |
| FLNA-PRDX6-CDH13 | 0.876 | 0.81 | 0.942 |
| TUBA4A-GSTO1-PRDX6 | 0.876 | 0.81 | 0.942 |
| TUBA4A-GSTO1 | 0.876 | 0.81 | 0.942 |
| TUBA4A-PRDX6 | 0.876 | 0.81 | 0.942 |
| TUBA4A-CDH13 | 0.876 | 0.81 | 0.942 |
| GSTO1-PRDX6 | 0.876 | 0.81 | 0.942 |
| PRDX6 | 0.876 | 0.81 | 0.942 |
| FLNA-CDH13 | 0.876 | 0.81 | 0.942 |
| FLNA-GSTO1 | 0.866 | 0.797 | 0.934 |
| TUBA4A | 0.866 | 0.797 | 0.934 |
| FLNA-TUBA4A | 0.855 | 0.785 | 0.926 |
| FLNA-PRDX6 | 0.855 | 0.785 | 0.926 |
| GSTO1 | 0.855 | 0.785 | 0.926 |
| FLNA | 0.813 | 0.738 | 0.889 |
| CDH13 | 0.652 | 0.573 | 0.732 |

0.6
0.7
0.8
0.9
AUC

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 20140274772 A1
- WO 2018148600 A1
- WO 2015115922 A1
- US 20130230877 A1
- US 20160153053 A1


### Non-patent literature cited in the description


- **VICTORIA EL-KHOURY et al.** Identification of beta-arrestin-1 as a diagnostic biomarker in lung cancer. *British Journal of Cancer,* 2018, vol. 119, 580-590
- **HUIYAN NIU et al.** *Expression profile of RhoGD 12 in lung cancers and role of RhoGD 12 in lung cancer metastasis,* 2010, vol. 24, 465-471
- **COHEN JD ; LI L ; WANG Y ; THOBURN C ; AFSARI B ; DANILOVA L et al.** Detection and localization of surgically resectable cancers with a multi-analyte blood test. *Science,* 2018, vol. 359, 926-30
- **ALTSCHUL et al.** *J Mol Biol,* 1990, vol. 215, 403-10
- **TATUSOVA ; MADDEN.** *FEMS Microbiol Lett,* 1999, vol. 174, 247-250
- **LOWRY et al.** *J Biol Chem,* 1951, vol. 193, 265
- **HARTREE.** *Anal Biochem,* 1972, vol. 48, 422-427
- Mass Spectrometry of Proteins and Peptides. Methods in Molecular Biology. Humana Press, 2000, vol. 146
- **BIEMANN.** *Methods Enzymol,* 1990, vol. 193, 455-79
- *Methods in Enzymology,* vol. 402
- Biological Mass Spectrometry. Academic Press, 2005
- **MEYER M. ; BIDLINGMEYER, B. A.** Practical HPLC Methodology and Applications. John Wiley & Sons Inc, 1993
- **BOURMAUD A ; GALLIEN S ; DOMON B.** Parallel reaction monitoring using quadrupole-Orbitrap mass spectrometer: Principle and applications. *Proteomics.,* 2016, vol. 16, 2146-59
- **ROBIN X. ; PANELOMIX.** a threshold-based algorithm to create panels of biomarkers. *Translational Proteomics,* 2013, vol. 1 (1), 57-64
- **KIM YJ et al.** Quantification of SAA1 and SAA2 in lung cancer plasma using the isotype-specific PRM assays. *Proteomics,* 2015, vol. 15, 3116-3125
- **ROBIN X. ; PANELOMIX.** Combination of Biomarkers. *Methods Mol Biol,* 2019, vol. 1959, 261-273
- **SALHIA B ; KIEFER J ; ROSS JT et al.** Integrated genomic and epigenomic analysis of breast cancer brain metastasis. *PLoS One,* 2014, vol. 9, e85448
- **ZHANG H ; KENNEDY J ; LEE LW et al.** Integrated Strategy for Lung Cancer Biomarker Candidate Discovery by Quantitative Proteomics Profiling on Tumor and Adjacent Normal Lung Tissue (abstract). *59 th ASMS Conference on Mass Spectrometry and Allied Topics Denver,* 2011
- **ZHANG H ; WHITEAKER J ; LIN C et al.** Prioritization of Plasma-Based Predictive Markers for Chemotherapy in Lung Cancer Using Fractionation and Targeted Mass Spectrometry (abstract). *ASMS Conference on Mass Spectrometry and Allied Topics.,* 2013